(19) Europäisches Patentamt
European Patent Office
Office européen des brevets

(11) **EP 3 892 634 A1**

(12) **EUROPEAN PATENT APPLICATION**
published in accordance with Art. 153(4) EPC

(43) Date of publication:
**13.10.2021 Bulletin 2021/41**

(51) Int Cl.:
*C07K 16/28* (2006.01)   *A61P 35/00* (2006.01)
*A61K 39/395* (2006.01)   *A61P 37/00* (2006.01)
*C12N 15/13* (2006.01)   *C12N 15/63* (2006.01)
*A61P 29/00* (2006.01)

(21) Application number: **19891086.1**

(22) Date of filing: **29.11.2019**

(86) International application number:
**PCT/CN2019/121941**

(87) International publication number:
**WO 2020/108611 (04.06.2020 Gazette 2020/23)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **30.11.2018 CN 201811448228**

(71) Applicants:
• **Jiangsu Hengrui Medicine Co., Ltd.
Lianyungang, Jiangsu 222047 (CN)**
• **Shanghai Hengrui Pharmaceutical Co., Ltd.
Shanghai 200245 (CN)**

(72) Inventors:
• **LIAO, Cheng
Lianyungang, Jiangsu 222047 (CN)**

• **JIANG, Jiahua
Lianyungang, Jiangsu 222047 (CN)**
• **XU, Zupeng
Lianyungang, Jiangsu 222047 (CN)**
• **ZHANG, Lianshan
Lianyungang, Jiangsu 222047 (CN)**
• **LIN, Yuan
Lianyungang, Jiangsu 222047 (CN)**
• **LIN, Kan
Lianyungang, Jiangsu 222047 (CN)**
• **QIAN, Xueming
Lianyungang, Jiangsu 222047 (CN)**
• **TENG, Fei
Lianyungang, Jiangsu 222047 (CN)**

(74) Representative: **Sonzogni, Laura Gabriella et al
Dragotti & Associati S.r.l.
Via Nino Bixio, 7
20129 Milano (IT)**

(54) **ANTI-CD40 ANTIBODY, ANTIGEN BINDING FRAGMENT AND PHARMACEUTICAL USE THEREOF**

(57) The present invention relates to an anti-CD40 antibody, an antigen binding fragment and a pharmaceutical use thereof. Heavy-chain constant regions of the anti-CD40 antibody and the antigen binding fragment thereof contain mutations. Due to the mutations, the anti-CD40 antibody loses the binding activity to FcγRIII, and the binding of the anti-CD40 antibody and FcγRIIB is enhanced, thereby losing the antibody-dependent cytotoxicity (ADCC) but improving FcγRIIB-mediated antibody crosslinking. The mutations in the heavy-chain constant regions enhance the activation of CD40, and enhance the presentation of dendritic cells to antigens. The anti-CD40 antibody and the antigen binding fragment thereof can be used as anti-cancer drugs to treat CD40-mediated diseases or symptoms.

EP 3 892 634 A1

**Description**

The present application claims the priority of Chinese patent application "anti-CD40 antibody, antigen-binding fragment and pharmaceutical use thereof" (application number CN201811448228.1) filed on November 30, 2018.

**FIELD OF THE INVENTION**

The present disclosure relates to an anti-CD40 antibody or antigen-binding fragment thereof comprising mutation(s) in the heavy chain constant region, a chimeric antibody or a humanized antibody comprising CDRs of the anti-CD40 antibody, and a pharmaceutical composition comprising the anti-human CD40 antibody or antigen-binding fragment thereof, and the use of the same as an anticancer agent.

**BACKGROUND OF THE INVENTION**

Cancers have become the biggest health challenge faced by human society for a long time. Traditional therapies such as surgery, chemotherapy and radiotherapy show little effect in the treatment of disseminated solid tumors. Tumor immunotherapy is a hot spot in the field of tumor therapy, and tumor immunotherapy by T cells is at a core position. Tumor immunotherapy makes full use of killer T cells, and mobilizes the killer T cells in tumor patients to kill the tumor. Tumor immunotherapy may be one of the most effective and safest ways to treat tumors. Tumor immunotherapy currently has favorable prospects for the treatment of several different types of cancers, including disseminated metastatic tumors.

The activation of T cells in the human body adopts a dual-signal pathway system: MHC-antigen peptides are presented to T cells through antigen presenting cells (APC) to provide the first signal; a series of co-stimulatory molecules are required to provide the second signal, and then T cells produce a normal immune response. This dual-signal pathway system plays a vital role in the balance of the immune system *in vivo.* It strictly regulates the body's different immune responses to self-antigens and non-self-antigens. In the absence of the second signal provided by the co-stimulatory molecule, T cells will not be responsive or generate a sustained specific immune response, consequently resulting in tolerance. Therefore, the second signal pathway plays a very critical regulatory role in the entire process of the body's immune response.

CD40 is one of the glycoproteins expressed on the cell surface. It is a type I intra-membrane glycoprotein with a molecular weight of about 48kDa. CD40 belongs to the tumor necrosis factor receptor (TNFR) superfamily and plays an important role in the immune system. CD40 is expressed in a variety of immune cells, such as B cells, dendritic cells, monocytes and macrophages. When signal transduction occurs through CD40, specialized antigen-presenting cells are activated. The natural ligand of CD40 is named as CD154 or CD40L, and it is known to be expressed mainly on mature T lymphocytes. CD40L-mediated signal transduction can trigger some cellular biological events, including immune cell activation, proliferation, and the production of cytokines and chemokines. CD40 signaling is very important for T cell-dependent immune responses, especially in the context of tumor environment. CD40-stimulated dendritic cells can activate tumor-specific effector T cells, which have the potential to eradicate tumor cells.

The expression of CD40 can be found in many normal cells and tumor cells including B lymphocytes. For example, melanoma is a tumor that expresses CD40, and 30% to 70% of solid tumors also exhibit CD40 expression. At present, it is known that the activation of CD40 can effectively trigger anti-tumor responses (Tong et al., Cancer Gene Therapy, 2003, 10: 1-13), including immune activation of tumor-specific T cell responses, direct effect on the apoptosis of CD40-positive tumors, and stimulation-mediated humoral response of ADCC. It has been observed that the eradication of tumor is strongly correlated with the presence of tumor-specific cytotoxic T lymphocytes. At the same time, it should be noticed that systemic administration of CD40-antibody is associated with side effects, such as shock syndrome and cytokine release syndrome (van Mierlo et al., Proc. Natl. Acad. Sci. USA, 2002, 99: 5561-5566).

At present, many international pharmaceutical companies are developing monoclonal antibodies against CD40 as mentioned above, which specifically stimulate immune activation to maximize the patient's own immune system to respond to tumors, so as to achieve the purpose of killing tumor cells. Related patents involve such as PCT/CN2018/089252, CN1198647, CN1369015, CN1582165, CN100430419, CN101014386, CN101237882, CN101289510, CN101490086, CN103842382, CN104918957, WO2002028904, WO2011123489, WO2012149356, WO2013034904, WO201509853, WO2016196314, WO2017040932, WO2017004006, etc. So far, anti-CD40 antibodies available from Pfizer (related products have been licensed to Roche), Alligator and other companies have been observed having favorable tumor killing effects in preclinical animal models, and have entered Phase I clinical trials.

As for mutations in antibody constant region, WO2006019447, WO2014145806, US8734791, US9657106, US8084582, WO2008150494, WO2004099249 disclose mutations of S267E, L328F, and N325S of the antibody heavy chain. The mutations delete the binding ability of the antibody to FcγRIII, whereas enhance the binding ability to FcγRIIB. Therefore, the antibody-dependent cellular cytotoxicity (ADCC) is deleted, while FcγRIIB-mediated cross-linking is enhanced, thereby enhancing the activation of CD40 and enhancing the antigen-presentation by dendritic cells.

The present disclosure aims to provide an anti-CD40 antibody with high affinity, high selectivity, and high biological activity, which lacks of antibody-dependent cellular cytotoxicity (ADCC), but has enhanced FcγRIIB-mediated crosslinking, thereby inhibiting *in vivo* tumor growth. The antibodies of the present disclosure can be used as medicament or used in a composition for the treatment of cancers mediated by CD40 and mediated by CD40 pathway.

## SUMMARY OF THE INVENTION

The present disclosure provides a CD40 antibody or antigen-binding fragment thereof, which comprises:

a light chain variable region of the antibody, comprising at least one LCDR shown as a sequence selected from the group consisting of: SEQ ID NO: 6, SEQ ID NO: 7, SEQ ID NO: 8; SEQ ID NO: 14, SEQ ID NO: 15, SEQ ID NO: 16; SEQ ID NO: 42, SEQ ID NO: 43, SEQ ID NO: 44; SEQ ID NO: 50, SEQ ID NO: 51, SEQ ID NO: 52; SEQ ID NO: 58, SEQ ID NO: 59 and SEQ ID NO: 60; and/or
a heavy chain variable region of the antibody, comprising at least one HCDR shown as a sequence selected from the group consisting of: SEQ ID NO: 3, SEQ ID NO: 4, SEQ ID NO: 5, SEQ ID NO: 11, SEQ ID NO: 12, SEQ ID NO: 13; SEQ ID NO: 39, SEQ ID NO: 40, SEQ ID NO: 41; SEQ ID NO: 47, SEQ ID NO: 48, SEQ ID NO :49; SEQ ID NO:55, SEQ ID NO:56 and SEQ ID NO:57.

In some embodiments, the anti-CD40 antibody or antigen-binding fragment thereof as described above, comprises a light chain variable region comprising LCDR1 as shown in SEQ ID NO: 6, SEQ ID NO: 14, SEQ ID NO: 42, SEQ ID NO: 50, or SEQ ID NO: 58.

In some embodiments, the anti-CD40 antibody or antigen-binding fragment thereof as described above, comprises a light chain variable region of the antibody comprising LCDR2 as shown in SEQ ID NO: 7, SEQ ID NO: 15, SEQ ID NO: 43, SEQ ID NO: 51 or SEQ ID NO: 59.

In some embodiments, the anti-CD40 antibody or antigen-binding fragment thereof as described above, comprises a light chain variable region of the antibody comprising LCDR3 as shown in SEQ ID NO: 8, SEQ ID NO: 16, SEQ ID NO: 44, SEQ ID NO: 52 or SEQ ID NO: 60.

In some embodiments, the anti-CD40 antibody or antigen-binding fragment thereof as described above, comprises a heavy chain variable region of the antibody comprising HCDR1 as shown in SEQ ID NO: 3, SEQ ID NO: 11, SEQ ID NO: 39, SEQ ID NO: 47 or SEQ ID NO: 55.

In some embodiments, the anti-CD40 antibody or antigen-binding fragment thereof as described above, comprises a heavy chain variable region of the antibody comprising HCDR2 as shown in SEQ ID NO: 4, SEQ ID NO: 12, SEQ ID NO: 40, SEQ ID NO: 48 or SEQ ID NO: 56.

In some embodiments, the anti-CD40 antibody or antigen-binding fragment thereof as described above, comprises a heavy chain variable region of the antibody comprising HCDR3 as shown in SEQ ID NO: 5, SEQ ID NO: 13, SEQ ID NO: 41, SEQ ID NO: 49 or SEQ ID NO: 57.

In some embodiments, the anti-CD40 antibody or antigen-binding fragment thereof as described above, comprises a light chain variable region of the antibody comprising LCDR1, LCDR2 and LCDR3 as shown in SEQ ID NO: 6, SEQ ID NO: 7 and SEQ ID NO: 8, respectively.

In some embodiments, the anti-CD40 antibody or antigen-binding fragment thereof as described above, comprises a light chain variable region of the antibody comprising LCDR1, LCDR2 and LCDR3 as shown in SEQ ID NO: 14, SEQ ID NO: 15 and SEQ ID NO: 16, respectively.

In some embodiments, the anti-CD40 antibody or antigen-binding fragment thereof as described above, comprises a light chain variable region of the antibody comprising LCDR1, LCDR2 and LCDR3 as shown in SEQ ID NO: 42, SEQ ID NO: 43 and SEQ ID NO: 44, respectively.

In some embodiments, the anti-CD40 antibody or antigen-binding fragment thereof as described above, comprises a light chain variable region of the antibody comprising LCDR1, LCDR2 and LCDR3 as shown in SEQ ID NO: 50, SEQ ID NO: 51 and SEQ ID NO: 52, respectively.

In some embodiments, the anti-CD40 antibody or antigen-binding fragment thereof as described above, comprises a light chain variable region of the antibody comprising LCDR1, LCDR2 and LCDR3 as shown in SEQ ID NO: 58, SEQ ID NO: 59 and SEQ ID NO: 60, respectively.

In some embodiments, the anti-CD40 antibody or antigen-binding fragment thereof as described above, comprises a heavy chain variable region of the antibody comprising HCDR1, HCDR2 and HCDR3 as shown in SEQ ID NO: 3, SEQ ID NO: 4 and SEQ ID NO: 5, respectively.

In some embodiments, the anti-CD40 antibody or antigen-binding fragment thereof as described above, comprises a heavy chain variable region of the antibody comprising HCDR1, HCDR2 and HCDR3 as shown in SEQ ID NO: 11, SEQ ID NO: 12 and SEQ ID NO: 13, respectively.

In some embodiments, the anti-CD40 antibody or antigen-binding fragment thereof as described above, comprises a heavy chain variable region of the antibody comprising HCDR1, HCDR2 and HCDR3 as shown in SEQ ID NO: 39, SEQ ID NO: 40 and SEQ ID NO: 41, respectively.

In some embodiments, the anti-CD40 antibody or antigen-binding fragment thereof as described above, comprises a heavy chain variable region of the antibody comprising HCDR1, HCDR2 and HCDR3 as shown in SEQ ID NO: 47, SEQ ID NO: 48 and SEQ ID NO: 49, respectively.

In some embodiments, the anti-CD40 antibody or antigen-binding fragment thereof as described above, comprises a heavy chain variable region of the antibody comprising HCDR1, HCDR2 and HCDR3 as shown in SEQ ID NO: 55, SEQ ID NO: 56 and SEQ ID NO: 57, respectively.

In some particular embodiments, the anti-CD40 antibody or antigen-binding fragment thereofcomprises a light chain variable region of the antibody comprising:

LCDR1, LCDR2 and LCDR3 as shown in SEQ ID NO: 6, SEQ ID NO: 7 and SEQ ID NO: 8, respectively; or
LCDR1, LCDR2 and LCDR3 as shown in SEQ ID NO: 14, SEQ ID NO: 15 and SEQ ID NO: 16, respectively; or
LCDR1, LCDR2 and LCDR3 as shown in SEQ ID NO: 42, SEQ ID NO: 43 and SEQ ID NO: 44, respectively; or
LCDR1, LCDR2 and LCDR3 as shown in SEQ ID NO: 50, SEQ ID NO: 51 and SEQ ID NO: 52, respectively; or
LCDR1, LCDR2 and LCDR3 as shown in SEQ ID NO: 58, SEQ ID NO: 59 and SEQ ID NO: 60, respectively; and,
a heavy chain variable region of the antibody comprising:

HCDR1, HCDR2 and HCDR3 as shown in SEQ ID NO: 3, SEQ ID NO: 4 and SEQ ID NO: 5, respectively; or
HCDR1, HCDR2 and HCDR3 as shown in SEQ ID NO: 11, SEQ ID NO: 12 and SEQ ID NO: 13, respectively; or
HCDR1, HCDR2 and HCDR3 as shown in SEQ ID NO: 39, SEQ ID NO: 40 and SEQ ID NO: 41, respectively; or
HCDR1, HCDR2 and HCDR3 as shown in SEQ ID NO: 47, SEQ ID NO: 48 and SEQ ID NO: 49, respectively; or
HCDR1, HCDR2 and HCDR3 as shown in SEQ ID NO: 55, SEQ ID NO: 56 and SEQ ID NO: 57, respectively.

In some particular embodiments, the anti-CD40 antibody or antigen-binding fragment thereof can be any one selected from:

(1) the light chain variable region of the antibody comprises LCDR1, LCDR2 and LCDR3 as shown in SEQ ID NO: 6, SEQ ID NO: 7 and SEQ ID NO: 8, respectively; the heavy chain variable region of the antibody comprises HCDR1, HCDR2 and HCDR3 as shown in SEQ ID NO: 3, SEQ ID NO: 4 and SEQ ID NO: 5, respectively;

(2) the light chain variable region of the antibody comprises LCDR1, LCDR2 and LCDR3 as shown in SEQ ID NO: 14, SEQ ID NO: 15 and SEQ ID NO: 16, respectively; the heavy chain variable region of the antibody comprises HCDR1, HCDR2 and HCDR3 as shown in SEQ ID NO: 11, SEQ ID NO: 12 and SEQ ID NO:13, respectively;

(3) the light chain variable region of the antibody comprises LCDR1, LCDR2 and LCDR3 as shown in SEQ ID NO: 42, SEQ ID NO: 43 and SEQ ID NO: 44, respectively; the heavy chain variable region of the antibody comprises HCDR1, HCDR2 and HCDR3 as shown in SEQ ID NO: 39, SEQ ID NO:40 and SEQ ID NO:41, respectively;

(4) the light chain variable region of the antibody comprises LCDR1, LCDR2 and LCDR3 as shown in SEQ ID NO: 50, SEQ ID NO: 51 and SEQ ID NO: 52, respectively; the heavy chain variable region of the antibody comprises HCDR1, HCDR2 and HCDR3 as shown in SEQ ID NO: 47, SEQ ID NO:48 and SEQ ID NO:49, respectively; and

(5) the light chain variable region of the antibody comprises LCDR1, LCDR2 and LCDR3 as shown in SEQ ID NO: 58, SEQ ID NO: 59 and SEQ ID NO: 60, respectively; the heavy chain variable region of the antibody comprises HCDR1, HCDR2 and HCDR3 as shown in SEQ ID NO: 55, SEQ ID NO:56 and SEQ ID NO:57, respectively.

In some particular embodiments, the light chain variable region sequence of the antibody is selected from the group consisting of SEQ ID NO: 2 or SEQ ID NO: 10; the heavy chain variable region sequence is selected from SEQ ID NO: 1 or SEQ ID NO: 9.

The anti-CD40 antibody or antigen-binding fragment thereof described above can be a murine antibody or a chimeric antibody.

In some particular embodiments, the amino acid sequence of the heavy chain variable region of the murine antibody or the chimeric antibody is as shown in SEQ ID NO: 1, and the amino acid sequence of the light chain variable region is as shown in SEQ ID NO: 2.

In other particular embodiments, the amino acid sequence of the heavy chain variable region of the murine antibody or the chimeric antibody is as shown in SEQ ID NO: 9, and the amino acid sequence of the light chain variable region is as shown in SEQ ID NO: 10.

In other particular embodiments, the light chain variable region LCVR of the antibody or the antigen fragment is as shown in sequence SEQ ID NO: 38, and the heavy chain variable region HCVR is as shown in sequence SEQ ID NO: 37.

In other particular embodiments, the light chain variable region LCVR of the antibody or the antigen fragment is as shown in sequence SEQ ID NO: 46, and the heavy chain variable region HCVR is as shown in sequence SEQ ID NO: 45.

In other particular embodiments, the light chain variable region LCVR of the antibody or the antigen fragment is as shown in sequence SEQ ID NO: 54, and the heavy chain variable region HCVR is as shown in sequence SEQ ID NO: 53.

In some particular embodiments, the anti-CD40 antibody or antigen-binding fragment thereof is a murine antibody, a chimeric antibody, a humanized antibody, a human antibody or fragment(s) thereof.

In some particular embodiments, when the anti-CD40 antibody or antigen-binding fragment thereof is a murine antibody or fragment thereof, the light chain variable region of the antibody further comprises light chain FR region(s) or light chain constant region(s) of murine κ, λ chain or variant(s) thereof; and/or the heavy chain variable region of the antibody further comprises the heavy chain FR region(s) or heavy chain constant region(s) of murine IgG1, IgG2, IgG3, IgG4 or variant(s) thereof.

In some particular embodiments, when the anti-CD40 antibody or antigen-binding fragment thereof is a chimeric antibody or fragment thereof, it comprises the light chain constant region(s) of human κ, λ chain or variant(s) thereof, and/or comprises the heavy chain constant region(s) of human IgG1, IgG2, IgG3 or IgG4 or variant(s) thereof. In some particular embodiments, the light chain variable region sequence is as shown in SEQ ID NO: 2 or SEQ ID NO: 10, and/or the heavy chain variable region sequence is as shown in SEQ ID NO: 1 or SEQ ID NO: 9.

In some particular embodiments, when the anti-CD40 antibody or antigen-binding fragment thereof is a humanized antibody or fragment thereof, the light chain sequence of the antibody is: SEQ ID NO: 18 or SEQ ID NO: 20 or variant thereof; in particular, the variant has 0-10 amino acid change(s) in the light chain, more specifically, has amino acid mutation(s) at positions 2 and 3. The amino acids after mutation at positions 2 and 3 are each independently selected from I, V or L; the heavy chain sequence of the antibody is: SEQ ID NO: 17 or SEQ ID NO: 19 or variant thereof; the variant has 0-10 amino acid change(s) in the heavy chain, more specifically, has amino acid mutation(s) at positions 6 and 8. The amino acids after mutation are each independently selected from I, A or L.

In some particular embodiments of the anti-CD40 humanized antibody or fragment thereof as described above, the heavy chain variable region of the humanized antibody further comprises heavy chain constant region(s) or FR region(s) of human IgG1, IgG2, IgG3, IgG4 or variant(s) thereof, in particular comprises heavy chain constant region(s) or FR region(s) of human IgG1, IgG2 or IgG4, in particular comprises heavy chain constant region(s) or FR region(s) of human IgG1 or IgG2; and/or comprises light chain FR region(s) of human κ, λ chain or variant(s) thereof.

In some particular embodiments of the anti-CD40 humanized antibody or fragment thereof as described above, the light chain FR region sequence on the light chain variable region of the humanized antibody is derived from, for example, a human germline light chain IGkV1-33 as shown in sequence SEQ ID NO: 22; or derived from a human germline light chain IGkV2-28 as shown in sequence SEQ ID NO: 24.

In some particular embodiments of the anti-CD40 humanized antibody or fragment thereof as described above, the light chain variable region variant of the humanized antibody particularly has 0-10 amino acid change(s) in the light chain variable region; more particularly, has amino acid mutation(s) at positions 2 and 3; in particular, the amino acids after mutation are I, V or L.

In some particular embodiments, the anti-CD40 humanized antibody or the fragment thereof as described above further comprises a light chain constant region of a human kappa, lambda chain or variant thereof.

In some particular embodiments of the anti-CD40 humanized antibody or fragment thereof as described above, the heavy chain FR region sequence on the heavy chain variable region of the humanized antibody is derived from, for example, a human germline heavy chain IGHV1-69 as shown in sequence SEQ ID NO: 21, and/or derived from a human germline heavy chain IGkV1-33 as shown in sequence SEQ ID NO: 22; derived from a human germline heavy chain IGHV1-2 as shown in sequence SEQ ID NO: 23, and/or derived from a human germline heavy chain IGkV2-28 as shown in sequence SEQ ID NO: 24.

In some particular embodiments of the anti-CD40 humanized antibody or fragment thereof, the heavy chain variable region is selected from sequence as shown in one of SEQ ID NOs: 25-30 or variant thereof, and the light chain variable region is selected from sequence as shown in one of SEQ ID NOs: 31-36 or variant thereof.

In some particular embodiments of the anti-CD40 humanized antibody or fragment thereof, the heavy chain variable region is as shown in SEQ ID NO: 26 or variant thereof, and the light chain variable region is as shown in SEQ ID NO: 33 or variant thereof.

In some particular embodiments, the heavy chain variable region is as shown in SEQ ID NO: 30 or variant thereof, and the light chain variable region is as shown in sequence SEQ ID NO: 34 or variant thereof.

In some particular embodiments, the heavy chain of the humanized anti-CD40 antibody is as shown in SEQ ID NO: 17, and the light chain is as shown in SEQ ID NO: 18.

In some particular embodiments, the heavy chain is as shown in SEQ ID NO: 19, and the light chain is as shown in SEQ ID NO: 20.

In some particular embodiments of the anti-CD40 humanized antibody or fragment thereof, the humanized antibody heavy chain sequence is as shown in SEQ ID NO: 61, 62, 63, 64 or 67 or variant thereof, and/or the light chain variable region is as shown in SEQ ID NO: 18, 20 or variant thereof.

In some particular embodiments, the heavy chain sequence of the anti-CD40 humanized antibody or the fragment thereof is the sequence as shown in SEQ ID NO: 61 or 62 or variant thereof, and the light chain sequence is the sequence as shown in SEQ ID NO: 18 or variant thereof; the heavy chain sequence is the sequence as shown in SEQ ID NO: 63, 64 or 67 or variant thereof, and the light chain sequence is the sequence as shown in SEQ ID NO: 20 or variant thereof.

The variant has 0-10 amino acid change(s) in the heavy chain variable region, in particular has amino acid mutations at positions 6 and 8, and in particular the amino acids after mutation are I, A or L.

Herein, the sequence shown in SEQ ID NO: 61 comprises an amino acid residue mutated into glutamic acid (E) on position 266 corresponding to SEQ ID NO: 17 (e.g. S266E);

the sequence shown in SEQ ID NO: 62 comprises an amino acid residue mutated into glutamic acid (E) on position 266 corresponding to SEQ ID NO: 17 (e.g. S266E), an amino acid residue mutated into serine (S) on position 324 corresponding to SEQ ID NO: 17 (e.g., N324S), and an amino acid residue mutated into phenylalanine (F) on position 327 corresponding to SEQ ID NO: 17 (e.g., L327F);

the sequence shown in SEQ ID NO: 63 comprises an amino acid residue mutated into glutamic acid (E) on position 262 corresponding to SEQ ID NO: 19 (e.g. S262E);

the sequence shown in SEQ ID NO: 64 comprises an amino acid residue mutated into glutamic acid (E) on position 262 corresponding to SEQ ID NO: 19 (e.g. S262E), and an amino acid residue mutated into phenylalanine (F) on position 323 corresponding to SEQ ID NO: 19 (for example, L323F);

the sequence shown in SEQ ID NO: 67 comprises an amino acid residue mutated into glutamic acid (E) on position 262 corresponding to SEQ ID NO: 19 (e.g. S262E); an amino acid residue mutated into serine (S) on position 320 corresponding to SEQ ID NO: 19 (e.g., N320S), and an amino acid residue mutated into phenylalanine (F) on position 323 corresponding to SEQ ID NO: 19 (e.g., L323F). Among them, the numbering of amino acid position is in accordance with the natural order. In some embodiments, the amino acid at the last position (such as lysine) of the heavy chain amino acid sequence of the anti-CD40 antibody or antigen-binding fragment thereof described above is mutated into alanine (A).

In some particular embodiments, the amino acid at the last position of the heavy chain sequence as shown in SEQ ID NO: 61, 62, 63, 64, or 67 is mutated into A.

In other particular embodiments, an antibody is provided, which comprises a heavy chain as shown in SEQ ID NO: 69 and a light chain as shown in SEQ ID NO: 66.

In other particular embodiments, an antibody is provided, which comprises a heavy chain as shown in SEQ ID NO: 68 and a light chain as shown in SEQ ID NO: 66.

In some particular embodiments of the anti-CD40 antibody or antigen-binding fragment thereof as described above, the antigen-binding fragment is Fab, Fv, sFv, F(ab')$_2$, linear antibody, single-chain antibody, nanobody, domain antibody or multispecific antibody.

The present disclosure further provides a single-chain antibody, which comprises the heavy chain variable region and the light chain variable region of the anti-CD40 antibody or antigen-binding fragment thereof as described above.

The present disclosure further provides a multispecific antibody, which comprises the heavy chain variable region and the light chain variable region of the anti-CD40 antibody or antigen-binding fragment thereof as described above.

The present disclosure further provides a nucleic acid molecule (DNA or RNA) that encodes the anti-CD40 antibody or antigen-binding fragment thereof, multispecific antibody or single-chain antibody as described above.

The present disclosure further provides an expression vector comprising the nucleic acid molecule as described above.

The present disclosure further provides a host cell, which comprises or is transformed with the expression vector as described above. In some particular embodiments, the host cell is bacterium, yeast or mammalian cell, in particular *Escherichia coli*, *Pichia pastoris*, Chinese hamster ovary (CHO) cell or human embryonic kidney (HEK) 293 cell.

The present disclosure further provides an antibody-drug conjugate comprising the anti-CD40 antibody light chain variable region and/or heavy chain variable region as described above. The antibody-drug conjugate is well-known in the art, and is formed by connecting antibody, linker and drug. The known linkers involve cleavable linkers and non-cleavable linkers. For example, linkers involve but are not limited to SMCC, SPDP and the like. Drugs are also well-known in the art, such as DM1, DM4, MMAE, MMAF, etc.

The present disclosure further provides a pharmaceutical composition, which comprises the anti-CD40 antibody or antigen-binding fragment thereof, multispecific antibody or single-chain antibody, and pharmaceutically acceptable excipient(s), diluent(s) or carrier(s).

In some embodiments, the unit dose of the pharmaceutical composition may comprise 0.01% to 99% (by weight) of the anti-CD40 antibody or the fragment thereof, or the amount of the CD40 antibody or the fragment thereof in unit dose of the pharmaceutical composition(s) is from 0.1 mg to 2000 mg; in some embodiments, from 1 mg to 1000 mg.

The present disclosure further provides the use of the anti-CD40 antibody or antigen-binding fragment thereof, the multispecific antibody, the single-chain antibody or the pharmaceutical composition(s) comprising the same as described above, in the preparation of a medicament for the treatment of CD40-mediated or CD40L-mediated diseases or conditions; in particular the disease is cancer; in particular the cancer is selected from the group consisting of lymphoma, breast cancer, ovarian cancer, prostate cancer, pancreatic cancer, kidney cancer, lung cancer, liver cancer, gastric cancer, colorectal cancer, bladder cancer, rhabdomyosarcoma, esophageal cancer, cervical cancer, multiple myeloma, leukemia, gallbladder cancer, glioblastoma and melanoma.

The present disclosure further provides a method for treating and preventing CD40- or CD40L-mediated diseases or conditions, the method comprising contacting a subject with a prophylactically effective amount or a therapeutically effective amount of the anti-CD40 antibody or antigen-binding fragment thereof, the multispecific antibody, the single-chain antibody or the pharmaceutical composition(s) thereof as described above; in particular the disease or condition is cancer; in particular the cancer is selected from the group consisting of lymphoma, breast cancer, ovarian cancer, prostate cancer, pancreatic cancer, kidney cancer, lung cancer, liver cancer, gastric cancer, colorectal cancer, bladder cancer, rhabdomyosarcoma, esophageal cancer, cervical cancer, multiple myeloma, leukemia, gallbladder cancer, glioblastoma and melanoma.

The present disclosure further provides the use of the anti-CD40 antibody or antigen-binding fragment thereof, the multispecific antibody, the single-chain antibody or the pharmaceutical composition(s) thereof as described above in the preparation of a medicament for improving the symptom(s) of a patient suffering from autoimmune diseases.

The present disclosure further provides the use of the anti-CD40 antibody or antigen-binding fragment thereof, the multispecific antibody, the single-chain antibody or the pharmaceutical composition(s) thereof as described above in the preparation of a medicament for improving the symptom(s) of a patient suffering from inflammatory diseases.

## BRIEF DESCRIPTION OF THE DRAWINGS

Figure 1 shows the activating effect of the murine anti-human CD40 antibodies on DC cells based on the CD80 activating molecule.

Figure 2 shows the activating effect of the murine anti-human CD40 antibodies on DC cells based on the CD86 activating molecule.

Figure 3 shows the tumor growth curve of Raji transplanted lymphoma, after co-transplanting Raji transplanted lymphoma with human PBMC and DC cells.

Figure 4 shows the body weight change curve of NOG mice, after co-transplanting Raji transplanted lymphoma with human PBMC and DC cells into NOG mice.

Figure 5 shows the tumor growth curve after intraperitoneal injection of a single dose of the anti-CD40 antibodies into mouse model of MC38 colon cancer.

Figure 6 shows the activating effect of the anti-CD40 antibodies having mutation(s) in the heavy chain constant region on DC cells.

Figure 7A and Figure 7B show that the anti-CD40 antibodies having mutation(s) in the heavy chain constant region activate DC cells and promote cytokine production.

## DETAILED DESCRIPTION OF THE INVENTION

### 1. TERMS

In order to more readily understand the present disclosure, certain technical and scientific terms are in particular defined below. Unless clearly indicated elsewhere in the document, all other technical and scientific terms used herein have the meaning commonly understood by one of ordinary skilled in the art to which the present disclosure pertains.

As used herein, the three-letter code and the single-letter code for amino acids are as described in J. Biol. Chem, 243, (1968) p3558.

As used herein, "antibody" refers to immunoglobulin, a structure of four-peptide chains connected together by disulfide bonds between two identical heavy chains and two identical light chains. Different immunoglobulin heavy chain constant regions exhibit different amino acid compositions and sequence orders, hence present different kinds of antigenicity. Accordingly, immunoglobulins can be divided into five categories, or called as immunoglobulin isotypes, namely IgM, IgD, IgG, IgA and IgE; their corresponding heavy chains are μ chain, δ chain, γ chain, α chain and ε chain, respectively. According to its amino acid composition of hinge region and the number and location of heavy chain disulfide bonds, the same type of Ig can be divided into different sub-categories, for example, IgG can be divided into IgG1, IgG2, IgG3, and IgG4. Light chains can be divided into κ or λ chain, due to different constant regions. Each IgG among the five types has κ or λ chain.

In the present disclosure, the antibody light chain described herein further comprises a light chain constant region, which comprises a human or murine κ, λ chain or variant(s) thereof.

In the present disclosure, the antibody heavy chain described herein further comprises a heavy chain constant region, which comprises human or murine IgG1, IgG2, IgG3, IgG4 or variant(s) thereof.

The sequence of about 110 amino acids close to the N-terminus of the antibody heavy and light chains, is highly variable, known as variable region (V region); the rest sequence of amino acid close to the C-terminus is relatively stable, known as constant region (C region). Variable region comprises three hypervariable regions (HVRs) and four relatively conserved framework regions (FRs). The three hypervariable regions determine the specificity of the antibody, also known as complementarity determining regions (CDRs). Each light chain variable region (VL) and each heavy chain variable region (VH) is composed of three CDRs and four FRs, with sequential order from the amino terminus to the carboxyl terminus being: FR1, CDR1, FR2, CDR2, FR3, CDR3, and FR4. The three light chain CDRs refer to LCDR1, LCDR2, and LCDR3; and the three heavy chain CDRs refer to HCDR1, HCDR2 and HCDR3.

The term "antigen-presenting cell" or "APC" is a cell which displays a foreign antigen to form a complex with MHC on its surface. T cells recognize this complex using the T cell receptor (TCR). Examples of APCs include, but are not limited to, dendritic cells (DC), peripheral blood mononuclear cells (PBMC), monocytes, B lymphoblasts and monocyte-derived dendritic cells (DC). The term "antigen presentation" refers to the process during which APCs capture antigens and make them to be recognized by T cells, for example as a component of MHC-I/MHC-II conjugates.

The term "CD40" includes any variant or isoform of CD40 that is naturally expressed by a cell. The antibodies of the present disclosure can be cross-reactive with CD40 from non-human species. Alternatively, the antibodies may also be specific for human CD40 and may not exhibit cross-reactivity with other species. CD40 or any variant or isoform thereof can be isolated from cells or tissues in which they are naturally expressed, or produced by recombinant techniques using common techniques in the art and those described herein. Preferably, the anti-CD40 antibodies target human CD40 having normal glycosylation pattern.

The term "murine antibody" in the present disclosure refers to a monoclonal antibody against human CD40 prepared according to the knowledge and skills in the art. During the preparation, the test subject is injected with CD40 antigen, and then the hybridoma expressing the antibody showing desired sequences or functional features is isolated. In a preferred embodiment of the present disclosure, the murine CD40 antibody or antigen-binding fragment thereof may further comprise light chain constant region of murine κ, λ chain or variant thereof, or further comprise heavy chain constant region of murine IgG1, IgG2, IgG3 or IgG4 or variant thereof.

The term "human antibody" includes antibodies having variable and constant regions of human germline immunoglobulin sequences. Human antibodies of the present disclosure can include amino acid residues that are not encoded by human germline immunoglobulin sequences (such as mutations introduced by random or site-specific mutagenesis *in vitro* or by somatic mutation *in vivo*). However, the term "human antibody" does not include such antibodies in which CDR sequences derived from another mammalian species germline, such as a mouse, have been grafted into human framework sequence (i.e. "humanized antibody").

The term "humanized antibody", also known as CDR-grafted antibody, refers to an antibody generated by grafting non-human CDR sequences into a variable region framework of a human antibody. Humanized antibody overcomes the strong immune response induced by the chimeric antibody that carries a large amount of heterologous protein components. To avoid the decrease in activity caused by reducing the immunogenicity, the variable region of the antibody is subjected to minimum back-mutation to maintain the activity.

The term "chimeric antibody", is an antibody which is formed by fusing the variable region of a first species (such as murine) antibody with the constant region of another species (such as human) antibody, so as to alleviate the heterologous antibody-induced immune response. To establish a murine-human chimeric antibody, a hybridoma secreting specific murine monoclonal antibody is first established, variable region genes are then cloned from murine hybridoma cells, and then constant region genes of human antibody are cloned, the murine variable region genes are ligated with human constant region genes to form a chimeric gene which can be inserted into a human vector, and finally the chimeric antibody molecule is expressed in a eukaryotic or prokaryotic industrial system. The constant region of human antibody is selected from the heavy chain constant region derived from human IgG1, IgG2, IgG3 or IgG4 or variant(s) thereof; and preferably comprises heavy chain constant region derived from human IgG1 or IgG2.

The term "antigen-binding fragment", referred to as antigen-binding fragments of an antibody or antibody analogs, usually comprises at least part of the antigen-binding regions or variable regions (for example, one or more CDRs) of a parental antibody. Antibody fragments retain at least partial binding specificity of the parent antibody. Generally, when the activity is expressed in mole, the antibody fragment retains at least 10% of the parent binding activity. Preferably, the antibody fragment retains at least 20%, 50%, 70%, 80%, 90%, 95% or more binding affinity of the parent antibody to the target. Examples of antigen-binding fragments include, but are not limited to: Fab, Fab', F(ab')$_2$, Fv fragment, linear antibody, single-chain antibody, nanobody, domain antibody, and multispecific antibody. Engineered variants of antibody are reviewed in Holliger and Hudson (2005) Nat. Biotechnol. 23: 1126-1136.

The "Fab fragment" consists of one light chain and one CH1 and variable region of heavy chain. The heavy chain of a Fab molecule cannot form a disulfide bond with another heavy chain molecule.

The "Fc" region comprises two heavy chain fragments having CH2 and CH3 domains of the antibody. The two heavy chain fragments are held together by two or more disulfide bonds and also by hydrophobic interaction of CH3 domain.

The "F(ab')$_2$ fragment" comprises two light chains and two heavy chains comprising the portion of constant region between CH1 and CH2 domains, thereby forming an inter-chain disulfide bond between the two heavy chains. Therefore, F(ab')$_2$ fragment is composed of two Fab' fragments held together by disulfide bond between the two heavy chains.

The "Fv region" comprises variable regions from both heavy and light chains, but lacks the constant region.

The term "multispecific antibody" is used in its broadest sense to encompass antibodies having multi-epitope specificity. These multispecific antibodies involve, but are not limited to, antibodies comprising a heavy chain variable region (VH) and a light chain variable region (VL), wherein the VH-VL unit has multi-epitope specificity; antibodies having two or more VL and VH regions, each VH-VL unit binding to different target or different epitope of the same target; antibodies having two or more single variable regions, each single variable region binding to different target or different epitope of the same target; full length antibodies, antibody fragments, diabodies, bispecific diabodies and triabodies, antibody fragments that have been covalently or non-covalently linked, and the like.

In the context of this application, when referring to a mutation position of the heavy chain constant region, the term "position(s) n corresponding to SEQ ID NO: m" or "position(s) n of SEQ ID NO: m" means: in different antibody numbering systems, a mutation site is comparable or equivalent to position n of SEQ ID NO: m, in terms of position. The skilled persons know that current antibody numbering systems include but are not limited to EU, Kabat, Chothia, IMGT (Lefranc, 2003) and AHo (Honegger and Plückthun, 2001) and so on. When a certain position is defined as position "n" according to one numbering system, it may be defined as position n' according to another numbering system. The skilled persons can easily determine the corresponding relationship between specific sites according to different numbering systems (for example, EU numbering) based on common knowledge.

The term "antibody-drug conjugate" (ADC) refers to one or more chemically synthesized molecules (including but not limited to cytotoxic agents) conjugated to an antibody or an antibody fragment.

The term "single-chain antibody" is a single-chain recombinant protein linked by a linker peptide between the heavy chain variable region (VH) and the light chain variable region (VL) of an antibody. It is the smallest antibody fragment with complete antigen binding sites.

The term "domain antibody fragment" is an immunoglobulin fragment with immunological function, which only comprises a heavy chain variable region or a light chain variable region chain. In some cases, two or more VH regions are covalently linked to a peptide linker to form a bivalent domain antibody fragment. Two VH regions of the bivalent domain antibody fragment can target the same or different antigens.

The term "binding to CD40" in the present disclosure refers to the ability to interact with human CD40. The term "antigen-binding site(s)" in the present disclosure refers to a discrete three-dimensional spatial site on an antigen that can be recognized by the antibody or the antigen-binding fragment of present disclosure.

The term "epitope" refers to a site on an antigen that is specifically bound by an immunoglobulin or antibody. Epitopes may be formed from adjacent amino acids or nonadjacent amino acids but juxtaposed by tertiary folding of protein. Epitopes formed from adjacent amino acids are typically retained after exposure to denaturing solvent; however epitopes formed via tertiary folding are typically lost after treatment with denaturing solvent. Epitopes usually have a unique spatial conformation, including at least 3 to 15 amino acids. Methods for determining which epitope is bound by a given antibody are well known in the art, including immunoblotting and immunoprecipitation assays and the like. Methods for determining the spatial conformation of an epitope include techniques in the art and techniques described herein, such as X-ray crystallography and two-dimensional nuclear magnetic resonance and the like.

As used in the present disclosure, the terms "specifically bind" and "selectively bind" refer to the binding of an antibody to an epitope on a predetermined antigen. Typically, when human CD40 is used as an analyte and an antibody is used as a ligand, the antibody binds to a predetermined antigen at an equilibrium dissociation constant ($K_D$) of less than about $10^{-7}$ M or even less, as measured by surface plasmon resonance (SPR) techniques in an instrument, and the affinity of the antibody for binding to a predetermined antigen is at least twice higher than that for binding to a non-specific antigen other than the predetermined antigen or closely related antigen (such as BSA, etc). The term "antibody recognizing an antigen" can be used interchangeably herein with the term "antibody specifically binding to".

The term "cross-reactivity" refers to the ability of an antibody of the present disclosure to bind to CD40 from a different species. For example, an antibody of the present disclosure that binds to human CD40 can also bind to CD40 from another species. Cross-reactivity is measured by detecting specific reactivity with purified antigens in binding assays (e.g., SPR and ELISA), or by detecting the binding or functional interactions with cells that express CD40 physiologically. Methods for determining cross-reactivity include standard binding assays as described herein, such as surface plasmon resonance (SPR) analysis, or flow cytometry.

The term "inhibiting" or "blocking" can be used interchangeably and encompasses both partial and complete inhibition/blocking. Preferably, the inhibition/blocking of a ligand can reduce the normal level or alter the type of activity when ligand binding occurs without inhibition or blocking. Inhibition and blocking are also intended to include any measurable decrease of ligand-binding affinity when contacted with an anti-CD40 antibody, compared to that when not contacted with an anti-CD40 antibody.

The term "inhibiting growth" (e.g., when referring to cells) is intended to include any measurable decrease in cell growth.

The terms "inducing an immune response" and "enhancing an immune response" are used interchangeably and refer to the stimulation (i.e., passive or adaptive) of an immune response to a particular antigen. In the context of CDC or ADCC, the term "induction" refers to the stimulation of particular direct cytotoxic mechanism.

As used in present disclosure, the term "ADCC", namely antibody-dependent cell-mediated cytotoxicity, refers to that the cells expressing Fc receptors directly kill the target cells coated by an antibody by recognizing the Fc segment of the antibody. ADCC effector function of the antibody can be reduced or eliminated by modifying the Fc segment of IgG. The modification refers to mutations in antibody heavy chain constant region, such as mutations selected from N297A, L234A, L235A in IgG1; IgG2/4 chimera; or F235E or L234A/E235A mutation in IgG4.

Methods for producing and purifying antibodies and antigen-binding fragments are well known in the art and can be found, for example, in Antibody Experimental Technology Guide of Cold Spring Harbor, Chapters 5-8 and 15. For example, mice can be immunized with human CD40, or fragments thereof and the resulting antibodies can then be re-natured, purified and sequenced by using conventional methods well known in the art. Antigen-binding fragments can also be prepared by conventional methods. The antibody or the antigen-binding fragment of the present disclosure is genetically engineered to introduce one or more human framework regions (FRs) to a non-human derived CDR. Human FR germline sequences can be obtained from ImMunoGeneTics (IMGT) via their website http://imgt.cines.fr, or from The Immunoglobulin FactsBook, 2001ISBN012441351.

The engineered antibody or antigen-binding fragment of the present disclosure may be prepared and purified using conventional methods. For example, cDNA sequence encoding the corresponding antibody may be cloned and recombined into a GS expression vector. The recombinant immunoglobulin expression vector may then stably transfect CHO cells. As a more recommended method well known in the art, mammalian expression system will result in glycosylation of antibody, typically at the highly conserved N-terminus in the FC region. Stable clones are obtained through expression of an antibody specifically binding to human antigen. Positive clones may be expanded in a serum-free culture medium for antibody production in bioreactors. Culture medium, into which an antibody has been secreted, may be purified and collected by conventional techniques. The antibody may be filtered and concentrated using common techniques. Soluble mixture and aggregate may be effectively removed by common techniques, including size exclusion or ion exchange. The obtained product may be immediately frozen, for example at -70°C, or may be lyophilized.

The antibody of the present disclosure refers to a monoclonal antibody. The monoclonal antibody (mAb) of the present disclosure refers to an antibody obtained from a single clone of cell strain, and the cell strain is not limited to a eukaryotic, a prokaryotic or a phage clonal cell strain. Monoclonal antibodies or antigen-binding fragments can be obtained recombinantly using, for example, hybridoma techniques, recombinant technique, phage display technique, synthetic technique (e.g., CDR-grafting), or other techniques in the prior art.

"Administration", "administering" and "treatment," as applied to an animal, human, experimental subject, cell, tissue, organ, or biological fluid, refer to contacting an exogenous pharmaceutical, therapeutic agent, diagnostic agent, or composition with the animal, human, subject, cell, tissue, organ, or biological fluid. "Administration", "administering" and "treatment" can refer, e.g., to therapeutic, pharmacokinetic, diagnostic, research, and experimental methods. Treatment of a cell encompasses contacting a reagent with the cell, as well as contacting a reagent with a fluid, wherein the fluid is in contact with the cell. "Administration", "administering" and "treatment" also means *in vitro* and *ex vivo* treatments, e.g., of a cell, by a reagent, diagnostic, composition, or by another cell. "Treatment" as it applies to a human, veterinary, or a subject to be studied, refers to therapeutic treatment, prophylactic or preventative measures, research and diagnostic applications.

"Treat" means to administer a therapeutic agent, such as a composition comprising any of the antibodies or antigen-binding fragment thereof in the present disclosure, internally or externally to a subject having one or more disease symptoms for which the agent has known therapeutic activity. Typically, the agent is administered in an amount effective to alleviate one or more disease symptoms in the treated subject or cohort of subjects, regardless of by inducing the regression of such symptom(s) or by inhibiting the progression to any clinically un-measurable degree.

The amount of a therapeutic agent that is effective to alleviate any particular disease symptom (also referred to "therapeutically effective amount") may vary according to factors such as the disease state, age, and weight of the subject, and the ability of the agent to elicit a desired response in the subject. Whether a disease symptom has been alleviated can be assessed by any clinical measurement typically used by physicians or other skilled healthcare providers to assess the severity or progression status of that symptom. Even if an embodiment of the present disclosure (e.g., a treatment method or article of manufacture) is not effective in alleviating the disease symptom(s) of interest in every subject, it does alleviate the target disease symptom(s) of interest in a statistically significant number of subjects, as determined by any statistical test known in the art (such as, the Student's t-test, the chi-square test, the U-test according to Mann and Whitney, the Kruskal-Wallis test (H-test), Jonckheere-Terpstra-test and the Wilcoxon-test).

"Conservative modification" or "conservative substitution or replacement" means that an amino acid with similar characteristics (such as charge, side chain size, hydrophobicity/hydrophilicity, main chain conformation and rigidity, etc.) can be used to replace an amino acid in a protein; such substitution can be frequently performed without changing the biological activity of the protein. Those skilled in the art know that, generally speaking, substitution for a single amino acid in a non-essential region of a polypeptide does not substantially change the biological activity (see, for example, Watson et al. (1987) Molecular Biology of the Gene, The Benjamin/Cummings Pub. Co., page 224, 4th edition). In addition, the substitution for amino acid having similar structure or function is unlikely to disrupt biological activity. The common conservative substitutions of amino acids are as follows:

| Original residue | Exemplary substituents | Preferred substituents |
| --- | --- | --- |
| Ala (A) | Val, Leu, Ile | Val |
| Arg (R) | Lys, Gln, Asn | Lys |
| Asn (N) | Gln, His, Asp, Lys, Arg | Gln |
| Asp (D) | Glu, Asn | Glu |
| Cys (C) | Ser, Ala | Ser |
| Gln (Q) | Asn, Glu | Asn |
| Glu (E) | Asp, Gln | Asp |
| Gly (G) | Ala | Ala |
| His (H) | Arg, Asn, Gln, Lys | Arg |
| Ile (I) | Leu, Val, Met, Ala, Phe, Norleucine | Leu |
| Leu (L) | Ile, Norleucine, Val, Met, Ala, Phe | Ile |
| Lys (K) | Arg, Gln, Asn | Arg |
| Met (M) | Leu, Phe, Ile | Leu |
| Phe (F) | Tyr, Leu, Val, Ile, Ala | Tyr |
| Pro (P) | Ala | Ala |
| Ser (S) | Thr | Thr |
| Thr (T) | Ser | Ser |
| Trp (W) | Tyr, Phe | Tyr |
| Tyr (Y) | Phe, Trp, Thr, Ser | Phe |
| Val (V) | Leu, Ile, Met, Phe, Ala, Norleucine | Leu. |

"Effective amount" involves an amount sufficient to ameliorate or prevent a symptom or sign of a medical condition. Effective amount also means an amount sufficient to allow or to facilitate diagnosis. An effective amount for a particular subject or veterinary subject may vary depending on factors such as the condition being treated, the general health of the subject, the route and dose of administration and the severity of side effects. An effective amount can be the maximal dose or dosing regimen that avoids significant side effects or toxic effects.

"Exogenous" refers to substances that are produced outside an organism, cell, or human body, depending on the background.

"Endogenous" refers to substances that are produced inside an organism, cell, or human body, depending on the background.

"Homology" refers to the sequence similarity between two polynucleotide sequences or between two polypeptides. When the positions in the two compared sequences are occupied by the same base or amino acid residue (for example, if each position of two DNA molecules is occupied by adenine), then the molecules are deemed to be homologous at that position. The percent homology between two sequences is a function of the number of matching or homologous positions shared by two sequences divided by the number of all positions to be compared × 100%. For example, in an optimal sequence alignment, if 6 of the 10 positions in two sequences match with each other or are homologous, then the two sequences will be deemed as 60% homologous. Generally speaking, the comparison is performed, when two sequences are aligned to obtain the optimal percentage of homology.

As used herein, the expressions "cell," "cell line," and "cell culture" are used interchangeably and all such designations include its progeny. Thus, the term "transformed cell" refers to the primary subject cell and cultures derived therefrom without considering the number of passages. It is also understood that all progeny may not be precisely identical in the aspect of DNA component and/or content, due to deliberate or indeliberate mutations. Mutant progeny that have the same function or biological activity as that of original cell are also covered by this term.

"Optional" or "optionally" means that the event or situation that follows may but not necessarily occur, and the description includes the instances in which the event or circumstance occurs or does not occur. For example, "optionally comprises 1 to 3 antibody heavy chain variable region(s)" means the antibody heavy chain variable region with specific sequence can be, but not necessarily, present.

## EXAMPLES

The following examples are used to further describe the present invention, but these examples do not limit the scope of the present invention. The experimental methods that do not specify specific conditions in the examples of the present invention usually follow conventional conditions, such as Antibodies: A Laboratory Manual, Molecular Cloning Manual from Cold Spring Harbor; or according to the conditions recommended by the manufacturer of materials or products. The reagents for which the sources are not specifically indicated are conventional reagents commercially available.

## EXAMPLE 1 THE SEQUENCES AND PREPARATION OF IMMUNE-ANTIGEN AND SCREENING-ANTIGEN

His-tagged human CD40 (h-CD40-his) recombinant protein, Fc-tagged human CD40 (h-CD40-Fc) recombinant protein, His-tagged mouse CD40 (m-CD40-his) recombinant protein and His-tagged rhesus monkey CD40 (rhesus-CD40-his) recombinant protein (#CD0-C52H7) were all purified commercial protein reagents purchased from Acrobiosystems, and the each sequence source is shown in Table 1. The protein reagents can be used in each test of the following examples.

Table 1. Sources for amino acid sequences of recombinant proteins

| Name | Amino acid sequence (from the beginning to the end) | Genbank accession No. |
|---|---|---|
| h-CD40-his | Glu21-Arg193 | AAH12419.1 |
| h-CD40-Fc | Glu21-Arg193 | NP_001241.1 |
| m-CD40-his | Val24-Arg193 | P27512 |
| rhesus-CD40-his | Glu21-Arg193 | NP_001252791.1 |

## EXAMPLE 2 PREPARATION OF ANTIBODY HYBRIDOMA

Anti-human CD40 monoclonal antibody was produced by immunizing mice. Laboratory C57BL/6 mouse: female, 6 to 8 week-old (JOINN Laboratories (Suzhou) New Medicament Research Center Co., Ltd., animal production license number: 201503259). Breeding environment: SPF level.

After the mice being purchased, they were kept in a laboratory environment for 1 week, adjusted to 12/12 hours light/dark cycle; at a temperature of 20-25°C; with humidity of 40-60%. The adapted mice were assigned into 2 cages, 5 in each cage.

The immune-antigen is a modified human-CD40 recombinant protein with an Fc tag (h-CD40-Fc, prepared in a phosphate buffer solution, at 1 μg/μl). Emulsification was performed with Freund's adjuvant (Sigma, Lot No.: F5881/F5506): Freund's complete adjuvant (CFA) for the first emulsification; and nucleic acid adjuvant (CpG, Sangon Biotech) and injectable aluminum (Imject Alum, Thermo, Lot No.: PH203866) for the rest booster immunizations. The immunization date was on day 0, 14, 28, 42, 56, and 70. Blood was collected for blood test on day 21, 35, 49, 63, and 77. The mouse serum was detected by ELISA method to determine the antibody titer in the mouse serum.

After the fourth immunization, mouse with a high and stable antibody titer was selected for spleen cell fusion. 3 days before fusion, booster immunization was performed by intraperitoneal (IP) injection of 10 μg/mouse of antigen formulated in phosphate buffer solution. Optimized PEG-mediated fusion steps were used to fuse splenic lymphocytes and myeloma cells Sp2/0 cells (ATCC® CRL-8287™) to obtain hybridoma cells, and five monoclonal hybridoma cell lines showing favorable *in vitro* activity were selected.

**EXAMPLE 3 ELISA BINDING ASSAY**

ELISA assay was used to detect the binding properties of anti-CD40 antibodies. CD40 recombinant protein was directly coated with his tag. After the antibody was added, a secondary antibody (HRP-conjugated anti-Fc antibody) and HRP substrate TMB were added to detect the binding activity of the antibody to the antigen.

Human or rhesus monkey CD40-his protein was coated onto a 96-well micro-titer plate, 100 $\mu$l per well at a concentration of 0.5 $\mu$g/mL, and incubated overnight at 4°C. The plate was washed with washing buffer for three times, 250 $\mu$l per well. The plate was shaken for 10 seconds during each washing to ensure sufficient washing. 200 $\mu$l/well blocking solution was added and incubated at room temperature for 2 hours. The plate was washed with washing buffer for three times, 250 $\mu$l per well. The plate was shaken for 10 seconds during each washing to ensure sufficient washing. 100 $\mu$l of anti-CD40 antibody to be tested diluted with diluent was added into each well and incubated for 1 hour at room temperature. The plate was washed with washing buffer for three times, 250 $\mu$l per well. 100 $\mu$l of HRP-labeled goat anti-human IgG secondary antibody diluted at 1:20000 with a diluent was added to each well, and incubated for 1 hour at room temperature. The plate was washed with washing buffer for three times, 250 $\mu$l per well. 100 $\mu$l TMB was added to each well, and reaction was performed for 15 minutes in the dark. 50 $\mu$l of 0.16M sulfuric acid was added into each well. Thermo MultiSkanFc microplate reader was used to read OD value under 450nm, and EC50 value for each CD40 antibody to binding to CD40 was calculated.

Table 2. Results of ELISA binding assay of murine hybridoma antibodies against CD40 from different germ lines

| Antibody strain | ELISA EC50 (ng/mL) | | |
|---|---|---|---|
| | hCD40-his | Rhesus CD40-his | murine CD40-his |
| 1D9 | 10.01 | 9.808 | no binding |
| 2H6 | 7.063 | 7.207 | no binding |
| 9E5 | 5.996 | 6.704 | no binding |
| 14C10 | 8.808 | 9.494 | no binding |
| 38B4 | 12.9 | 11.81 | no binding |

**EXAMPLE 4 TEST OF ANTI-CD40 ANTIBODY BLOCKING THE BINDING BETWEEN CD40 AND CD40L**

In this test, through an *in vitro* blocking assay, the anti-human CD40 antibodies thus screened were tested for their blocking the binding between human CD40 and human CD40L.

The particular method was as follows: the Fc-tagged CD40 recombinant protein (h-CD40-Fc) was coated onto a 96-well micro-titer plate, anti-CD40 antibody was added to fully bind to and occupy the epitopes, and then his-tagged CD40L was added, and His tag was detected to calculate the amount of CD40 binding to CD40L, and the IC50 value for CD40 antibody to block the CD40 activity sites was calculated.

Human CD40-Fc protein was coated onto a 96-well micro-titer plate, 100 $\mu$l per well at a concentration of 1 $\mu$g/mL, and incubated overnight at 4°C. The plate was washed with washing buffer for three times, 250 $\mu$l per well. The plate was shaken for 10 seconds during each washing to ensure sufficient washing. 200 $\mu$l/well blocking solution was added and incubated at room temperature for 2 hours. The plate was washed with washing buffer for three times, 250 $\mu$l per well. The plate was shaken for 10 seconds during each washing to ensure sufficient washing. 100 $\mu$l of anti-CD40 antibody to be tested diluted with diluent was added into each well and incubated for 1 hour at room temperature. The plate was washed with washing buffer for three times, 250 $\mu$l per well. 100$\mu$l of diluted CD40L-his was added into each well, and incubated for 1 hour at room temperature. The plate was washed with washing buffer for three times. 100 $\mu$l of HRP-labeled anti-his tag secondary antibody diluted at 1:2000 with a diluent was added to each well, and incubated for 1 hour at room temperature. The plate was washed with washing buffer for three times, 250 $\mu$l per well. 100 $\mu$l TMB was added to each well, and reaction was performed for 15 minutes in the dark. 50 $\mu$l of 0.16M sulfuric acid was added into each well. Thermo MultiSkanFc microplate reader was used to read OD value under 450nm, and IC50 value for CD40 antibody to block the binding of CD40 to CD40L was calculated.

Table 3. Results of ELISA assay for blocking the binding of human hCD40/hCD40L

| Antibody strain | IC50 ($\mu$g/mL) |
|---|---|
| 1D9 | 0.2634 |
| 2H6 | 0.2682 |

(continued)

| Antibody strain | IC50 ($\mu$g/mL) |
|---|---|
| 9E5 | 0.2787 |
| 14C10 | 0.3001 |
| 38B4 | 0.2934 |

## EXAMPLE 5 DETERMINATION OF AFFINITY BY BIACORE

According to the method described in the instruction available from Human Anti-capture Kit (Cat.# BR-1008-39, GE), human anti-capture antibody was covalently coupled to biosensing chip CM5 of the Biacore instrument (Biacore X100, GE), a certain amount of chimeric or humanized antibodies to be tested was affinity-captured, and then a series of concentration gradients of CD40 antigen (CD40 antigen purchased from Acrobiosystems) flowed through the surface of the chip. Biacore instrument (Biacore X100, GE) was used to detect the reaction signal in real-time, thus to obtain the association and dissociation curves. After each cycle of dissociation was completed, the biochip was washed and regenerated with a regeneration solution provided by the Human Anti-capture Kit. The amino coupling kit used in the test was purchased from GE (Cat. # BR-1000-50, GE), and HBS-EP + 10$\times$ buffer solution (Cat. # BR-1006-69, GE) was diluted to 1$\times$ (pH 7.4) with double distilled water.

The data obtained from the test was fitted against a (1:1) Binding model using BiacoreX100 evaluation software2.0 GE, and the affinity value was obtained, as shown in Table 10 and Table 11.

## EXAMPLE 6 TEST OF ACTIVITY OF ANTI-CD40 ANTIBODY ON REPORTER GENE IN CELLS

HEK-Blue CD40L cells were purchased from Invivogen (Cat#hkb-cd40). The cells were stably transfected with human CD40 gene and NF-kB-mediated SEAP genome. SEAP secreted in the supernatant can be detected by SEAP substrate QUANTI-Blue, to characterize the activation level of CD40 signaling pathway. In this test, the activation of HEK-Blue CD40L cells was detected, and the *in vitro* activity of CD40 antibodies was evaluated in cell according to EC50.

The HEK-Blue CD40L cells were cultivated in DMEM medium comprising 10% FBS, 100 $\mu$g/mL Zeocin and 30 $\mu$g/mL Blasticidin, and sub-cultured for 2 to 3 times per week at a passage ratio of 1:5 or 1:10. During sub-culturing, the medium was removed, the cell layer was rinsed with 5 mL of 0.25% trypsin, then the trypsin was removed, the cells were incubated in an incubator for 3 to 5 minutes, and then fresh medium was added to re-suspend the cells. 100 $\mu$L of cell suspension was added to a 96-well cell culture plate at a density of $5 \times 10^5$ cells/mL. The medium was DMEM comprising 10% FBS, 100$\mu$g/mL bleomycin Zeocin and 30 $\mu$g/mL blasticidin, and 100$\mu$l of sterile water alone was added to the periphery wells of the 96-well plate. The culture plate was incubated in an incubator for 24 hours (37°C, 5% CO$_2$). Once the adherence of cells was observed, 100 $\mu$l of the antibody to be tested at a gradient of dilutions was added to each well. The culture plate was incubated in an incubator for 20-24 hours (37°C, 5% CO$_2$). 40$\mu$l of cell supernatant was transferred from each well to a new 96-well flat bottom plate, 160$\mu$l QUANTI-Blue substrate solution was added, and the culture plate was incubated in an incubator in the dark for 1-3 hours. The absorbance at 620 nm was measured with a microplate reader (Thermo MultiSkanFc), and EC50 value was calculated to evaluate the activity of the CD40 antibody *in vitro* in cells.

Table 4. Test results of activity of anti-CD40 antibody on reporter gene in cells

| Antibody strain | test of activity in HEK293-CD40L cells, EC50 ($\mu$g/mL) | |
|---|---|---|
| 1D9 | +++ | 0.01454 |
| 2H6 | +++ | 0.01511 |
| 9E5 | ++ | 0.01712 |
| 14C10 | +++ | 0.01087 |
| 38B4 | ++ | 0.0365 |

## EXAMPLE 7 TEST OF ANTI-CD40 ANTIBODY TO ACTIVATE DC CELLS

PBMCs were isolated from the peripheral blood of normal human subject, and then monocytes were sorted using CD14 MACS beads. RPMI 1640 medium comprising 10 ng/mL IL4 and 100 ng/mL GM-CSF was added for cultivation for 6 days to induce MoDC cells (dendritic cells derived from monocytes). Cells were collected after 6 days, $1 \times 10^5$ cells were taken, and stained with CD209-PE, CD1a-PerCP/Cy5.5 and CD14-PE/Cy7 for analyzing whether MoDC has been successfully induced by FACS (the above operations are routine operations in the art).

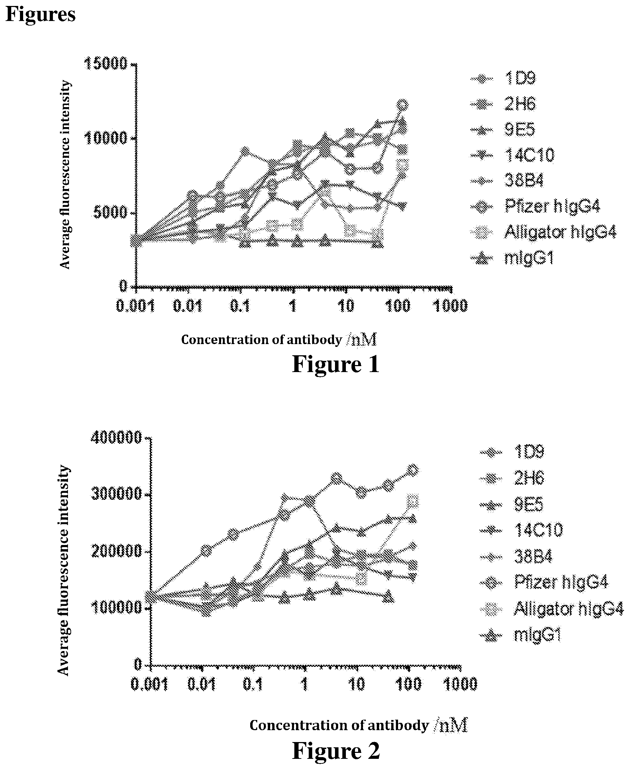

The successfully induced DCs were collected, each antibody to be tested and control antibody were added, and the corresponding dilution gradient of concentrations were set up (see Figure 1 for the gradient of concentrations of antibody). After cultivating for 48 hours, the cells were collected and stained for CD80, CD86 and HLA-DR, and data was collected by FACS.

According to the data in a test of activating primary DC cells, all of the five murine antibodies showed obvious activity on activating molecules CD80 and CD86 on the surface of DC cells, in a dose-dependent way. The overall effect was comparable to, equivalent to, or even slightly better than that of the two control antibodies (CP-870,893 from Pfizer, and ADC-1013 from Alligator Bioscience) (See Figure 1 and Figure 2).

**EXAMPLE 8 CLONING AND SEQUENCING OF ANTI-CD40 ANTIBODY**

The hybridoma subclones of the 5 antibodies identified from the above screening were taken, the hybridoma cells at logarithmic growth phase were collected; RNA was extracted with Trizol (Invitrogen, 15596-018) (following the instructions in the kit), and reverse transcription (PrimeScript™ Reverse Transcriptase, Takara, cat # 2680A) was performed. The cDNA obtained by reverse transcription was amplified by PCR using mouse Ig-Primer Set (Novagen, TB326 Rev.B0503), and delivered to a company for sequencing. Finally, the sequences of 5 murine antibodies were obtained.

(1) The sequences of heavy chain and light chain variable region of murine monoclonal antibody 2H6 are as follows:

2H6 HCVR
QVQLQQSGAELVRPGTSVKVSCKAS*GYAFSDYLIE*WAKQRPGQGLEWIG*VINPGS GGSNYNEKIKD*RATLTADKSSSTAYMQLSSLTSEDSAVYFCAR*GGGGFTY*WGQGT LVTVSA (SEQ ID NO: 1);

2H6 LCVR
EIQLTQTTSSLSASLGDRVTISC<u>RASQDISNYLN</u>WYQQKPDGTIKLLLN<u>FASRLHS</u> GVPSRFSGSGSGTDFFLTISNLEQDDIATYFC<u>QQGSTLPWT</u>FGGGTKLEIK (SEQ ID NO: 2);

The CDR sequences included therein are shown in Table 5 below:

Table 5. CDR sequences of 2H6

| Name | Sequence | SEQ ID NO |
|---|---|---|
| HCDR1 | GYAFSDYLIE | SEQ ID NO: 3 |
| HCDR2 | VINPGSGGSNYNEKIKD | SEQ ID NO: 4 |
| HCDR3 | GGGGFTY | SEQ ID NO: 5 |
| LCDR1 | RASQDISNYLN | SEQ ID NO: 6 |
| LCDR2 | FASRLHS | SEQ ID NO: 7 |
| LCDR3 | QQGSTLPWT | SEQ ID NO: 8 |

(2) The sequences of heavy chain and light chain variable region of 9E5 are as follows:

9E5 HCVR
QVQLQQPGADLVKPGASVKMSCKAS<u>GYILTTYWIT</u>WVKQRPGQGLEWIG<u>DIHP GSGSTKYNEKFKS</u>KATLTVDTSSSTAYMQLTRLSSEDSAVYYCAR<u>RDY</u>WGQGT TLTVSS (SEQ ID NO: 9);

9E5 LCVR
DVLMTQSPLSLPVSLGDQASISCRSSQNIVNSQGNTYLEWYLQKPGESPKLLIYK
VTNRFSGVPDRFSGSGSGTDFTLKISRVEAEDLGVYYCFQASLVPWTFGGGTKL
EIK (SEQ ID NO: 10);

The CDR sequences included therein are shown in Table 6 below:

Table 6. CDR sequences of 9E5

| Name | Sequence | SEQ ID NO |
|---|---|---|
| HCDR1 | GYILTTYWIT | SEQ ID NO: 11 |
| HCDR2 | DIHPGSGSTKYNEKFKS | SEQ ID NO: 12 |
| HCDR3 | RDY | SEQ ID NO: 13 |
| LCDR1 | RSSQNIVNSQGNTYLE | SEQ ID NO: 14 |
| LCDR2 | KVTNRFS | SEQ ID NO: 15 |
| LCDR3 | FQASLVPWT | SEQ ID NO: 16 |

(3) The sequences of heavy chain and light chain variable region of 1D9 are as follows:

1D9 HCVR
QVRLQQSGAELVRPGTSMRVSCKASGYAFTNYLINWVKQRPGQGLEWIGILNP
GSGGTNYNENFKDKATLTADKSSNTAYMQLSSLTSEDSAVYFCIRGSPGFAYWG
QGTLVTVSA (SEQ ID NO: 37);

1D9 LCVR
DIQMTQTTSSLSASLGDRVTISCRASQDINIYLNWYQQKPDGTVKLLIYSTSGLH
SGVPSRFNGSGSGTDYSLTISNLEQEDIATYFCQQGYTLPYTFGGGTKLEIK (SEQ
ID NO: 38);

The CDR sequences included therein are shown in Table 7 below:

Table 7. CDR sequences of 1D9

| Name | Sequence | SEQ ID NO |
|---|---|---|
| HCDR1 | GYAFTNYLIN | SEQ ID NO: 39 |
| HCDR2 | ILNPGSGGTNYNENFKD | SEQ ID NO: 40 |
| HCDR3 | GSPGFAY | SEQ ID NO: 41 |
| LCDR1 | RASQDINIYLN | SEQ ID NO: 42 |
| LCDR2 | STSGLHS | SEQ ID NO: 43 |
| LCDR3 | QQGYTLPYT | SEQ ID NO: 44 |

(4) The sequences of heavy chain and light chain variable region of 14C10 are as follows:

14C10 HCVR
QVQVQQSGAELVRPGTSVKVSCKASGYAFTNYLIEWVKQRPGQGLEWIGVINP
EFGGTNYNEKFKGKATLTADKSSSTAYMQLSSLTSEDSAVYFCARGGGGFTYW
GQGTLVTVSA (SEQ ID NO: 45);

14C10 LCVR
HIQMTQTTSSLSASLGDRVTISC<u>RASQDISSHLN</u>WYQQKPDGTVKLLIS<u>YTSRLH</u>SGVPSRFSGSGSGADYSLTISNLEQEDIATYFC<u>QQGNTLPWT</u>FGGGTKLEIK
(SEQ ID NO: 46);

The CDR sequences included therein are shown in Table 8 below:

Table 8. CDR sequences of 14C10

| Name | Sequence | SEQ ID NO |
|------|----------|-----------|
| HCDR1 | GYAFTNYLIE | SEQ ID NO: 47 |
| HCDR2 | VINPEFGGTNYNEKFKG | SEQ ID NO: 48 |
| HCDR3 | GGGGFTY | SEQ ID NO: 49 |
| LCDR1 | RASQDISSHLN | SEQ ID NO: 50 |
| LCDR2 | YTSRLHS | SEQ ID NO: 51 |
| LCDR3 | QQGNTLPWT | SEQ ID NO: 52 |

(5) The sequences of heavy chain and light chain variable region of 38B4 are as follows:

38B4 HCVR
QVRLKQSGAELVRPGASVKVSCKAS<u>GYTFTDYYIN</u>WVKQRPGQGLEWIA<u>GIYPGTGNTYYNEKFKG</u>KATLTAERSSSTAYMQLTSLTSEDSAVYFCTR<u>RGLPSLCFDY</u>WGQGTTLTVSS (SEQ ID NO: 53);

38B4 LCVR
DFQMTQTTSSLSASLGDRVTISC<u>SASQGISNYLN</u>WYQQKPDGTVKLLIY<u>YTSSLH</u>SGVPSRFSGSGSGTDYSLTISNLEPEDIATYYC<u>QQYSKLPPT</u>FGGGTKLEIK (SEQ ID NO: 54);

The CDR sequences included therein are shown in Table 9 below:

Table 9. CDR sequences of 38B4

| Name | Sequence | SEQ ID NO |
|------|----------|-----------|
| HCDR1 | GYTFTDYYIN | SEQ ID NO: 55 |
| HCDR2 | GIYPGTGNTYYNEKFKG | SEQ ID NO: 56 |
| HCDR3 | RGLPSLCFDY | SEQ ID NO: 57 |
| LCDR1 | SASQGISNYLN | SEQ ID NO: 58 |
| LCDR2 | YTSSLHS | SEQ ID NO: 59 |
| LCDR3 | QQYSKLPPT | SEQ ID NO: 60 |

Among them, the optimal two strains of antibodies (2H6 and 9E5) were selected for follow-up development. The obtained variable region sequences were respectively connected to human antibody IgG1 constant region sequences to obtain human-mouse chimeric antibody sequences. Using molecular cloning technology, the sequence of the chimeric antibody was inserted into pCP expression vector (purchased from Mabspace Biosciences), and then the sequence was identified by PCR (molecular cloning and other molecular biological operations in this part are carried out according to conventional operation conditions. For more details, please refer to "Molecular Cloning: A Laboratory Manual"). HEK293 cell expression system was used to obtain human-mouse chimeric antibodies 2H6-C and 9E5-C.
The chimeric antibodies purified by MabSelect SuRe affinity chromatography (GE Lifesciences) were tested for various activities *in vitro.* The data are shown in Table 10.

Table 10. *In vitro* activity of chimeric antibodies

| Chimeric antibody | human CD40-his ELISA EC50 (ng/mL) | human hCD40/hCD40L, blocking ELISA IC50 ($\mu$g/mL) | HEK293-CD40 cell-binding EC50 ($\mu$g/mL) | Biacore affinity $K_D$ (M) |
|---|---|---|---|---|
| 2H6-C | 4.565 | 0.6275 | 0.02593 | 3.98 |
| 9E5-C | 1.346 | 0.1218 | 0.03333 | 2.68 |
| Pfizer control | 5.628 | 0.2583 | 0.01638 | 20.35 |
| (hIgG4) | | | | |
| Alligator control (hIgG1) | 3.288 | 0.7233 | 0.39650 | 65.9 |

**EXAMPLE 9 HUMANIZATION TEST OF MURINE ANTIBODY**

Based on the obtained typical VH/VLCDR structures of the murine antibodies 2H6 and 9E5, the heavy chain variable region and light chain variable region sequences were aligned against an antibody germline database to obtain a human germline template with high homology.

The human germline light chain framework region is derived from the human kappa light chain gene. The human germline light chain template for the antibody of present disclosure is preferably Vk1-33/JK4 (for 2H6) or Vk2-28/JK4 (for 9E5).

The human germline heavy chain framework region is derived from the human heavy chain. The human germline heavy chain template for the antibody of present disclosure is preferably VH1-69/JH6 (for 2H6) or VH1-2/JH6 (for 9E5), as shown below:

Preferable human germline heavy chain template IGHV1-69 for 2H6

QVQLVQSGAEVKKPGSSVKVSCKASGGTFSSYAISWVRQAPGQGLEWMGGIIPI FGTANYAQKFQGRVTITADKSTSTAYMELSSLRSEDTAVYYCAR  (SEQ  ID  NO: 21);

Preferable human germline light chain template IGkV1-33 for 2H6

DIQMTQSPSSLSASVGDRVTITCQASQDISNYLNWYQQKPGKAPKLLIYDASNL ETGVPSRFSGSGSGTDFTFTISSLQPEDIATYYCQQYDNLP (SEQ ID NO: 22);

Preferable human germline heavy chain template IGHV1-2 for 9E5

QVQLVQSGAEVKKPGASVKVSCKASGYTFTGYYMHWVRQAPGQGLEWMGWI NPNSGGTNYAQKFQGRVTMTRDTSISTAYMELSRLRSDDTAVYYCAR  (SEQ  ID NO: 23);

Preferable human germline light chain template IGkV2-28 for 9E5

DIVMTQSPLSLPVTPGEPASISCRSSQSLLHSNGYNYLDWYLQKPGQSPQLLIYL GSNRASGVPDRFSGSGSGTDFTLKISRVEAEDVGVYYCMQALQTP (SEQ ID NO: 24);

The CDR regions of the murine antibody were grafted onto the selected humanized template, to replace the humanized variable regions, and then recombined with corresponding human IgG constant regions (preferably IgG1 for heavy chain; and kappa for light chain). Based on the three-dimensional structure of the murine antibody, back-mutations were performed on the embedded residues, the residues that directly interact with CDR regions, and the residues that have an important impact on conformation of VL and VH, and the amino acid residues in CDR regions that are not chemically stable were optimized to obtain the final humanized molecules.

The sequences of the heavy chain variable regions are shown in SEQ ID NOs: 25-30;
The sequences of the light chain variable regions are shown in SEQ ID NOs: 31-36.

hu2H6-H1a:

QVQLVQSGAEVKKPGSSVKVSCKASGGTFSDYLIEWVRQAPGQGLEWMGVIN
PGSGGSNYNEKIKDRVTITADKSTSTAYMELSSLRSEDTAVYYCARGGGGFTYW
GQGTLVTVSS (SEQ ID NO: 25);

hu2H6-H1b:

QVQLVQSGAEVKKPGSSVKVSCKASGYAFSDYLIEWVRQAPGQGLEWMGVIN
PGSGGSNYNEKIKDRVTLTADKSTSTAYMELSSLRSEDTAVYYCARGGGGFTYW
GQGTLVTVSS (SEQ ID NO: 26);

hu2H6-H1c:

QVQLVQSGAEVKKPGSSVKVSCKASGYAFSDYLIEWVRQAPGQGLEWIGVINP
GSGGSNYNEKIKDRATLTADKSTSTAYMELSSLRSEDTAVYYCARGGGGFTYW
GQGTLVTVSSFGQGTKLEIK (SEQ ID NO: 27);

hu9E5-H1a:

QVQLVQSGAEVKKPGASVKVSCKASGYTFTTYWITWVRQAPGQGLEWMGDIH
PGSGSTKYNEKFKSRVTMTVDTSISTAYMELSRLRSEDTAVYYCARRDYWGQG
TTVTVSS (SEQ ID NO: 28);

hu9E5-H1b:

QVQLVQSGAEVKKPGASVKVSCKASGYTFTTYWITWVRQAPGQGLEWMGDIH
PGSGSTKYNEKFKSRVTLTVDTSISTAYMELSRLRSEDTAVYYCARRDYWGQGT
TVTVSS (SEQ ID NO: 29);

hu9E5-H1c:

QVQLVQSGAEVKKPGASVKVSCKASGYILTTYWITWVRQAPGQGLEWMGDIH
PGSGSTKYNEKFKSRVTLTVDTSISTAYMELSRLRSEDTAVYYCARRDYWGQGT
TVTVSS (SEQ ID NO: 30);

hu2H6-L1a:

DIQMTQSPSSLSASVGDRVTITCRASQDISNYLNWYQQKPGKAPKLLLNFASRL
HSGVPSRFSGSGSGTDFTFTISSLQPEDIATYYCQQGSTLPWTFGGGTKVEIK
(SEQ ID NO: 31);

hu2H6-L1b:

DIQLTQSPSSLSASVGDRVTITCRASQDISNYLNWYQQKPGKAPKLLLNFASRLH SGVPSRFSGSGSGTDFTFTISSLQPEDIATYYCQQGSTLPWTFGGGTKVEIK (SEQ ID NO: 32);

hu2H6-L1c:

DIQLTQSPSSLSASVGDRVTITCRASQDISNYLNWYQQKPGKTIKLLLNFASRLHS GVPSRFSGSGSGTDFTFTISSLQPEDIATYYCQQGSTLPWTFGGGTKVEIK (SEQ ID NO: 33);

hu9E5-L1a:

DIVMTQSPLSLPVTPGEPASISCRSSQNIVNSQGNTYLEWYLQKPGQSPQLLIYK VTNRFSGVPDRFSGSGSGTDFTLKISRVEAEDVGVYYCFQASLVPWTFGGGTKV EIK (SEQ ID NO: 34);

hu9E5-L1b:

DVVMTQSPLSLPVTPGEPASISCRSSQNIVNSQGNTYLEWYLQKPGQSPQLLIYK VTNRFSGVPDRFSGSGSGTDFTLKISRVEAEDVGVYYCFQASLVPWTFGGGTKV EIK (SEQ ID NO: 35);

hu9E5-L1c:

DVLMTQSPLSLPVTPGEPASISCRSSQNIVNSQGNTYLEWYLQKPGQSPQLLIYK VTNRFSGVPDRFSGSGSGTDFTLKISRVEAEDVGVYYCFQASLVPWTFGGGTKV EIK (SEQ ID NO: 36).

By expression test of the above combinations of light and heavy chains and by comparisons between different numbers of back mutations, the humanized antibody molecules hu2H6 (with H1b heavy chain and L1c light chain) and hu9E5 (with H1c heavy chain and L1a light chain) were finally selected, and the respective complete light chain and heavy chain sequences are shown in SEQ ID NO: 17-20.

hu2H6 HC:

QVQLVQSGAEVKKPGSSVKVSCKASGYAFSDYLIEWVRQAPGQGLEWMGVIN PGSGGSNYNEKIKDRVTLTADKSTSTAYMELSSLRSEDTAVYYCARGGGGFTYW GQGTLVTVSSASTKGPSVFPLAPSSKSTSGGTAALGCLVKDYFPEPVTVSWNSG ALTSGVHTFPAVLQSSGLYSLSSVVTVPSSSLGTQTYICNVNHKPSNTKVDKKVE PKSCDKTHTCPPCPAPELLGGPSVFLFPPKPKDTLMISRTPEVTCVVVDVSHEDP EVKFNWYVDGVEVHNAKTKPREEQYNSTYRVVSVLTVLHQDWLNGKEYKCK VSNKALPAPIEKTISKAKGQPREPQVYTLPPSRDELTKNQVSLTCLVKGFYPSDIA VEWESNGQPENNYKTTPPVLDSDGSFFLYSKLTVDKSRWQQGNVFSCSVMHEA LHNHYTQKSLSLSPGK (SEQ ID NO: 17);

hu2H6 LC:

DIQLTQSPSSLSASVGDRVTITCRASQDISNYLNWYQQKPGKTIKLLLNFASRLHS
GVPSRFSGSGSGTDFTFTISSLQPEDIATYYCQQGSTLPWTFGGGTKVEIKRTVAA
PSVFIFPPSDEQLKSGTASVVCLLNNFYPREAKVQWKVDNALQSGNSQESVTEQ
DSKDSTYSLSSTLTLSKADYEKHKVYACEVTHQGLSSPVTKSFNRGEC (SEQ ID
NO: 18);

hu9E5 HC:

QVQLVQSGAEVKKPGASVKVSCKASGYILTTYWITWVRQAPGQGLEWMGDIH
PGSGSTKYNEKFKSRVTLTVDTSISTAYMELSRLRSEDTAVYYCARRDYWGQGT

TVTVSSASTKGPSVFPLAPSSKSTSGGTAALGCLVKDYFPEPVTVSWNSGALTSG
VHTFPAVLQSSGLYSLSSVVTVPSSSLGTQTYICNVNHKPSNTKVDKKVEPKSCD
KTHTCPPCPAPELLGGPSVFLFPPKPKDTLMISRTPEVTCVVVDVSHEDPEVKFN
WYVDGVEVHNAKTKPREEQYNSTYRVVSVLTVLHQDWLNGKEYKCKVSNKA
LPAPIEKTISKAKGQPREPQVYTLPPSRDELTKNQVSLTCLVKGFYPSDIAVEWES
NGQPENNYKTTPPVLDSDGSFFLYSKLTVDKSRWQQGNVFSCSVMHEALHNHY
TQKSLSLSPGK (SEQ ID NO: 19);

hu9E5 LC:

DIVMTQSPLSLPVTPGEPASISCRSSQNIVNSQGNTYLEWYLQKPGQSPQLLIYK
VTNRFSGVPDRFSGSGSGTDFTLKISRVEAEDVGVYYCFQASLVPWTFGGGTKV
EIKRTVAAPSVFIFPPSDEQLKSGTASVVCLLNNFYPREAKVQWKVDNALQSGN
SQESVTEQDSKDSTYSLSSTLTLSKADYEKHKVYACEVTHQGLSSPVTKSFNRG
EC (SEQ ID NO: 20).

## EXAMPLE 10 TEST DATA OF HUMANIZED ANTIBODY

The present disclosure shows the binding activity and blocking activity of humanized antibodies hu2H6 and hu9E5 to human CD40 and rhesus CD40, as shown in Table 11.

The results show that the humanized anti-human CD40 antibodies of present disclosure have ELISA binding and blocking activity comparable to that of positive antibodies Pfizer/Alligator. In particular, the affinity of hu9E5 to human CD40 measured by Biacore is 10 times or more than that of the antibody Alligator, a positive control, and 4 times or more than that of the Pfizer Control.

Table 11 *In vitro* activities of humanized antibodies hu2H6 and hu9E5

| | human CD40- his ELISA EC50 (ng/mL) | Rhesus CD40-his ELISA EC50 (ng/mL) | human hCD40/hCD40L, blocking ELISA IC50 ($\mu$g/mL) | HEK293-CD40 cell-binding EC50 ($\mu$g/mL) | Biacore affinity $K_D$ (M) |
|---|---|---|---|---|---|
| Hu2H6-11 | 3.680 | 2.945 | 0.6735 | 0.01538 | 1.120E-8 |
| Hu9E5-25 | 1.650 | 1.661 | 0.3084 | 0.13970 | 5.301E-9 |
| Alligator control (hIgGl) | 1.293 | 1.243 | 0.6471 | 1.36200 | 1.66E-7 |
| Pfizer control (hIgG4) | 3.976 | 3.561 | 0.3106 | 0.01907 | 2.035E-8 |

**EXAMPLE 11 INHIBITION OF MOUSE TUMOR GROWTH BY ANTI-CD40 ANTIBODY**

Peripheral blood of normal human subject was taken, and PBMCs of healthy human subject were separated by density gradient centrifugation. The monocytes were isolated with CD14+microbeads kit, the CD14+monocytes were isolated according to procedure provided by the kit, i.e., 20 $\mu$l anti-CD14 microbeads were added to every $10^7$ cells, and incubated at 4°C for 15 minutes. Then, the cells were added to magnetic column, and the column was washed for three times, the cells were collected from the magnetic column, namely CD14+ monocytes. CD14+ monocytes were added with RPMI 1640 medium comprising 10 ng/mL IL-4 and 100 ng/mL GM-CSF, and were cultivated for 6 days (the method for cultivating is a conventional method in the art); then the MoDC cells were induced and cultivated, and the remaining cells were added with RPMI 1640 comprising IL-2; the suspended cells were collected after cultivating (the method for cultivating and the method for collecting cells are conventional methods in the art), T cells were sorted by CD3+ microbead kit. Six days later, MoDC cells and CD3+T cells were collected and separated; and mixed with Raji cells (Cell Bank of Shanghai Academy of Biological Sciences, cultivated in RPMI1640 medium comprising 10% fetal bovine serum) at a ratio of 1:5:20. The mixture was used to subcutaneously inoculate each NOG mouse (Nanjing Galaxy Biopharma Co., Ltd, adaptive breeding for 5 days). The laboratory animals were kept in an independent ventilated cage with constant temperature and humidity. The temperature in the breeding room was 18.0-26.0°C, the humidity was 40-70%, and the ventilation rate was 10-20 times per hour. The alternating time for day and night was 12h/12h.

Human IgG1 antibody control group, hu2H6, hu9E5 and control antibody G12 group (i.e. ADC-1013 from Alligator Bioscience) were divided in the test, and the dose was 3 mg/kg for each group. Each group of 5 mice was injected once a week, for six weeks, with 3 consecutive doses.

The procedures for the test were as follows:

(1) The long diameter and short diameter of tumor were measured twice a week, with a vernier caliper, and the tumor volume ($mm^3$) was calculated as = $0.5 \times$ (tumor long diameter $\times$ tumor short diameter$^2$).

(2) Relative inhibition rate of tumor TGI (%): TGI%= (1-T/C) $\times$ 100%. T/C% is the relative proliferation rate of tumor (i.e. the percentage value of the tumor volume or tumor weight in the treatment group relative to the control group, at a certain time point). T and C are tumor volume (TV) or tumor weight (TW) of the treatment group and IgG1 control group at a specific time point, respectively.

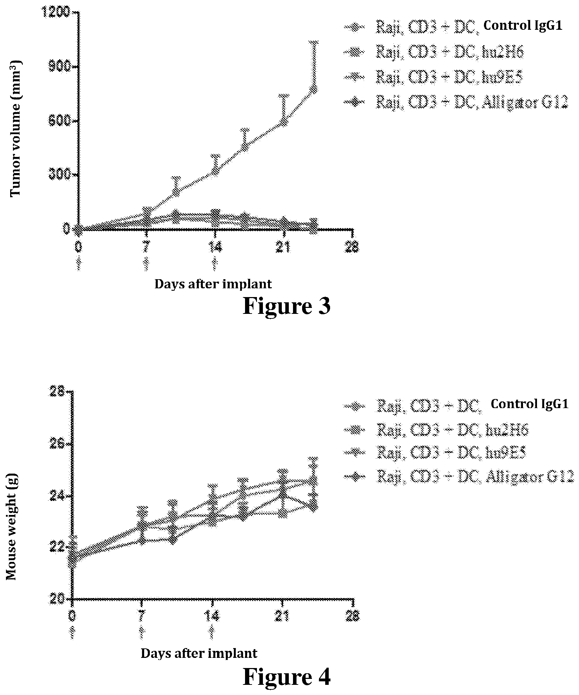

The results show that the humanized anti-CD40 antibodies hu2H6 and hu9E5 have very significant anti-tumor effects when compared to that of the IgG1 control. The tumor was almost completely eliminated on day 21 after administration; the anti-tumor effect was equivalent to or slightly better than that of the control antibody G12, as shown in Figure 3 and Figure 4.

**EXAMPLE 12 PREPARATION OF ANTI-CD40 ANTIBODY COMPRISING MUTATION(S) IN HEAVY CHAIN CONSTANT REGION**

In this example, variants of the anti-CD40 antibody described above were prepared, which have mutation(s) in the heavy chain constant region.

In particular:

- The amino acid at position 266 of hu2H6 heavy chain of SEQ ID NO: 17 was mutated from serine (S) to glutamic acid (E), to obtain mutant hu2H6-M;
- The amino acid at position 266 of hu2H6 heavy chain of SEQ ID NO: 17 was mutated from serine (S) to glutamic acid (E), the amino acid at position 324 was mutated from asparagine (N) to serine (S), and the amino acid at position 327 was mutated from leucine (L) to phenylalanine (F), to obtain mutant hu2H6-SELFNS;
- The amino acid at position 262 of hu9E5 heavy chain of SEQ ID NO: 19 was mutated from serine (S) to glutamic acid (E), to obtain mutant hu9E5-M;
- The amino acid at position 262 of hu9E5 heavy chain of SEQ ID NO: 19 was mutated from serine (S) to glutamic acid (E), and the amino acid at position 323 was mutated from leucine (L) to phenylalanine (F), to obtain mutant hu9E5-SELF;
- The amino acid at position 262 of hu9E5 heavy chain of SEQ ID NO: 19 was mutated from serine (S) to glutamic acid (E), the amino acid at position 320 was mutated from asparagine (N) to serine (S), and the amino acid at position 323 was mutated from leucine (L) to phenylalanine (F), to obtain mutant hu9E5-SELFNS.

The heavy chain sequences of hu2H6-M and hu2H6-SELFNS are shown in SEQ ID NOs: 61 and 62, and the light chain sequence is shown in SEQ ID NO: 18.

The heavy chain sequences of hu9E5-M, hu9E5-SELF, and hu9E5-SELFNS are shown in SEQ ID NO: 63, 64 and 67 and the light chain sequence is shown in SEQ ID NO: 20.

hu2H6-M HC:

QVQLVQSGAEVKKPGSSVKVSCKASGYAFSDYLIEWVRQAPGQGLEWMGVIN
PGSGGSNYNEKIKDRVTLTADKSTSTAYMELSSLRSEDTAVYYCARGGGGFTYW
GQGTLVTVSSASTKGPSVFPLAPSSKSTSGGTAALGCLVKDYFPEPVTVSWNSG
ALTSGVHTFPAVLQSSGLYSLSSVVTVPSSSLGTQTYICNVNHKPSNTKVDKKVE
PKSCDKTHTCPPCPAPELLGGPSVFLFPPKPKDTLMISRTPEVTCVVVDVEHEDP

EVKFNWYVDGVEVHNAKTKPREEQYNSTYRVVSVLTVLHQDWLNGKEYKCK
VSNKALPAPIEKTISKAKGQPREPQVYTLPPSRDELTKNQVSLTCLVKGFYPSDIA
VEWESNGQPENNYKTTPPVLDSDGSFFLYSKLTVDKSRWQQGNVFSCSVMHEA
LHNHYTQKSLSLSPGK (SEQ ID NO: 61);

hu2H6-SELFNS HC:

QVQLVQSGAEVKKPGSSVKVSCKASGYAFSDYLIEWVRQAPGQGLEWMGVIN
PGSGGSNYNEKIKDRVTLTADKSTSTAYMELSSLRSEDTAVYYCARGGGGFTYW
GQGTLVTVSSASTKGPSVFPLAPSSKSTSGGTAALGCLVKDYFPEPVTVSWNSG
ALTSGVHTFPAVLQSSGLYSLSSVVTVPSSSLGTQTYICNVNHKPSNTKVDKKVE
PKSCDKTHTCPPCPAPELLGGPSVFLFPPKPKDTLMISRTPEVTCVVVDVEHEDP
EVKFNWYVDGVEVHNAKTKPREEQYNSTYRVVSVLTVLHQDWLNGKEYKCK
VSSKAFPAPIEKTISKAKGQPREPQVYTLPPSRDELTKNQVSLTCLVKGFYPSDIA
VEWESNGQPENNYKTTPPVLDSDGSFFLYSKLTVDKSRWQQGNVFSCSVMHEA
LHNHYTQKSLSLSPGK (SEQ ID NO: 62);

hu9E5-M HC:

QVQLVQSGAEVKKPGASVKVSCKASGYILTTYWITWVRQAPGQGLEWMGDIH
PGSGSTKYNEKFKSRVTLTVDTSISTAYMELSRLRSEDTAVYYCARRDYWGQGT
TVTVSSASTKGPSVFPLAPSSKSTSGGTAALGCLVKDYFPEPVTVSWNSGALTSG
VHTFPAVLQSSGLYSLSSVVTVPSSSLGTQTYICNVNHKPSNTKVDKKVEPKSCD
KTHTCPPCPAPELLGGPSVFLFPPKPKDTLMISRTPEVTCVVVDVEHEDPEVKFN
WYVDGVEVHNAKTKPREEQYNSTYRVVSVLTVLHQDWLNGKEYKCKVSNKA
LPAPIEKTISKAKGQPREPQVYTLPPSRDELTKNQVSLTCLVKGFYPSDIAVEWES
NGQPENNYKTTPPVLDSDGSFFLYSKLTVDKSRWQQGNVFSCSVMHEALHNHY
TQKSLSLSPGK (SEQ ID NO: 63);

hu9E5-SELF HC:

QVQLVQSGAEVKKPGASVKVSCKASGYILTTYWITWVRQAPGQGLEWMGDIH
PGSGSTKYNEKFKSRVTLTVDTSISTAYMELSRLRSEDTAVYYCARRDYWGQGT
TVTVSSASTKGPSVFPLAPSSKSTSGGTAALGCLVKDYFPEPVTVSWNSGALTSG
VHTFPAVLQSSGLYSLSSVVTVPSSSLGTQTYICNVNHKPSNTKVDKKVEPKSCD
KTHTCPPCPAPELLGGPSVFLFPPKPKDTLMISRTPEVTCVVVDVEHEDPEVKFN
WYVDGVEVHNAKTKPREEQYNSTYRVVSVLTVLHQDWLNGKEYKCKVSNKA
FPAPIEKTISKAKGQPREPQVYTLPPSRDELTKNQVSLTCLVKGFYPSDIAVEWES
NGQPENNYKTTPPVLDSDGSFFLYSKLTVDKSRWQQGNVFSCSVMHEALHNHY
TQKSLSLSPGK (SEQ ID NO: 64);

hu9E5-SELFNS HC:

QVQLVQSGAEVKKPGASVKVSCKASGYILTTYWITWVRQAPGQGLEWMGDIH
PGSGSTKYNEKFKSRVTLTVDTSISTAYMELSRLRSEDTAVYYCARRDYWGQGT
TVTVSSASTKGPSVFPLAPSSKSTSGGTAALGCLVKDYFPEPVTVSWNSGALTSG
VHTFPAVLQSSGLYSLSSVVTVPSSSLGTQTYICNVNHKPSNTKVDKKVEPKSCD
KTHTCPPCPAPELLGGPSVFLFPPKPKDTLMISRTPEVTCVVVDVEHEDPEVKFN
WYVDGVEVHNAKTKPREEQYNSTYRVVSVLTVLHQDWLNGKEYKCKVSSKA
FPAPIEKTISKAKGQPREPQVYTLPPSRDELTKNQVSLTCLVKGFYPSDIAVEWES
NGQPENNYKTTPPVLDSDGSFFLYSKLTVDKSRWQQGNVFSCSVMHEALHNHY
TQKSLSLSPGK (SEQ ID NO: 67).

In addition, the amino acid K at the last position of SEQ ID NO: 61, 62, 63, 64 and 67 can be replaced with A. This mutation does not affect the activity of the antibody, but can improve the drugability of the antibody to a certain degree.

In addition, according to the variable region of another anti-CD40 antibody APX005S267E described in CN104918957A (i.e. amino acids at positions 1-120), antibody 005M was prepared as a positive control, and the amino acid sequence from positions 121 to 450 of the heavy chain of antibody 005M is the same as the amino acid sequence from positions 113 to 442 of heavy chain of antibody hu9E5-M. The specific sequences of 005M are as follows:

005M-HC:

QVQLVESGGGVVQPGRSLRLSCAASGFSFSSTYVCWVRQAPGKGLEWIACIYT
GDGTNYSASWAKGRFTISKDSSKNTVYLQMNSLRAEDTAVYFCARPDITYGFAI
NFWGPGTLVTVSSASTKGPSVFPLAPSSKSTSGGTAALGCLVKDYFPEPVTVSW
NSGALTSGVHTFPAVLQSSGLYSLSSVVTVPSSSLGTQTYICNVNHKPSNTKVDK
KVEPKSCDKTHTCPPCPAPELLGGPSVFLFPPKPKDTLMISRTPEVTCVVVDVEH
EDPEVKFNWYVDGVEVHNAKTKPREEQYNSTYRVVSVLTVLHQDWLNGKEY
KCKVSNKALPAPIEKTISKAKGQPREPQVYTLPPSRDELTKNQVSLTCLVKGFYP
SDIAVEWESNGQPENNYKTTPPVLDSDGSFFLYSKLTVDKSRWQQGNVFSCSVM
HEALHNHYTQKSLSLSPGK (SEQ ID NO:65);

005M-LC:

DIQMTQSPSSLSASVGDRVTIKCQASQSISSRLAWYQQKPGKPPKLLIYRASTLA
SGVPSRFSGSGSGTDFTLTISSLQPEDVATYYCQCTGYGISWPIGGGTKVEIKRTV
AAPSVFIFPPSDEQLKSGTASVVCLLNNFYPREAKVQWKVDNALQSGNSQESVT
EQDSKDSTYSLSSTLTLSKADYEKHKVYACEVTHQGLSSPVTKSFNRGEC (SEQ
ID NO:66);

The amino acid at position 331 of 005M heavy chain of SEQ ID NO: 65 was mutated from leucine (L) to phenylalanine (F) to obtain mutant APX005-SELF; The amino acid at position 328 of 005M heavy chain of SEQ ID NO: 65 was mutated from asparagine (N) to serine (S), and the amino acid on position 331 was mutated from leucine (L) to phenylalanine (F) to obtain the mutant APX005-SELFNS.

APX005-SELF HC (L331F):

QVQLVESGGGVVQPGRSLRLSCAASGFSFSSTYVCWVRQAPGKGLEWIACIYT
GDGTNYSASWAKGRFTISKDSSKNTVYLQMNSLRAEDTAVYFCARPDITYGFAI
NFWGPGTLVTVSSASTKGPSVFPLAPSSKSTSGGTAALGCLVKDYFPEPVTVSW
NSGALTSGVHTFPAVLQSSGLYSLSSVVTVPSSSLGTQTYICNVNHKPSNTKVDK
KVEPKSCDKTHTCPPCPAPELLGGPSVFLFPPKPKDTLMISRTPEVTCVVVDVEH
EDPEVKFNWYVDGVEVHNAKTKPREEQYNSTYRVVSVLTVLHQDWLNGKEY
KCKVSNKA**F**PAPIEKTISKAKGQPREPQVYTLPPSRDELTKNQVSLTCLVKGFYP
SDIAVEWESNGQPENNYKTTPPVLDSDGSFFLYSKLTVDKSRWQQGNVFSCSVM
HEALHNHYTQKSLSLSPGK (SEQ ID NO:68);

APX005-SELFNS HC (L331F, N328S):

QVQLVESGGGVVQPGRSLRLSCAASGFSFSSTYVCWVRQAPGKGLEWIACIYT
GDGTNYSASWAKGRFTISKDSSKNTVYLQMNSLRAEDTAVYFCARPDITYGFAI
NFWGPGTLVTVSSASTKGPSVFPLAPSSKSTSGGTAALGCLVKDYFPEPVTVSW
NSGALTSGVHTFPAVLQSSGLYSLSSVVTVPSSSLGTQTYICNVNHKPSNTKVDK
KVEPKSCDKTHTCPPCPAPELLGGPSVFLFPPKPKDTLMISRTPEVTCVVVDVEH
EDPEVKFNWYVDGVEVHNAKTKPREEQYNSTYRVVSVLTVLHQDWLNGKEY

KCKVS**S**KA**F**PAPIEKTISKAKGQPREPQVYTLPPSRDELTKNQVSLTCLVKGFYPS
DIAVEWESNGQPENNYKTTPPVLDSDGSFFLYSKLTVDKSRWQQGNVFSCSVM
HEALHNHYTQKSLSLSPGK (SEQ ID NO:69);

The antibodies hu2H6-M, hu2H6-SELFNS, hu9E5-M, hu9E5-SELF, hu9E5-SELFNS and 005M, APX005-SELF and APX005-SELFNS were prepared and confirmed by sequencing.

**EXAMPLE 13 TEST OF ANTI-CD40 ANTIBODY COMPRISING MUTATION(S) IN HEAVY CHAIN CONSTANT REGION TO ACTIVATE DC CELLS**

PBMCs were isolated from the peripheral blood of normal human subject, and then monocytes were sorted using CD14 MACS beads. RPMI 1640 medium comprising 25 ng/mL IL-4 and 50 ng/mL GM-CSF was added for cultivation for 6 days to induce MoDC cells (dendritic cells derived from monocytes).

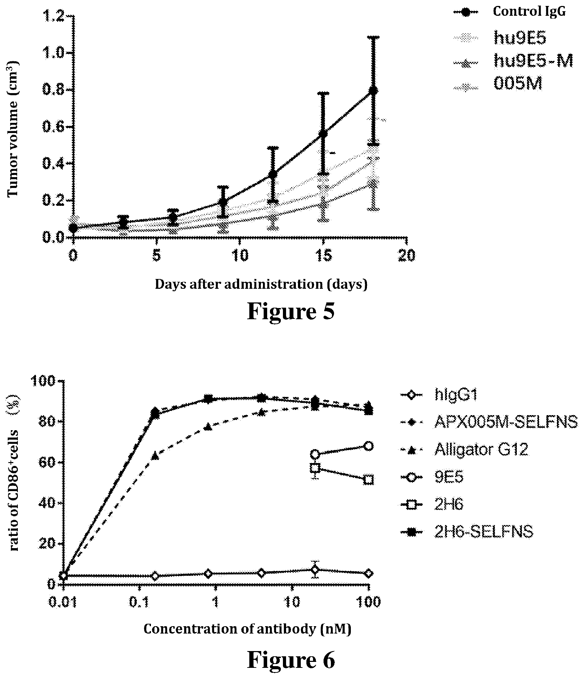

Cells were collected after 6 days, $1 \times 10^5$ cells were taken, and stained with CD209-PE, CD1a-PerCP/Cy5.5 and CD14-PE/Cy7 for analyzing whether MoDC has been successfully induced by FACS (the above operations are routine operations in the art). The successfully induced DCs were collected, each antibody to be tested and control antibody were added, and the corresponding concentration dilution gradients were set up to obtain antibody gradients: 0.01nM, 0.16nM, 0.8nM, 4nM, 20nM, 100nM. After cultivating for 48 hours, the cells were collected and stained using CD86 and HLA-DR staining, and data was collected by FACS. Both APX005M-SELFNS and 2H6-SELFNS showed stronger agonist activity than that of Alligator control antibody G12, and activated the activation molecule CD86 on the surface of DC cells in a dose-dependent way (See Figure 6).

**EXAMPLE 14 TEST OF ANTI-CD40 ANTIBODY COMPRISING MUTATION(S) IN HEAVY CHAIN CONSTANT REGION FOR ACTIVATION OF DC CELLS TO PRODUCE CYTOKINES**

PBMCs were isolated from the peripheral blood of normal human subject, and then monocytes were sorted using CD14 MACS beads. RPMI 1640 medium comprising 25 ng/mL IL-4 and 50 ng/mL GM-CSF was added for cultivation for 6 days to induce MoDC cells (dendritic cells derived from monocytes). Cells were collected after 6 days, $1 \times 10^5$ cells were taken, and stained with CD209-PE, CD1a-PerCP/Cy5.5 and CD14-PE/Cy7 for analyzing whether MoDC has been successfully induced by FACS (the above operations are routine operations in the art). The successfully induced DCs were collected, each antibody to be tested and control antibody were added, and the corresponding concentration dilution gradients were set up to obtain antibody gradients: 0.01nM, 0.16nM, 0.8nM, 4nM, 20nM, 100nM. After cultivating for 48 hours, the supernatant was collected and the content of IL-12 p40 was detected by ELISA.

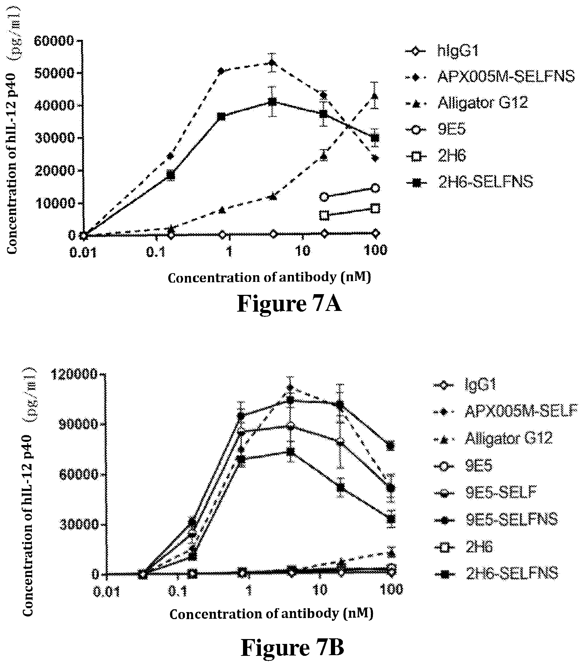

APX005M-SELFNS, APX005M-SELF, 2H6-SELFNS and 9E5-SELFNS all showed stronger agonist activity than that of Alligator control antibody G12, and promoted the secretion of cytokine IL-12 p40 from DC cells in a dose-dependent way. The results are shown in Figure 7A, Figure 7B and Table 12, Table 13.

Table 12. Results of anti-CD40 antibody promoting the secretion of cytokine IL-12 p40 from DC cells

| Concentration of antibody (nM) | hIgG1 | APX005M-SELFNS | Alligator G12 | 9E5 | 2H6 | 2H6-SELF NS |
|---|---|---|---|---|---|---|
| 100 | 65.205 ± 14.145 | 23129.857± 1123.371 | 42475.280± 4060.051 | 13998.987± 349.462 | 7668.062± 773.460 | 29300.717± 2741.181 |
| 20 | 76.817 ± 33.036 | 42648.367± 1338.211 | 24147.463± 1685.812 | 11397.870± 428.991 | 5633.816± 335.383 | 36753.370± 3812.485 |
| 4 | 35.016 ± 6.301 | 52787.687± 2854.792 | 11789.560± 375.848 | / | / | 40700.567± 4621.792 |
| 0.8 | 34.952 ± 4.832 | 50373.157± 518.778 | 7762.352± 189.066 | / | / | 36329.460± 790.604 |
| 0.16 | 20.899 ± 2.246 | 24311.430± 228.650 | 2110.560± 87.567 | / | / | 18411.147± 1639.285 |
| 0.01 | 35.562 ± 12.971 | 35.562± 12.971 | 35.562± 12.971 | / | / | 35.562± 12.971 |
| (Note: "/" means that the concentration was not tested). | | | | | | |

Table 13. Results of anti-CD40 antibody promoting the secretion of cytokine IL-12 p40 from DC cells

| Con. of antibody nM | IgGI | APX005M-SELF | Alligator G12 | 9E5 | 9E5-SELF | 9E5-SELFN S | 2H6 | 2H6-SELF NS |
|---|---|---|---|---|---|---|---|---|
| 100 | 0.000 | 51366.137 ± 6387,822 | 12099.219 ± 3111.489 | 2564.309 ± 605.549 | 50421.003 ±8453.659 | 75819.840 ± 2768.239 | 1426.910 ± 244.872 | 32000.920 ±5042.054 |
| 20 | 0.000 | 99039.430 ± 8959.730 | 6798.054 ± 1207.029 | 1320.558 ± 328.965 | 78407.500 ±15600.894 | 101297.360 ± 1534.936 | 2508.316 ± 627.554 | 51167.883± 5724.671 |
| 4 | 0.000 | 111253.733 ± 6263.173 | 1604.377± 533.314 | 889.822 ± 237.943 | 88086.533 ± 7487.812 | 103433.200 ±4360.661 | 1597.402 ±568.304 | 72797.640 ± 6296.468 |
| 0.8 | 0.000 | 74423.800 ±9486.879 | 698.189 ± 301.846 | 787.522 ± 278.889 | 84902.653 ± 7840.563 | 94355.880 ± 4121.238 | 564.707 ± 194.116 | 68460.980 ±4612.750 |
| 0.16 | 0.000 | 15199.523 ± 1874.331 | 0.000 | 0.000 | 24249.347 ± 5744.800 | 31196.040 ± 2942.471 | 0.000 | 10264.087 ± 2045.021 |
| 0.032 | 0.000 | 0.000 | 0.000 | 0.000 | 211.413± 105.920 | 642.149± 247.146 | 0.000 | 0.000 |
| 0.006 4 | 0.000 | 0.000 | 0.000 | 0.000 | 0.000 | 0.000 | 0.000 | 0.000 |

**EXAMPLE 15 INHIBITION OF MOUSE TUMOR GROWTH BY ANTI-CD40 ANTIBODY COMPRISING MUTATION(S) IN HEAVY CHAIN CONSTANT REGION**

In this example, the anti-tumor effect and safety of administration of CD40 antibody were evaluated by the size of tumor and the weight of mice on an MC38 tumor model of humanized hFcyR/hCD40 C57BL/6 mouse.

The method for cultivating and preparing MC38 cells: MC38 mouse colon cancer cell line was cultivated in DMEM (comprising 10% FBS, 1% penicillin-streptomycin, ImM sodium pyruvate and 10mM HEPES), and the cells were proliferated to reach a density of 80%-90% in the culture plate. Trypsin-EDTA (0.25%) was added and incubated at 37°C for 3 to 5 minutes for digestion, and medium comprising 10% FBS was used to terminate the reaction. The cells were centrifuged and washed for twice with PBS, and finally re-suspended in PBS to prepare a single cell suspension, and the cell density was adjusted to $10^7$ cells/mL for later use.

The method for establishing MC38 tumor model: the MC38 single cell suspension prepared above ($2\times10^6$ MC38 cells, 200 $\mu$L) was used to subcutaneously inoculate 32 humanized hFcyR/hCD40 C57BL/6 mice (provided by LI Fubin team, Department of Medicine, Shanghai Jiaotong University, kept at SPF level) at right flank on day 7. When the average tumor volume in mice reached about 55 mm$^3$, they were randomly divided into 4 groups with 8 mice in each group.

After grouping, a single dose of anti-CD40 antibody was administered intraperitoneally according to the regimen shown in Table 12. The tumor volume and the body weight was measured twice a week, and the data was recorded. Among them, the control IgG, hu9E5, and hu9E5-M were provided by Shanghai Hengrui Pharmaceutical Co., Ltd. and diluted with PBS to obtain a final concentration 0.3mg/mL.

Indicators for evaluating anti-tumor activity of antibody:

1) The tumor volume of the mice was measured continuously after the subject mice were divided into groups, and the size of tumor volume was used as an indicator to evaluate the anti-tumor activity of the antibody to be tested. The formula to calculate tumor volume (TV) is as follows:

$$TV = 0.5 \times L_{short} \times L_{short} \times L_{long},$$

where $L_{short}$ is the shortest diameter of tumor, and $L_{long}$ is the longest diameter of tumor.

2) T/C% is the relative tumor proliferation rate, i.e., the percentage value of tumor volume in the treatment group relative to that in the control group, at a certain time point, which is calculated as follows:

$$T/C\% = (T-T_0)/(C-C_0) \times 100$$

where T and C refer to the tumor volume at the end of the test; $T_0$, $C_0$ refer to the tumor volume at the beginning of the test.

3) Relative inhibition rate of tumor TGI (%) = (1-T/C) $\times$ 100%.

Data expression and statistical processing: All data were analyzed by GraphPad Prism 5.0 software. The data are expressed as Mean $\pm$ standard deviation, and one-way ANOVA analysis was used between groups. P<0.05 indicates that the difference is statistically significant.

Table 14. Test grouping and dosing regimen

| Test grouping | Number | Grouping | Dose (mg/kg) | Dosing regimen | Administration route |
|---|---|---|---|---|---|
| Group 1 | 8 | Control (Control IgG) | 3 | D0, D3, D6 | ip |
| Group 2 | 8 | hu9E5 | 3 | D0, D3, D6 | ip |
| Group 3 | 8 | hu9E5-M | 3 | D0, D3, D6 | ip |
| Group 4 | 8 | 005M | 3 | D0, D3, D6 | ip |
| (Note: once every three days, and for 3 times in total; ip: intraperitoneal injection). | | | | | |

The *in vivo* activity results for each group of antibodies in hFcyR/hCD40Tg mouse MC38 tumor model can be judged by the change in tumor volume. After the control antibody and test antibodies were administered on day 0, day 3, and day 6, the growth of mouse tumor volume was inhibited in hu9E5 group, hu9E5-M group and 005M group, when compared with that in control group (control IgG). The relative tumor inhibition rates were 42.0%, 68.9%, and 53.8%, respectively. Hu9E5 has certain anti-tumor activity (p>0.05), hu9E5-M and 005M have strong anti-tumor activity (p<0.05), and hu9E5-M has more beneficial effects than that of 005M, as shown in Table 15 and Figure 5.

Table 15. Tumor volume (cm)$^3$

|  | Grouping | tumor volume (Mean±SEM) | | Inhibition rate of tumor volume (%) |
| --- | --- | --- | --- | --- |
|  |  | D0 | D18 | D18 |
| Group 1 | Control (Control IgG) | 0.052± 0.015 | 0.794± 0.29 | / |
| Group 2 | hu9E5 | 0.053± 0.016 | 0.483± 0.159 | 42.0% |
| Group 3 | hu9E5-M | 0.060± 0.022 | 0.291± 0.139 | 68.9%* |
| Group 4 | 005M | 0.069± 0.03 | 0.412± 0.105 | 53.8%* |
| (Note: *p <0.05 or lower, indicating statistical significance). | | | | |

The results show that the amino acid mutation (from S to E) at position 266 corresponding to SEQ ID NO: 17 or at position 262 corresponding to SEQ ID NO: 19 can significantly improve the *in vivo* anti-tumor effect of the anti-CD40 antibodies of present application.

Although the specific embodiments of the present invention are described above, those skilled in the art should understand that these embodiments are only for exemplary purpose, various changes or modifications can be made to these embodiments without departing from the principle and essence of the present invention. Therefore, the protection scope of the present invention is defined by the appended claims.

## Claims

1. An anti-CD40 antibody or antigen-binding fragment thereof, which comprises mutation(s) in heavy chain constant region, wherein:

1) a light chain variable region of the antibody comprises LCDR1, LCDR2 and LCDR3 as shown in SEQ ID NO: 6, SEQ ID NO: 7 and SEQ ID NO: 8, respectively; and
a heavy chain variable region of the antibody comprises HCDR1, HCDR2 and HCDR3 as shown in SEQ ID NO: 3, SEQ ID NO: 4 and SEQ ID NO: 5, respectively;

or,

2) a light chain variable region of the antibody comprises LCDR1, LCDR2 and LCDR3 as shown in SEQ ID NO: 14, SEQ ID NO: 15 and SEQ ID NO: 16, respectively; and
a heavy chain variable region of the antibody comprises HCDR1, HCDR2 and HCDR3 as shown in SEQ ID NO: 11, SEQ ID NO: 12 and SEQ ID NO: 13, respectively;

or,

3) a light chain variable region of the antibody comprises LCDR1, LCDR2 and LCDR3 as shown in SEQ ID NO: 42, SEQ ID NO: 43 and SEQ ID NO: 44, respectively; and
a heavy chain variable region of the antibody comprises HCDR1, HCDR2 and HCDR3 as shown in SEQ ID NO: 39, SEQ ID NO: 40 and SEQ ID NO: 41, respectively;

or,

4) a light chain variable region of the antibody comprises LCDR1, LCDR2 and LCDR3 as shown in SEQ ID NO: 50, SEQ ID NO: 51 and SEQ ID NO: 52, respectively; and
a heavy chain variable region of the antibody comprises HCDR1, HCDR2 and HCDR as shown in SEQ ID NO: 47, SEQ ID NO: 48 and SEQ ID NO: 49, respectively;

or,

5) a light chain variable region of the antibody comprises LCDR1, LCDR2 and LCDR3 as shown in SEQ ID NO: 58, SEQ ID NO: 59 and SEQ ID NO: 60, respectively; and
a heavy chain variable region of the antibody comprises HCDR1, HCDR2 and HCDR3 as shown in SEQ ID NO: 55, SEQ ID NO: 56 and SEQ ID NO: 57, respectively.

2. The anti-CD40 antibody or antigen-binding fragment thereof according to claim 1, wherein the antibody or antigen-binding fragment thereof is a murine antibody, a chimeric antibody, a humanized antibody, a human antibody or antigen-binding fragment thereof.

3. The anti-CD40 antibody or antigen-binding fragment thereof according to claim 2, wherein the variable region amino acid sequences of the murine antibody or the chimeric antibody are selected from the group consisting of:

1) a heavy chain variable region as shown in SEQ ID NO: 1, and a light chain variable region as shown in SEQ ID NO: 2;
2) a heavy chain variable region as shown in SEQ ID NO: 9, and a light chain variable region as shown in SEQ ID NO: 10;
3) a heavy chain variable region as shown in SEQ ID NO: 37, and a light chain variable region as shown in SEQ ID NO: 38;
4) a heavy chain variable region as shown in SEQ ID NO: 45, and a light chain variable region as shown in SEQ ID NO: 46; and
5) a heavy chain variable region as shown in SEQ ID NO: 53, and a light chain variable region as shown in SEQ ID NO: 54.

4. The anti-CD40 antibody or antigen-binding fragment thereof according to claim 2, wherein the heavy chain variable region of the humanized antibody further comprises:

heavy chain FRs of human IgG1, IgG2, IgG3 or IgG4 or variants thereof,
preferably, heavy chain FRs of human IgG1, IgG2 or IgG4 ,
more preferably, heavy chain FRs of human IgG1 or IgG2.

5. The anti-CD40 antibody or antigen-binding fragment thereof according to claim 2, wherein:
the light chain FRs in the light chain variable region of the humanized antibody are derived from:

a human germline light chain IGkV1-33 sequence as shown in SEQ ID NO: 22, or
a human germline light chain IGkV2-28 sequence as shown in SEQ ID NO: 24;
the heavy chain FRs in the heavy chain variable region of the humanized antibody are derived from:
a human germline heavy chain IGHV1-69 sequence as shown in SEQ ID NO: 21, or
a human germline heavy chain IGHV1-2 sequence as shown in SEQ ID NO: 23;

preferably, the light chain FRs in the light chain variable region of the humanized antibody are derived from a human germline light chain IGkV1-33 as shown in SEQ ID NO: 22, and the heavy chain FRs in the heavy chain variable region of the humanized antibody are derived from a human germline heavy chain IGHV1-69 as shown in SEQ ID NO: 21; or,

the light chain FRs in the light chain variable region of the humanized antibody are derived from a human germline light chain IGkV2-28 as shown in SEQ ID NO: 24, and the heavy chain FRs in the heavy chain variable region are derived from a human germline heavy chain IGHV1-2 as shown in SEQ ID NO: 23.

6. The anti-CD40 antibody or antigen-binding fragment thereof according to claim 5, wherein:

the humanized antibody light chain is as shown in SEQ ID NO: 18 or SEQ ID NO: 20 or variant thereof; the variant has 0 to 10 amino acid mutation(s) in the light chain variable region, preferably the variant has mutation(s) at amino acid positions 2 and/or 3, more preferably the amino acid residue after mutation is I, V or L; and/or before mutation of the heavy chain constant region, the humanized antibody heavy chain is as shown in SEQ ID NO: 17 or SEQ ID NO: 19 or variant thereof; the variant has 0 to 10 amino acid mutation(s) in the heavy chain variable region, preferably the variant has mutation(s) at amino acid positions 6 and/or 8, more preferably the amino acid residue after mutation is I, A or L.

7. The anti-CD40 antibody or antigen-binding fragment thereof according to claim 6, wherein:

the heavy chain variable region is as shown in one of SEQ ID NOs: 25-30 or variant thereof,
the light chain variable region is as shown in one of SEQ ID NOs: 31-36 or variant thereof;
preferably, selected from the following:

the heavy chain variable region is as shown in SEQ ID NO: 26 or variant thereof, and the light chain variable region is as shown in SEQ ID NO: 33 or variant thereof; or
the heavy chain variable region is as shown in SEQ ID NO: 30 or variant thereof, and the light chain variable region is as shown in SEQ ID NO: 34 or variant thereof; or
the heavy chain variable region is as shown in SEQ ID NO: 17 or variant thereof, and the light chain variable region is as shown in SEQ ID NO: 18 or variant thereof; or
the heavy chain variable region is as shown in SEQ ID NO: 19 or variant thereof, and the light chain variable region is as shown in SEQ ID NO: 20 or variant thereof.

8. The anti-CD40 antibody or antigen-binding fragment thereof according to any one of claims 1-7, wherein the heavy chain comprises mutation(s) of amino acid residue(s) at position(s) selected from the group consisting of:

1) position 262 corresponding to SEQ ID NO: 19 or position 266 corresponding to SEQ ID NO: 17; and/or
2) position 320 corresponding to SEQ ID NO: 19 or position 324 corresponding to SEQ ID NO: 17; and/or
3) position 323 corresponding to SEQ ID NO: 19 or position 327 corresponding to SEQ ID NO: 17.

9. The anti-CD40 antibody or antigen-binding fragment thereof according to claim 8, wherein the heavy chain comprises mutation(s) selected from the group consisting of:

1) the amino acid residue at position 262 corresponding to SEQ ID NO: 19 or at position 266 corresponding to SEQ ID NO: 17 is mutated to glutamic acid; and/or
2) the amino acid residue at position 320 corresponding to SEQ ID NO: 19 or at position 324 corresponding to SEQ ID NO: 17 is mutated to serine; and/or
3) the amino acid residue at position 323 corresponding to SEQ ID NO: 19 or at position 327 corresponding to SEQ ID NO: 17 is mutated to phenylalanine.

10. The anti-CD40 antibody or antigen-binding fragment thereof according to claim 9, wherein the heavy chain comprises mutation(s) selected from the following, or the combination thereof:

1) 262E corresponding to SEQ ID NO: 19;
2) 262E and 323F corresponding to SEQ ID NO: 19;
3) 262E, 320S and 323F corresponding to SEQ ID NO: 19;
4) 266E corresponding to SEQ ID NO: 17;
5) 266E, 324S and 327F corresponding to SEQ ID NO: 17.

**11.** The anti-CD40 antibody or antigen-binding fragment thereof according to any one of claims 1-2 and 4-10, wherein the humanized antibody comprises:
a heavy chain as shown in SEQ ID NO: 61 or 62, and a light chain as shown in SEQ ID NO: 18; or the humanized antibody comprises:

a heavy chain as shown in SEQ ID NO: 63, 64 or 67, and
a light chain as shown in SEQ ID NO: 20.

**12.** The anti-CD40 antibody or antigen-binding fragment thereof according to any one of claims 1-11, wherein the amino acid residue at the carboxyl terminus of the heavy chain is mutated to alanine residue;
preferably, the amino acid residue at the carboxyl terminus of the heavy chain as shown in SEQ ID NO: 61, 62, 63, 64 or 67 is mutated to alanine residue.

**13.** A single-chain antibody, which comprises:
the light chain variable region and the heavy chain variable region as defined in any one of claims 1-12.

**14.** An antibody-drug conjugate, wherein the antibody comprises the light chain variable region and the heavy chain variable region as defined in any one of claims 1-12; preferably, comprises the anti-CD40 antibody or antigen-binding fragment thereof as defined in any one of claims 1-12.

**15.** A nucleic acid molecule encoding the anti-CD40 antibody or the antigen-binding fragment of any one of claims 1-12, or the single-chain antibody of claim 13.

**16.** A vector comprising the nucleic acid molecule of claim 15.

**17.** A host cell comprising or expressing the vector of claim 16.

**18.** The host cell according to claim 17, wherein the host cell is selected from the group consisting of bacterium, yeast, and mammalian cell;

preferably, the bacterium is *Escherichia coli*;
preferably, the yeast is *Pichia pastoris*;
preferably, the mammalian cell is Chinese hamster ovary cell or human embryonic kidney 293 cell.

**19.** A pharmaceutical composition comprising:

the anti-CD40 antibody or antigen-binding fragment thereof of any one of claims 1-12, the single-chain antibody of claim 13, or the antibody-drug conjugate of claim 14, and
a pharmaceutically acceptable excipient, diluent or carrier.

**20.** Use of any one selected from the group consisting of the anti-CD40 antibody or antigen-binding fragment thereof of any one of claims 1-12, the single-chain antibody of claim 13, the antibody-drug conjugate of claim 14 and the pharmaceutical composition of claim 19, in the preparation of a medicament, and the medicament is used to treat or prevent a CD40-mediated or CD40L-mediated disease or condition;

preferably, the disease or condition is cancer;
most preferably, the cancer is selected from the group consisting of: lymphoma, breast cancer, ovarian cancer, prostate cancer, pancreatic cancer, kidney cancer, lung cancer, liver cancer, stomach cancer, colorectal cancer, bladder cancer, rhabdomyosarcoma, esophageal cancer, cervical cancer, multiple myeloma, leukemia, gallbladder cancer, glioblastoma and melanoma.

**21.** Use of any one selected from the group consisting of the anti-CD40 antibody or antigen-binding fragment thereof of any one of claims 1-12, the single-chain antibody of claim 13, the antibody-drug conjugate of claim 14 and the pharmaceutical composition of claim 19 in the preparation of a medicament; and the medicament is used to improve symptom(s) of a patient suffering from an autoimmune disease or an inflammatory disease.

**22.** A method for preventing or treating CD40-mediated or CD40L-mediated disease, the method comprising contacting a subject with a prophylactically effective amount or a therapeutically effective amount of any one selected from the group consisting of the anti-CD40 antibody or antigen-binding fragment thereof of any one of claims 1-12, the single-chain antibody of claim 13, the antibody-drug conjugate of claim 14 and the pharmaceutical composition of claim 19;

preferably, the disease is cancer;
most preferably, the cancer is selected from the group consisting of lymphoma, breast cancer, ovarian cancer, prostate cancer, pancreatic cancer, kidney cancer, lung cancer, liver cancer, stomach cancer, colorectal cancer, bladder cancer, rhabdomyosarcoma, esophageal cancer, cervical cancer, multiple myeloma, leukemia, gallbladder cancer, glioblastoma and melanoma.

23. A method for improving symptom(s) of an autoimmune disease or an inflammatory disease, the method comprising contacting a subject with a prophylactically effective amount or a therapeutically effective amount of any one selected from the group consisting of the anti-CD40 antibody or antigen-binding fragment thereof of any one of claims 1-12, the single-chain antibody of claim 13, the antibody-drug conjugate of claim 14, and the pharmaceutical composition of claim 19.

## Figures

Figure 1

Figure 2

Figure 3

Figure 4

Figure 5

Figure 6

**Figure 7A**

**Figure 7B**

## INTERNATIONAL SEARCH REPORT

| International application No. |
| --- |
| **PCT/CN2019/121941** |

| A. | CLASSIFICATION OF SUBJECT MATTER |
| --- | --- |

C07K 16/28(2006.01)i; A61P 35/00(2006.01)i; A61K 39/395(2006.01)i; A61P 37/00(2006.01)i; C12N 15/13(2006.01)i; C12N 15/63(2006.01)i; A61P 29/00(2006.01)i

According to International Patent Classification (IPC) or to both national classification and IPC

| B. | FIELDS SEARCHED |
| --- | --- |

Minimum documentation searched (classification system followed by classification symbols)

C07K; A61P; A61K; C12N

Documentation searched other than minimum documentation to the extent that such documents are included in the fields searched

Electronic data base consulted during the international search (name of data base and, where practicable, search terms used)

DWPI; CNABS; CNMED; CNTXT; WOTXT; USTXT; EPTXT; PUBMED; CNKI; ELESVIER; 万方; WANGFANG; NCBI GenBank; EMBL-EBI; 中国专利生物序列检索系统; China Patents Biological Sequence Search System: CD40, 抗体, CDR, 可变区, 人源, 框架区, 重链, 突变, 单链抗体, 抗体-药物, 偶联物; antibod, VH, VL, humanized, FR, heavy chain, Fc, mutat +, scFv, ADC, antibody-drug, conjugate.

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| PX | WO 2018219327 A1 (JIANGSU HENGRUI MEDICINE CO., LTD. et al.) 06 December 2018 (2018-12-06) <br> claims 4-13, 16, and 17, and description, embodiments 3-9 | 1-7, 11-23 |
| A | CN 102918063 A (BOEHRINGER INGELHEIM INTERNATIONAL GMBH) 06 February 2013 (2013-02-06) <br> entire document | 1-23 |
| A | CN 101508734 A (KYOWA HAKKO KIRIN CO., LTD.) 19 August 2009 (2009-08-19) <br> entire document | 1-23 |
| A | CN 104788565 A (XENCOR, INC.) 22 July 2015 (2015-07-22) <br> entire document | 1-23 |
| A | WO 2015091853 A2 (ALLIGATOR BIOSCIENCE AB) 25 June 2015 (2015-06-25) <br> entire document | 1-23 |
| A | WO 2017205742 A1 (ABBVIE BIOTHERAPEUTICS INC.) 30 November 2017 (2017-11-30) <br> entire document | 1-23 |

☑ Further documents are listed in the continuation of Box C.    ☑ See patent family annex.

| * | Special categories of cited documents: |
| --- | --- |
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
| --- | --- |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| **17 February 2020** | **25 February 2020** |

| Name and mailing address of the ISA/CN | Authorized officer |
| --- | --- |
| **China National Intellectual Property Administration (ISA/ CN)** <br> **No. 6, Xitucheng Road, Jimenqiao Haidian District, Beijing 100088** <br> **China** | |
| Facsimile No. **(86-10)62019451** | Telephone No. |

Form PCT/ISA/210 (second sheet) (January 2015)

EP 3 892 634 A1

**INTERNATIONAL SEARCH REPORT**

| International application No. |
| --- |
| **PCT/CN2019/121941** |

**C.    DOCUMENTS CONSIDERED TO BE RELEVANT**

| Category* | Citation of document, with indication, where appropriate, of the relevant passages | Relevant to claim No. |
| --- | --- | --- |
| A | US 2018273630 A1 (APEXIGEN, INC) 27 September 2018 (2018-09-27) <br> entire document | 1-23 |

**INTERNATIONAL SEARCH REPORT**

International application No.

**PCT/CN2019/121941**

**Box No. I      Nucleotide and/or amino acid sequence(s) (Continuation of item 1.c of the first sheet)**

1.  With regard to any nucleotide and/or amino acid sequence disclosed in the international application, the international search was carried out on the basis of a sequence listing:

    a.  ☑ forming part of the international application as filed:

        ☑ in the form of an Annex C/ST.25 text file.

        ☐ on paper or in the form of an image file.

    b.  ☐ furnished together with the international application under PCT Rule 13*ter*.1(a) for the purposes of international search only in the form of an Annex C/ST.25 text file.

    c.  ☐ furnished subsequent to the international filing date for the purposes of international search only:

        ☐ in the form of an Annex C/ST.25 text file (Rule 13*ter*.1(a)).

        ☐ on paper or in the form of an image file (Rule 13*ter*.1(b) and Administrative Instructions, Section 713).

2.  ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3.  Additional comments:

Form PCT/ISA/210 (continuation of first sheet) (January 2015)

**INTERNATIONAL SEARCH REPORT**

International application No.

**PCT/CN2019/121941**

**Box No. II     Observations where certain claims were found unsearchable (Continuation of item 2 of first sheet)**

This international search report has not been established in respect of certain claims under Article 17(2)(a) for the following reasons:

1. ☑    Claims Nos.: **22-23**
    because they relate to subject matter not required to be searched by this Authority, namely:

    [1]    The subject matter of claims 22 and 23 relates to a method for preventing and treating diseases in a subject, which falls under the method of surgery or therapy for the treatment of the human or animal body according to PCT Rule 39.1(iv). Nevertheless, the search is still performed for the corresponding pharmaceutical use.

2. ☐    Claims Nos.:
    because they relate to parts of the international application that do not comply with the prescribed requirements to such an extent that no meaningful international search can be carried out, specifically:

3. ☐    Claims Nos.:
    because they are dependent claims and are not drafted in accordance with the second and third sentences of Rule 6.4(a).

Form PCT/ISA/210 (continuation of first sheet) (January 2015)

### INTERNATIONAL SEARCH REPORT
#### Information on patent family members

| International application No. |
| --- |
| **PCT/CN2019/121941** |

| Patent document cited in search report | | | Publication date (day/month/year) | Patent family member(s) | | | Publication date (day/month/year) |
| --- | --- | --- | --- | --- | --- | --- | --- |
| WO | 2018219327 | A1 | 06 December 2018 | CA | 3064298 | A1 | 06 December 2018 |
| | | | | TW | 201902925 | A | 16 January 2019 |
| | | | | CN | 110267989 | A | 20 September 2019 |
| CN | 102918063 | A | 06 February 2013 | UY | 33306 | A | 31 October 2011 |
| | | | | EP | 2796467 | A1 | 29 October 2014 |
| | | | | CO | 6612271 | A2 | 01 February 2013 |
| | | | | SG | 183947 | A1 | 30 October 2012 |
| | | | | UA | 107827 | C2 | 25 February 2015 |
| | | | | US | 2014179907 | A1 | 26 June 2014 |
| | | | | AP | 4082 | A | 29 March 2017 |
| | | | | US | 2015315282 | A1 | 05 November 2015 |
| | | | | MX | 2012011112 | A | 15 October 2012 |
| | | | | JP | 2013523130 | A | 17 June 2013 |
| | | | | LT | 2796467 | T | 10 May 2018 |
| | | | | EP | 2552965 | A2 | 06 February 2013 |
| | | | | PL | 2796467 | T3 | 31 July 2018 |
| | | | | AP | 201206457 | D0 | 31 August 2012 |
| | | | | GE | P20166486 | B | 10 June 2016 |
| | | | | NZ | 602020 | A | 30 May 2014 |
| | | | | AU | 2011235214 | B2 | 23 April 2015 |
| | | | | KR | 101760242 | B1 | 21 July 2017 |
| | | | | HR | P20180542 | T1 | 04 May 2018 |
| | | | | JP | 5752865 | B1 | 22 July 2015 |
| | | | | BR | 112012024713 | A2 | 10 January 2017 |
| | | | | CL | 2012002737 | A1 | 15 February 2013 |
| | | | | HU | E038788 | T2 | 28 November 2018 |
| | | | | IL | 236740 | A | 30 November 2016 |
| | | | | MX | 340140 | B | 28 June 2016 |
| | | | | PT | 2796467 | T | 02 May 2018 |
| | | | | DK | 2796467 | T3 | 07 May 2018 |
| | | | | EA | 201201357 | A1 | 30 April 2013 |
| | | | | CY | 1120181 | T1 | 12 December 2018 |
| | | | | EP | 2796467 | B1 | 28 March 2018 |
| | | | | MA | 34091 | B1 | 05 March 2013 |
| | | | | EP | 3178851 | A1 | 14 June 2017 |
| | | | | MY | 155950 | A | 31 December 2015 |
| | | | | TW | I494126 | B | 01 August 2015 |
| | | | | PE | 20130159 | A1 | 10 March 2013 |
| | | | | KR | 20130064723 | A | 18 June 2013 |
| | | | | PE | 01592013 | A1 | 10 March 2013 |
| | | | | JP | 2015145383 | A | 13 August 2015 |
| | | | | IL | 236740 | D0 | 26 February 2015 |
| | | | | US | 2011243932 | A1 | 06 October 2011 |
| | | | | AU | 2011235214 | A1 | 13 September 2012 |
| | | | | US | 2019169301 | A1 | 06 June 2019 |
| | | | | ES | 2665592 | T3 | 26 April 2018 |
| | | | | EA | 031872 | B1 | 29 March 2019 |
| | | | | CA | 2794332 | A1 | 06 October 2011 |
| | | | | RS | 57114 | B1 | 29 June 2018 |
| | | | | CN | 102918063 | B | 29 June 2016 |

Form PCT/ISA/210 (patent family annex) (January 2015)

| International application No. |
| --- |
| **PCT/CN2019/121941** |

| Patent document cited in search report | | | Publication date (day/month/year) | Patent family member(s) | | | Publication date (day/month/year) |
| --- | --- | --- | --- | --- | --- | --- | --- |
| | | | | CA | 2794332 | C | 14 May 2019 |
| | | | | IL | 221639 | A | 31 July 2016 |
| CN | 101508734 | A | 19 August 2009 | AT | 374214 | T | 15 October 2007 |
| | | | | DK | 1391464 | T3 | 14 January 2008 |
| | | | | US | 2009123466 | A1 | 14 May 2009 |
| | | | | CA | 2658221 | C | 27 November 2012 |
| | | | | AU | 2009200017 | A1 | 05 February 2009 |
| | | | | US | 7193064 | B2 | 20 March 2007 |
| | | | | US | 2007077242 | A1 | 05 April 2007 |
| | | | | EP | 2011802 | A2 | 07 January 2009 |
| | | | | US | 2004120948 | A1 | 24 June 2004 |
| | | | | EP | 2009027 | B1 | 21 May 2014 |
| | | | | EP | 2011802 | A3 | 15 April 2009 |
| | | | | PT | 1391464 | E | 15 November 2007 |
| | | | | CA | 2658221 | A1 | 07 November 2002 |
| | | | | US | 7537763 | B2 | 26 May 2009 |
| | | | | US | 2010234578 | A1 | 16 September 2010 |
| | | | | EP | 2009027 | A1 | 31 December 2008 |
| CN | 104788565 | A | 22 July 2015 | IL | 265538 | D0 | 30 May 2019 |
| | | | | US | 8858937 | B2 | 14 October 2014 |
| | | | | AU | 2008261120 | A1 | 15 January 2009 |
| | | | | CN | 1867583 | A | 22 November 2006 |
| | | | | US | 2013243762 | A1 | 19 September 2013 |
| | | | | US | 2009068177 | A1 | 12 March 2009 |
| | | | | US | 2015079082 | A1 | 19 March 2015 |
| | | | | US | 8809503 | B2 | 19 August 2014 |
| | | | | US | 2016347837 | A1 | 01 December 2016 |
| | | | | SI | 2368911 | T1 | 30 October 2017 |
| | | | | US | 9353187 | B2 | 31 May 2016 |
| | | | | US | 2016318993 | A1 | 03 November 2016 |
| | | | | US | 2004132101 | A1 | 08 July 2004 |
| | | | | DK | 2368911 | T3 | 11 September 2017 |
| | | | | EP | 2368911 | B1 | 10 May 2017 |
| | | | | US | 2013156758 | A1 | 20 June 2013 |
| | | | | AU | 2008261120 | B2 | 15 March 2012 |
| | | | | AU | 2004236160 | B2 | 23 October 2008 |
| | | | | US | 10184000 | B2 | 22 January 2019 |
| | | | | EP | 1620467 | A2 | 01 February 2006 |
| | | | | CA | 2916863 | C | 21 August 2018 |
| | | | | HU | E034268 | T2 | 28 February 2018 |
| | | | | JP | 2007525443 | A | 06 September 2007 |
| | | | | CA | 2916863 | A1 | 18 November 2004 |
| | | | | KR | 100890586 | B1 | 25 March 2009 |
| | | | | US | 2009092599 | A1 | 09 April 2009 |
| | | | | PL | 2368911 | T3 | 29 December 2017 |
| | | | | KR | 20050116400 | A | 12 December 2005 |
| | | | | IL | 247597 | D0 | 30 November 2016 |
| | | | | PL | 2368911 | T4 | 29 December 2017 |
| | | | | WO | 2004099249 | A2 | 18 November 2004 |
| | | | | US | 8383109 | B2 | 26 February 2013 |

Form PCT/ISA/210 (patent family annex) (January 2015)

**INTERNATIONAL SEARCH REPORT**
Information on patent family members

International application No.

**PCT/CN2019/121941**

| Patent document cited in search report | | | Publication date (day/month/year) | Patent family member(s) | | | Publication date (day/month/year) |
|---|---|---|---|---|---|---|---|
| | | | | US | 10183999 | B2 | 22 January 2019 |
| | | | | JP | 4578467 | B2 | 10 November 2010 |
| | | | | WO | 2004099249 | A3 | 26 January 2006 |
| | | | | EP | 3101030 | A1 | 07 December 2016 |
| | | | | AU | 2004236160 | A1 | 18 November 2004 |
| | | | | CA | 2524399 | A1 | 18 November 2004 |
| | | | | CN | 102633880 | B | 25 February 2015 |
| | | | | BR | PI0410031 | A | 25 April 2006 |
| | | | | US | 8093359 | B2 | 10 January 2012 |
| | | | | US | 2012230980 | A1 | 13 September 2012 |
| | | | | KR | 20070116176 | A | 06 December 2007 |
| | | | | KR | 100956110 | B1 | 10 May 2010 |
| | | | | EP | 2368911 | A1 | 28 September 2011 |
| | | | | CN | 102633880 | A | 15 August 2012 |
| | | | | US | 2016347825 | A1 | 01 December 2016 |
| | | | | US | 2013156754 | A1 | 20 June 2013 |
| | | | | US | 9193798 | B2 | 24 November 2015 |
| WO | 2015091853 | A2 | 25 June 2015 | WO | 2015091853 | A3 | 20 August 2015 |
| | | | | GB | 201322583 | D0 | 05 February 2014 |
| | | | | EP | 3083688 | A2 | 26 October 2016 |
| | | | | US | 2016311916 | A1 | 27 October 2016 |
| WO | 2017205742 | A1 | 30 November 2017 | AU | 2017271602 | A1 | 13 December 2018 |
| | | | | US | 2019071509 | A1 | 07 March 2019 |
| | | | | SG | 11201810522 U | A | 28 December 2018 |
| | | | | CL | 2018003366 | A1 | 15 March 2019 |
| | | | | CN | 109476750 | A | 15 March 2019 |
| | | | | CO | 2018012699 | A2 | 20 August 2019 |
| | | | | MX | 2018014630 | A | 10 June 2019 |
| | | | | TW | 201806971 | A | 01 March 2018 |
| | | | | CA | 3025347 | A1 | 30 November 2017 |
| | | | | EP | 3464361 | A1 | 10 April 2019 |
| | | | | BR | 112018074468 | A2 | 06 March 2019 |
| | | | | KR | 20190014524 | A | 12 February 2019 |
| | | | | US | 10400041 | B2 | 03 September 2019 |
| | | | | IL | 263223 | D0 | 31 December 2018 |
| | | | | US | 2018186889 | A1 | 05 July 2018 |
| | | | | PH | 12018502456 | A1 | 21 October 2019 |
| | | | | US | 10519243 | B2 | 31 December 2019 |
| | | | | PE | 20190970 | A1 | 09 July 2019 |
| | | | | JP | 2019523221 | A | 22 August 2019 |
| | | | | US | 2017342159 | A1 | 30 November 2017 |
| | | | | AR | 108611 | A1 | 05 September 2018 |
| | | | | US | 10023645 | B1 | 17 July 2018 |
| | | | | DO | P2018000258 | A | 15 February 2019 |
| US | 2018273630 | A1 | 27 September 2018 | CN | 104918957 | A | 16 September 2015 |
| | | | | US | 9676861 | B2 | 13 June 2017 |
| | | | | CN | 104918957 | B | 16 November 2018 |
| | | | | KR | 20150079787 | A | 08 July 2015 |
| | | | | US | 2017246297 | A1 | 31 August 2017 |
| | | | | US | 9994640 | B2 | 12 June 2018 |

Form PCT/ISA/210 (patent family annex) (January 2015)

**INTERNATIONAL SEARCH REPORT**
Information on patent family members

International application No.

**PCT/CN2019/121941**

| Patent document cited in search report | Publication date (day/month/year) | Patent family member(s) | | | Publication date (day/month/year) |
|---|---|---|---|---|---|
| | | AU | 2013337903 | A1 | 07 May 2015 |
| | | EP | 2914627 | A1 | 09 September 2015 |
| | | AU | 2013337903 | B2 | 16 August 2018 |
| | | CN | 109265552 | A | 25 January 2019 |
| | | US | 2014120103 | A1 | 01 May 2014 |
| | | JP | 2015533853 | A | 26 November 2015 |
| | | HK | 1213581 | A1 | 08 July 2016 |
| | | CA | 2888763 | A1 | 08 May 2014 |
| | | NZ | 707086 | A | 26 July 2019 |
| | | WO | 2014070934 | A1 | 08 May 2014 |
| | | JP | 2018166522 | A | 01 November 2018 |
| | | CN | 110066335 | A | 30 July 2019 |
| | | AU | 2018256638 | A1 | 29 November 2018 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 201811448228
- CN 2018089252 W
- CN 1198647
- CN 1369015
- CN 1582165
- CN 100430419
- CN 101014386
- CN 101237882
- CN 101289510
- CN 101490086
- CN 103842382
- CN 104918957
- WO 2002028904 A
- WO 2011123489 A
- WO 2012149356 A
- WO 2013034904 A
- WO 201509853 A
- WO 2016196314 A
- WO 2017040932 A
- WO 2017004006 A
- WO 2006019447 A
- WO 2014145806 A
- US 8734791 B
- US 9657106 B
- US 8084582 B
- WO 2008150494 A
- WO 2004099249 A
- CN 104918957 A

**Non-patent literature cited in the description**

- **TONG et al.** *Cancer Gene Therapy,* 2003, vol. 10, 1-13
- **VAN MIERLO et al.** *Proc. Natl. Acad. Sci. USA,* 2002, vol. 99, 5561-5566
- *J. Biol. Chem,* 1968, vol. 243, 3558
- **HOLLIGER ; HUDSON.** *Nat. Biotechnol.,* 2005, vol. 23, 1126-1136
- Antibody Experimental Technology Guide of Cold Spring Harbor
- The Immunoglobulin FactsBook. 2001
- **WATSON et al.** Molecular Biology of the Gene. The Benjamin/Cummings Pub. Co, 1987, 224

Sequence listing

<110> JIANGSU HENGRUI MEDICINE CO., LTD.;
SHANGHAI HENGRUI PHARMACEUTICAL CO., LTD.

<120> ANTI-CD40 ANTIBODY, ANTIGEN-BINDING FRAGMENT AND PHARMACEUTICAL USE THEREOF

<130> 719108CPCT

<160> 69

<170> SIPOSequenceListing 1.0

<210> 1
<211> 116
<212> PRT
<213> Artificial Sequence

<220>
<221> CHAIN
<222> (1)..(116)
<223> Sequence of heavy chain variable region 2H6 HCVR of murine monoclonal antibody 2H6

<400> 1
```
Gln Val Gln Leu Gln Gln Ser Gly Ala Glu Leu Val Arg Pro Gly Thr
1               5                   10                  15
Ser Val Lys Val Ser Cys Lys Ala Ser Gly Tyr Ala Phe Ser Asp Tyr
            20                  25                  30
Leu Ile Glu Trp Ala Lys Gln Arg Pro Gly Gln Gly Leu Glu Trp Ile
        35                  40                  45
Gly Val Ile Asn Pro Gly Ser Gly Gly Ser Asn Tyr Asn Glu Lys Ile
    50                  55                  60
Lys Asp Arg Ala Thr Leu Thr Ala Asp Lys Ser Ser Ser Thr Ala Tyr
65                  70                  75                  80
Met Gln Leu Ser Ser Leu Thr Ser Glu Asp Ser Ala Val Tyr Phe Cys
                85                  90                  95
Ala Arg Gly Gly Gly Gly Phe Thr Tyr Trp Gly Gln Gly Thr Leu Val
            100                 105                 110
Thr Val Ser Ala
            115
```

<210> 2
<211> 107
<212> PRT
<213> Artificial Sequence

<220>
<221> CHAIN
<222> (1)..(107)
<223> Sequence of light chain variable region 2H6 LCVR of murine monoclonal antibody 2H6

<400> 2
```
Glu Ile Gln Leu Thr Gln Thr Thr Ser Ser Leu Ser Ala Ser Leu Gly
1               5                   10                  15
Asp Arg Val Thr Ile Ser Cys Arg Ala Ser Gln Asp Ile Ser Asn Tyr
            20                  25                  30
Leu Asn Trp Tyr Gln Gln Lys Pro Asp Gly Thr Ile Lys Leu Leu Leu
        35                  40                  45
```

```
            Asn Phe Ala Ser Arg Leu His Ser Gly Val Pro Ser Arg Phe Ser Gly
                50                  55                  60
            Ser Gly Ser Gly Thr Asp Phe Phe Leu Thr Ile Ser Asn Leu Glu Gln
            65                  70                  75                  80
            Asp Asp Ile Ala Thr Tyr Phe Cys Gln Gln Gly Ser Thr Leu Pro Trp
                            85                  90                  95
            Thr Phe Gly Gly Gly Thr Lys Leu Glu Ile Lys
                        100                 105


            <210>   3
            <211>   10
            <212>   PRT
            <213>   Artificial Sequence


            <220>
            <221>   DOMAIN
            <222>   (1)..(10)
            <223>   2H6 HCDR1


            <400>   3
            Gly Tyr Ala Phe Ser Asp Tyr Leu Ile Glu
            1               5                   10


            <210>   4
            <211>   17
            <212>   PRT
            <213>   Artificial Sequence


            <220>
            <221>   DOMAIN
            <222>   (1)..(17)
            <223>   2H6 HCDR2


            <400>   4
            Val Ile Asn Pro Gly Ser Gly Gly Ser Asn Tyr Asn Glu Lys Ile Lys
            1               5                   10                  15
            Asp


            <210>   5
            <211>   7
            <212>   PRT
            <213>   Artificial Sequence


            <220>
            <221>   DOMAIN
            <222>   (1)..(7)
            <223>   2H6 HCDR3


            <400>   5
            Gly Gly Gly Gly Phe Thr Tyr
            1               5


            <210>   6
            <211>   11
            <212>   PRT
            <213>   Artificial Sequence


            <220>
            <221>   DOMAIN
```

31

```
<222>   (1)..(11)
<223>   2H6 LCDR1


<400>   6
Arg Ala Ser Gln Asp Ile Ser Asn Tyr Leu Asn
1               5                   10


<210>   7
<211>   7
<212>   PRT
<213>   Artificial Sequence

<220>
<221>   DOMAIN
<222>   (1)..(7)
<223>   2H6 LCDR2


<400>   7
Phe Ala Ser Arg Leu His Ser
1               5


<210>   8
<211>   9
<212>   PRT
<213>   Artificial Sequence

<220>
<221>   DOMAIN
<222>   (1)..(9)
<223>   2H6 LCDR3


<400>   8
Gln Gln Gly Ser Thr Leu Pro Trp Thr
1               5


<210>   9
<211>   112
<212>   PRT
<213>   Artificial Sequence

<220>
<221>   CHAIN
<222>   (1)..(112)
<223>   Sequence of heavy chain variable region 9E5 HCVR of 9E5


<400>   9
Gln Val Gln Leu Gln Gln Pro Gly Ala Asp Leu Val Lys Pro Gly Ala
1               5                   10                  15
Ser Val Lys Met Ser Cys Lys Ala Ser Gly Tyr Ile Leu Thr Thr Tyr
                20                  25                  30
Trp Ile Thr Trp Val Lys Gln Arg Pro Gly Gln Gly Leu Glu Trp Ile
                35                  40                  45
Gly Asp Ile His Pro Gly Ser Gly Ser Thr Lys Tyr Asn Glu Lys Phe
            50                  55                  60
Lys Ser Lys Ala Thr Leu Thr Val Asp Thr Ser Ser Ser Thr Ala Tyr
65                  70                  75                  80
Met Gln Leu Thr Arg Leu Ser Ser Glu Asp Ser Ala Val Tyr Tyr Cys
                85                  90                  95
Ala Arg Arg Asp Tyr Trp Gly Gln Gly Thr Thr Leu Thr Val Ser Ser
```

100                    105                    110

```
<210>  10
<211>  112
<212>  PRT
<213>  Artificial Sequence

<220>
<221>  CHAIN
<222>  (1)..(112)
<223>  Sequence of light chain variable region 9E5 LCVR of 9E5


<400>  10
Asp Val Leu Met Thr Gln Ser Pro Leu Ser Leu Pro Val Ser Leu Gly
1               5                   10                  15
Asp Gln Ala Ser Ile Ser Cys Arg Ser Ser Gln Asn Ile Val Asn Ser
            20                  25                  30
Gln Gly Asn Thr Tyr Leu Glu Trp Tyr Leu Gln Lys Pro Gly Glu Ser
        35                  40                  45
Pro Lys Leu Leu Ile Tyr Lys Val Thr Asn Arg Phe Ser Gly Val Pro
    50                  55                  60
Asp Arg Phe Ser Gly Ser Gly Ser Gly Thr Asp Phe Thr Leu Lys Ile
65                  70                  75                  80
Ser Arg Val Glu Ala Glu Asp Leu Gly Val Tyr Tyr Cys Phe Gln Ala
                85                  90                  95
Ser Leu Val Pro Trp Thr Phe Gly Gly Gly Thr Lys Leu Glu Ile Lys
            100                 105                 110

<210>  11
<211>  10
<212>  PRT
<213>  Artificial Sequence

<220>
<221>  DOMAIN
<222>  (1)..(10)
<223>  9E5 HCDR1


<400>  11
Gly Tyr Ile Leu Thr Thr Tyr Trp Ile Thr
1               5                   10

<210>  12
<211>  17
<212>  PRT
<213>  Artificial Sequence

<220>
<221>  DOMAIN
<222>  (1)..(17)
<223>  9E5 HCDR2


<400>  12
Asp Ile His Pro Gly Ser Gly Ser Thr Lys Tyr Asn Glu Lys Phe Lys
1               5                   10                  15
Ser

<210>  13
<211>  3
```

```
<212>   PRT
<213>   Artificial Sequence


<220>
<221>   DOMAIN
<222>   (1)..(3)
<223>   9E5 HCDR3



<400>   13
Arg Asp Tyr
1


<210>   14
<211>   16
<212>   PRT
<213>   Artificial Sequence


<220>
<221>   DOMAIN
<222>   (1)..(16)
<223>   9E5 LCDR1



<400>   14
Arg Ser Ser Gln Asn Ile Val Asn Ser Gln Gly Asn Thr Tyr Leu Glu
1               5               10              15

<210>   15
<211>   7
<212>   PRT
<213>   Artificial Sequence

<220>
<221>   DOMAIN
<222>   (1)..(7)
<223>   9E5 LCDR2



<400>   15
Lys Val Thr Asn Arg Phe Ser
1               5

<210>   16
<211>   9
<212>   PRT
<213>   Artificial Sequence

<220>
<221>   DOMAIN
<222>   (1)..(9)
<223>   9E5 LCDR3



<400>   16
Phe Gln Ala Ser Leu Val Pro Trp Thr
1               5

<210>   17
<211>   446
<212>   PRT
<213>   Artificial Sequence
```

```
<220>
<221>   CHAIN
<222>   (1)..(446)
<223>   hu2H6 HC


<400>   17
Gln Val Gln Leu Val Gln Ser Gly Ala Glu Val Lys Lys Pro Gly Ser
1               5                   10                  15
Ser Val Lys Val Ser Cys Lys Ala Ser Gly Tyr Ala Phe Ser Asp Tyr
            20                  25                  30
Leu Ile Glu Trp Val Arg Gln Ala Pro Gly Gln Gly Leu Glu Trp Met
        35                  40                  45
Gly Val Ile Asn Pro Gly Ser Gly Gly Ser Asn Tyr Asn Glu Lys Ile
    50                  55                  60
Lys Asp Arg Val Thr Leu Thr Ala Asp Lys Ser Thr Ser Thr Ala Tyr
65                  70                  75                  80
Met Glu Leu Ser Ser Leu Arg Ser Glu Asp Thr Ala Val Tyr Tyr Cys
                85                  90                  95
Ala Arg Gly Gly Gly Gly Phe Thr Tyr Trp Gly Gln Gly Thr Leu Val
            100                 105                 110
Thr Val Ser Ser Ala Ser Thr Lys Gly Pro Ser Val Phe Pro Leu Ala
        115                 120                 125
Pro Ser Ser Lys Ser Thr Ser Gly Gly Thr Ala Ala Leu Gly Cys Leu
    130                 135                 140
Val Lys Asp Tyr Phe Pro Glu Pro Val Thr Val Ser Trp Asn Ser Gly
145                 150                 155                 160
Ala Leu Thr Ser Gly Val His Thr Phe Pro Ala Val Leu Gln Ser Ser
                165                 170                 175
Gly Leu Tyr Ser Leu Ser Ser Val Val Thr Val Pro Ser Ser Ser Leu
            180                 185                 190
Gly Thr Gln Thr Tyr Ile Cys Asn Val Asn His Lys Pro Ser Asn Thr
        195                 200                 205
Lys Val Asp Lys Lys Val Glu Pro Lys Ser Cys Asp Lys Thr His Thr
    210                 215                 220
Cys Pro Pro Cys Pro Ala Pro Glu Leu Leu Gly Gly Pro Ser Val Phe
225                 230                 235                 240
Leu Phe Pro Pro Lys Pro Lys Asp Thr Leu Met Ile Ser Arg Thr Pro
                245                 250                 255
Glu Val Thr Cys Val Val Val Asp Val Ser His Glu Asp Pro Glu Val
            260                 265                 270
Lys Phe Asn Trp Tyr Val Asp Gly Val Glu Val His Asn Ala Lys Thr
        275                 280                 285
Lys Pro Arg Glu Glu Gln Tyr Asn Ser Thr Tyr Arg Val Val Ser Val
    290                 295                 300
Leu Thr Val Leu His Gln Asp Trp Leu Asn Gly Lys Glu Tyr Lys Cys
305                 310                 315                 320
Lys Val Ser Asn Lys Ala Leu Pro Ala Pro Ile Glu Lys Thr Ile Ser
                325                 330                 335
Lys Ala Lys Gly Gln Pro Arg Glu Pro Gln Val Tyr Thr Leu Pro Pro
            340                 345                 350
Ser Arg Asp Glu Leu Thr Lys Asn Gln Val Ser Leu Thr Cys Leu Val
        355                 360                 365
Lys Gly Phe Tyr Pro Ser Asp Ile Ala Val Glu Trp Glu Ser Asn Gly
    370                 375                 380
Gln Pro Glu Asn Asn Tyr Lys Thr Thr Pro Pro Val Leu Asp Ser Asp
385                 390                 395                 400
Gly Ser Phe Phe Leu Tyr Ser Lys Leu Thr Val Asp Lys Ser Arg Trp
                405                 410                 415
Gln Gln Gly Asn Val Phe Ser Cys Ser Val Met His Glu Ala Leu His
            420                 425                 430
Asn His Tyr Thr Gln Lys Ser Leu Ser Leu Ser Pro Gly Lys
        435                 440                 445
```

<210> 18
<211> 214
<212> PRT
<213> Artificial Sequence

<220>
<221> CHAIN
<222> (1)..(214)
<223> hu2H6 LC


<400> 18
Asp Ile Gln Leu Thr Gln Ser Pro Ser Ser Leu Ser Ala Ser Val Gly
1               5                   10                  15
Asp Arg Val Thr Ile Thr Cys Arg Ala Ser Gln Asp Ile Ser Asn Tyr
                20                  25                  30
Leu Asn Trp Tyr Gln Gln Lys Pro Gly Lys Thr Ile Lys Leu Leu Leu
            35                  40                  45
Asn Phe Ala Ser Arg Leu His Ser Gly Val Pro Ser Arg Phe Ser Gly
        50                  55                  60
Ser Gly Ser Gly Thr Asp Phe Thr Phe Thr Ile Ser Ser Leu Gln Pro
65                  70                  75                  80
Glu Asp Ile Ala Thr Tyr Tyr Cys Gln Gln Gly Ser Thr Leu Pro Trp
                85                  90                  95
Thr Phe Gly Gly Gly Thr Lys Val Glu Ile Lys Arg Thr Val Ala Ala
            100                 105                 110
Pro Ser Val Phe Ile Phe Pro Pro Ser Asp Glu Gln Leu Lys Ser Gly
        115                 120                 125
Thr Ala Ser Val Val Cys Leu Leu Asn Asn Phe Tyr Pro Arg Glu Ala
    130                 135                 140
Lys Val Gln Trp Lys Val Asp Asn Ala Leu Gln Ser Gly Asn Ser Gln
145                 150                 155                 160
Glu Ser Val Thr Glu Gln Asp Ser Lys Asp Ser Thr Tyr Ser Leu Ser
                165                 170                 175
Ser Thr Leu Thr Leu Ser Lys Ala Asp Tyr Glu Lys His Lys Val Tyr
            180                 185                 190
Ala Cys Glu Val Thr His Gln Gly Leu Ser Ser Pro Val Thr Lys Ser
        195                 200                 205
Phe Asn Arg Gly Glu Cys
    210

<210> 19
<211> 442
<212> PRT
<213> Artificial Sequence

<220>
<221> CHAIN
<222> (1)..(442)
<223> hu9E5 HC


<400> 19
Gln Val Gln Leu Val Gln Ser Gly Ala Glu Val Lys Lys Pro Gly Ala
1               5                   10                  15
Ser Val Lys Val Ser Cys Lys Ala Ser Gly Tyr Ile Leu Thr Thr Tyr
                20                  25                  30
Trp Ile Thr Trp Val Arg Gln Ala Pro Gly Gln Gly Leu Glu Trp Met
            35                  40                  45
Gly Asp Ile His Pro Gly Ser Gly Ser Thr Lys Tyr Asn Glu Lys Phe
        50                  55                  60
Lys Ser Arg Val Thr Leu Thr Val Asp Thr Ser Ile Ser Thr Ala Tyr

```
            65                    70                    75                    80
Met Glu Leu Ser Arg Leu Arg Ser Glu Asp Thr Ala Val Tyr Tyr Cys
                85                    90                    95
Ala Arg Arg Asp Tyr Trp Gly Gln Gly Thr Thr Val Thr Val Ser Ser
               100                   105                   110
Ala Ser Thr Lys Gly Pro Ser Val Phe Pro Leu Ala Pro Ser Ser Lys
               115                   120                   125
Ser Thr Ser Gly Gly Thr Ala Ala Leu Gly Cys Leu Val Lys Asp Tyr
               130                   135                   140
Phe Pro Glu Pro Val Thr Val Ser Trp Asn Ser Gly Ala Leu Thr Ser
145                   150                   155                   160
Gly Val His Thr Phe Pro Ala Val Leu Gln Ser Ser Gly Leu Tyr Ser
                    165                   170                   175
Leu Ser Ser Val Val Thr Val Pro Ser Ser Leu Gly Thr Gln Thr
               180                   185                   190
Tyr Ile Cys Asn Val Asn His Lys Pro Ser Asn Thr Lys Val Asp Lys
               195                   200                   205
Lys Val Glu Pro Lys Ser Cys Asp Lys Thr His Thr Cys Pro Pro Cys
210                   215                   220
Pro Ala Pro Glu Leu Leu Gly Gly Pro Ser Val Phe Leu Phe Pro Pro
225                   230                   235                   240
Lys Pro Lys Asp Thr Leu Met Ile Ser Arg Thr Pro Glu Val Thr Cys
                    245                   250                   255
Val Val Val Asp Val Ser His Glu Asp Pro Glu Val Lys Phe Asn Trp
                    260                   265                   270
Tyr Val Asp Gly Val Glu Val His Asn Ala Lys Thr Lys Pro Arg Glu
               275                   280                   285
Glu Gln Tyr Asn Ser Thr Tyr Arg Val Val Ser Val Leu Thr Val Leu
               290                   295                   300
His Gln Asp Trp Leu Asn Gly Lys Glu Tyr Lys Cys Lys Val Ser Asn
305                   310                   315                   320
Lys Ala Leu Pro Ala Pro Ile Glu Lys Thr Ile Ser Lys Ala Lys Gly
                    325                   330                   335
Gln Pro Arg Glu Pro Gln Val Tyr Thr Leu Pro Pro Ser Arg Asp Glu
               340                   345                   350
Leu Thr Lys Asn Gln Val Ser Leu Thr Cys Leu Val Lys Gly Phe Tyr
               355                   360                   365
Pro Ser Asp Ile Ala Val Glu Trp Glu Ser Asn Gly Gln Pro Glu Asn
               370                   375                   380
Asn Tyr Lys Thr Thr Pro Pro Val Leu Asp Ser Asp Gly Ser Phe Phe
385                   390                   395                   400
Leu Tyr Ser Lys Leu Thr Val Asp Lys Ser Arg Trp Gln Gln Gly Asn
                    405                   410                   415
Val Phe Ser Cys Ser Val Met His Glu Ala Leu His Asn His Tyr Thr
                    420                   425                   430
Gln Lys Ser Leu Ser Leu Ser Pro Gly Lys
                    435                   440
```

```
<210>   20
<211>   219
<212>   PRT
<213>   Artificial Sequence

<220>
<221>   CHAIN
<222>   (1)..(219)
<223>   hu9E5 LC


<400>   20
Asp Ile Val Met Thr Gln Ser Pro Leu Ser Leu Pro Val Thr Pro Gly
1                   5                     10                    15
Glu Pro Ala Ser Ile Ser Cys Arg Ser Ser Gln Asn Ile Val Asn Ser
```

37

```
                   20                      25                      30
      Gln Gly Asn Thr Tyr Leu Glu Trp Tyr Leu Gln Lys Pro Gly Gln Ser
              35                      40                      45
      Pro Gln Leu Leu Ile Tyr Lys Val Thr Asn Arg Phe Ser Gly Val Pro
              50                      55                      60
      Asp Arg Phe Ser Gly Ser Gly Ser Gly Thr Asp Phe Thr Leu Lys Ile
      65                      70                      75                      80
      Ser Arg Val Glu Ala Glu Asp Val Gly Val Tyr Tyr Cys Phe Gln Ala
                      85                      90                      95
      Ser Leu Val Pro Trp Thr Phe Gly Gly Gly Thr Lys Val Glu Ile Lys
                      100                     105                     110
      Arg Thr Val Ala Ala Pro Ser Val Phe Ile Phe Pro Pro Ser Asp Glu
              115                     120                     125
      Gln Leu Lys Ser Gly Thr Ala Ser Val Val Cys Leu Leu Asn Asn Phe
              130                     135                     140
      Tyr Pro Arg Glu Ala Lys Val Gln Trp Lys Val Asp Asn Ala Leu Gln
      145                     150                     155                     160
      Ser Gly Asn Ser Gln Glu Ser Val Thr Glu Gln Asp Ser Lys Asp Ser
                      165                     170                     175
      Thr Tyr Ser Leu Ser Ser Thr Leu Thr Leu Ser Lys Ala Asp Tyr Glu
                      180                     185                     190
      Lys His Lys Val Tyr Ala Cys Glu Val Thr His Gln Gly Leu Ser Ser
              195                     200                     205
      Pro Val Thr Lys Ser Phe Asn Arg Gly Glu Cys
              210                     215
```

<210> 21
<211> 98
<212> PRT
<213> Artificial Sequence

<220>
<221> CHAIN
<222> (1)..(98)
<223> Preferable human germline heavy chain template IGHV1-69 for 2H6

```
<400> 21
Gln Val Gln Leu Val Gln Ser Gly Ala Glu Val Lys Lys Pro Gly Ser
1                   5                       10                      15
Ser Val Lys Val Ser Cys Lys Ala Ser Gly Gly Thr Phe Ser Ser Tyr
                20                      25                      30
Ala Ile Ser Trp Val Arg Gln Ala Pro Gly Gln Gly Leu Glu Trp Met
                35                      40                      45
Gly Gly Ile Ile Pro Ile Phe Gly Thr Ala Asn Tyr Ala Gln Lys Phe
              50                      55                      60
Gln Gly Arg Val Thr Ile Thr Ala Asp Lys Ser Thr Ser Thr Ala Tyr
65                      70                      75                      80
Met Glu Leu Ser Ser Leu Arg Ser Glu Asp Thr Ala Val Tyr Tyr Cys
                      85                      90                      95
Ala Arg
```

<210> 22
<211> 95
<212> PRT
<213> Artificial Sequence

<220>
<221> CHAIN
<222> (1)..(95)
<223> Preferable human germline light chain template IGkV1-33 for 2H6

```
<400>  22
Asp Ile Gln Met Thr Gln Ser Pro Ser Ser Leu Ser Ala Ser Val Gly
1               5                   10                  15
Asp Arg Val Thr Ile Thr Cys Gln Ala Ser Gln Asp Ile Ser Asn Tyr
            20                  25                  30
Leu Asn Trp Tyr Gln Gln Lys Pro Gly Lys Ala Pro Lys Leu Leu Ile
        35                  40                  45
Tyr Asp Ala Ser Asn Leu Glu Thr Gly Val Pro Ser Arg Phe Ser Gly
    50                  55                  60
Ser Gly Ser Gly Thr Asp Phe Thr Phe Thr Ile Ser Ser Leu Gln Pro
65                  70                  75                  80
Glu Asp Ile Ala Thr Tyr Tyr Cys Gln Gln Tyr Asp Asn Leu Pro
                85                  90                  95
```

```
<210>  23
<211>  98
<212>  PRT
<213>  Artificial Sequence

<220>
<221>  CHAIN
<222>  (1)..(98)
<223>  Preferable human germline heavy chain template IGHV1-2 for 9E5
```

```
<400>  23
Gln Val Gln Leu Val Gln Ser Gly Ala Glu Val Lys Lys Pro Gly Ala
1               5                   10                  15
Ser Val Lys Val Ser Cys Lys Ala Ser Gly Tyr Thr Phe Thr Gly Tyr
            20                  25                  30
Tyr Met His Trp Val Arg Gln Ala Pro Gly Gln Gly Leu Glu Trp Met
        35                  40                  45
Gly Trp Ile Asn Pro Asn Ser Gly Gly Thr Asn Tyr Ala Gln Lys Phe
    50                  55                  60
Gln Gly Arg Val Thr Met Thr Arg Asp Thr Ser Ile Ser Thr Ala Tyr
65                  70                  75                  80
Met Glu Leu Ser Arg Leu Arg Ser Asp Asp Thr Ala Val Tyr Tyr Cys
                85                  90                  95
Ala Arg
```

```
<210>  24
<211>  100
<212>  PRT
<213>  Artificial Sequence

<220>
<221>  CHAIN
<222>  (1)..(100)
<223>  Preferable human germline light chain template IGkV2-28 for 9E5
```

```
<400>  24
Asp Ile Val Met Thr Gln Ser Pro Leu Ser Leu Pro Val Thr Pro Gly
1               5                   10                  15
Glu Pro Ala Ser Ile Ser Cys Arg Ser Ser Gln Ser Leu Leu His Ser
            20                  25                  30
Asn Gly Tyr Asn Tyr Leu Asp Trp Tyr Leu Gln Lys Pro Gly Gln Ser
        35                  40                  45
Pro Gln Leu Leu Ile Tyr Leu Gly Ser Asn Arg Ala Ser Gly Val Pro
    50                  55                  60
Asp Arg Phe Ser Gly Ser Gly Ser Gly Thr Asp Phe Thr Leu Lys Ile
```

```
                65                      70                      75                      80
                Ser Arg Val Glu Ala Glu Asp Val Gly Val Tyr Tyr Cys Met Gln Ala
                            85                      90                      95
                Leu Gln Thr Pro
                            100


                <210>   25
                <211>   116
                <212>   PRT
                <213>   Artificial Sequence

                <220>
                <221>   CHAIN
                <222>   (1)..(116)
                <223>   hu2H6-H1a



                <400>   25
                Gln Val Gln Leu Val Gln Ser Gly Ala Glu Val Lys Lys Pro Gly Ser
                1                   5                   10                      15
                Ser Val Lys Val Ser Cys Lys Ala Ser Gly Gly Thr Phe Ser Asp Tyr
                            20                      25                      30
                Leu Ile Glu Trp Val Arg Gln Ala Pro Gly Gln Gly Leu Glu Trp Met
                            35                      40                      45
                Gly Val Ile Asn Pro Gly Ser Gly Gly Ser Asn Tyr Asn Glu Lys Ile
                            50                      55                      60
                Lys Asp Arg Val Thr Ile Thr Ala Asp Lys Ser Thr Ser Thr Ala Tyr
                65                      70                      75                      80
                Met Glu Leu Ser Ser Leu Arg Ser Glu Asp Thr Ala Val Tyr Tyr Cys
                            85                      90                      95
                Ala Arg Gly Gly Gly Gly Phe Thr Tyr Trp Gly Gln Gly Thr Leu Val
                            100                     105                     110
                Thr Val Ser Ser
                            115


                <210>   26
                <211>   116
                <212>   PRT
                <213>   Artificial Sequence

                <220>
                <221>   CHAIN
                <222>   (1)..(116)
                <223>   hu2H6-H1b



                <400>   26
                Gln Val Gln Leu Val Gln Ser Gly Ala Glu Val Lys Lys Pro Gly Ser
                1                   5                   10                      15
                Ser Val Lys Val Ser Cys Lys Ala Ser Gly Tyr Ala Phe Ser Asp Tyr
                            20                      25                      30
                Leu Ile Glu Trp Val Arg Gln Ala Pro Gly Gln Gly Leu Glu Trp Met
                            35                      40                      45
                Gly Val Ile Asn Pro Gly Ser Gly Gly Ser Asn Tyr Asn Glu Lys Ile
                            50                      55                      60
                Lys Asp Arg Val Thr Leu Thr Ala Asp Lys Ser Thr Ser Thr Ala Tyr
                65                      70                      75                      80
                Met Glu Leu Ser Ser Leu Arg Ser Glu Asp Thr Ala Val Tyr Tyr Cys
                            85                      90                      95
                Ala Arg Gly Gly Gly Gly Phe Thr Tyr Trp Gly Gln Gly Thr Leu Val
                            100                     105                     110
                Thr Val Ser Ser
                            115
```

<210> 27
<211> 126
<212> PRT
<213> Artificial Sequence

<220>
<221> CHAIN
<222> (1)..(126)
<223> hu2H6-H1c


<400> 27
Gln Val Gln Leu Val Gln Ser Gly Ala Glu Val Lys Lys Pro Gly Ser
1               5                   10                  15
Ser Val Lys Val Ser Cys Lys Ala Ser Gly Tyr Ala Phe Ser Asp Tyr
            20                  25                  30
Leu Ile Glu Trp Val Arg Gln Ala Pro Gly Gln Gly Leu Glu Trp Ile
        35                  40                  45
Gly Val Ile Asn Pro Gly Ser Gly Gly Ser Asn Tyr Asn Glu Lys Ile
    50                  55                  60
Lys Asp Arg Ala Thr Leu Thr Ala Asp Lys Ser Thr Ser Thr Ala Tyr
65                  70                  75                  80
Met Glu Leu Ser Ser Leu Arg Ser Glu Asp Thr Ala Val Tyr Tyr Cys
                85                  90                  95
Ala Arg Gly Gly Gly Gly Phe Thr Tyr Trp Gly Gln Gly Thr Leu Val
            100                 105                 110
Thr Val Ser Ser Phe Gly Gln Gly Thr Lys Leu Glu Ile Lys
            115                 120                 125

<210> 28
<211> 112
<212> PRT
<213> Artificial Sequence

<220>
<221> CHAIN
<222> (1)..(112)
<223> hu9E5-H1a


<400> 28
Gln Val Gln Leu Val Gln Ser Gly Ala Glu Val Lys Lys Pro Gly Ala
1               5                   10                  15
Ser Val Lys Val Ser Cys Lys Ala Ser Gly Tyr Thr Phe Thr Thr Tyr
            20                  25                  30
Trp Ile Thr Trp Val Arg Gln Ala Pro Gly Gln Gly Leu Glu Trp Met
        35                  40                  45
Gly Asp Ile His Pro Gly Ser Gly Ser Thr Lys Tyr Asn Glu Lys Phe
    50                  55                  60
Lys Ser Arg Val Thr Met Thr Val Asp Thr Ser Ile Ser Thr Ala Tyr
65                  70                  75                  80
Met Glu Leu Ser Arg Leu Arg Ser Glu Asp Thr Ala Val Tyr Tyr Cys
                85                  90                  95
Ala Arg Arg Asp Tyr Trp Gly Gln Gly Thr Thr Val Thr Val Ser Ser
            100                 105                 110

<210> 29
<211> 112
<212> PRT
<213> Artificial Sequence

<220>

```
<221>  CHAIN
<222>  (1)..(112)
<223>  hu9E5-H1b


<400>  29
Gln Val Gln Leu Val Gln Ser Gly Ala Glu Val Lys Lys Pro Gly Ala
1               5                   10                  15
Ser Val Lys Val Ser Cys Lys Ala Ser Gly Tyr Thr Phe Thr Thr Tyr
                20                  25                  30
Trp Ile Thr Trp Val Arg Gln Ala Pro Gly Gln Gly Leu Glu Trp Met
            35                  40                  45
Gly Asp Ile His Pro Gly Ser Gly Ser Thr Lys Tyr Asn Glu Lys Phe
        50                  55                  60
Lys Ser Arg Val Thr Leu Thr Val Asp Thr Ser Ile Ser Thr Ala Tyr
65                  70                  75                  80
Met Glu Leu Ser Arg Leu Arg Ser Glu Asp Thr Ala Val Tyr Tyr Cys
                85                  90                  95
Ala Arg Arg Asp Tyr Trp Gly Gln Gly Thr Thr Val Thr Val Ser Ser
            100                 105                 110

<210>  30
<211>  112
<212>  PRT
<213>  Artificial Sequence

<220>
<221>  CHAIN
<222>  (1)..(112)
<223>  hu9E5-H1c


<400>  30
Gln Val Gln Leu Val Gln Ser Gly Ala Glu Val Lys Lys Pro Gly Ala
1               5                   10                  15
Ser Val Lys Val Ser Cys Lys Ala Ser Gly Tyr Ile Leu Thr Thr Tyr
                20                  25                  30
Trp Ile Thr Trp Val Arg Gln Ala Pro Gly Gln Gly Leu Glu Trp Met
            35                  40                  45
Gly Asp Ile His Pro Gly Ser Gly Ser Thr Lys Tyr Asn Glu Lys Phe
        50                  55                  60
Lys Ser Arg Val Thr Leu Thr Val Asp Thr Ser Ile Ser Thr Ala Tyr
65                  70                  75                  80
Met Glu Leu Ser Arg Leu Arg Ser Glu Asp Thr Ala Val Tyr Tyr Cys
                85                  90                  95
Ala Arg Arg Asp Tyr Trp Gly Gln Gly Thr Thr Val Thr Val Ser Ser
            100                 105                 110

<210>  31
<211>  107
<212>  PRT
<213>  Artificial Sequence

<220>
<221>  CHAIN
<222>  (1)..(107)
<223>  hu2H6-L1a


<400>  31
Asp Ile Gln Met Thr Gln Ser Pro Ser Ser Leu Ser Ala Ser Val Gly
1               5                   10                  15
Asp Arg Val Thr Ile Thr Cys Arg Ala Ser Gln Asp Ile Ser Asn Tyr
```

```
                      20                      25                      30
         Leu Asn Trp Tyr Gln Gln Lys Pro Gly Lys Ala Pro Lys Leu Leu Leu
                  35                      40                      45
         Asn Phe Ala Ser Arg Leu His Ser Gly Val Pro Ser Arg Phe Ser Gly
                  50                      55                      60
         Ser Gly Ser Gly Thr Asp Phe Thr Phe Thr Ile Ser Ser Leu Gln Pro
         65                      70                      75                      80
         Glu Asp Ile Ala Thr Tyr Tyr Cys Gln Gln Gly Ser Thr Leu Pro Trp
                          85                      90                      95
         Thr Phe Gly Gly Gly Thr Lys Val Glu Ile Lys
                      100                     105


         <210>  32
         <211>  107
         <212>  PRT
         <213>  Artificial Sequence

         <220>
         <221>  CHAIN
         <222>  (1)..(107)
         <223>  hu2H6-L1b


         <400>  32
         Asp Ile Gln Leu Thr Gln Ser Pro Ser Ser Leu Ser Ala Ser Val Gly
         1                   5                       10                      15
         Asp Arg Val Thr Ile Thr Cys Arg Ala Ser Gln Asp Ile Ser Asn Tyr
                      20                      25                      30
         Leu Asn Trp Tyr Gln Gln Lys Pro Gly Lys Ala Pro Lys Leu Leu Leu
                  35                      40                      45
         Asn Phe Ala Ser Arg Leu His Ser Gly Val Pro Ser Arg Phe Ser Gly
                  50                      55                      60
         Ser Gly Ser Gly Thr Asp Phe Thr Phe Thr Ile Ser Ser Leu Gln Pro
         65                      70                      75                      80
         Glu Asp Ile Ala Thr Tyr Tyr Cys Gln Gln Gly Ser Thr Leu Pro Trp
                          85                      90                      95
         Thr Phe Gly Gly Gly Thr Lys Val Glu Ile Lys
                      100                     105


         <210>  33
         <211>  107
         <212>  PRT
         <213>  Artificial Sequence

         <220>
         <221>  CHAIN
         <222>  (1)..(107)
         <223>  hu2H6-L1c


         <400>  33
         Asp Ile Gln Leu Thr Gln Ser Pro Ser Ser Leu Ser Ala Ser Val Gly
         1                   5                       10                      15
         Asp Arg Val Thr Ile Thr Cys Arg Ala Ser Gln Asp Ile Ser Asn Tyr
                      20                      25                      30
         Leu Asn Trp Tyr Gln Gln Lys Pro Gly Lys Thr Ile Lys Leu Leu Leu
                  35                      40                      45
         Asn Phe Ala Ser Arg Leu His Ser Gly Val Pro Ser Arg Phe Ser Gly
                  50                      55                      60
         Ser Gly Ser Gly Thr Asp Phe Thr Phe Thr Ile Ser Ser Leu Gln Pro
         65                      70                      75                      80
         Glu Asp Ile Ala Thr Tyr Tyr Cys Gln Gln Gly Ser Thr Leu Pro Trp
                          85                      90                      95
```

```
Thr Phe Gly Gly Gly Thr Lys Val Glu Ile Lys
            100                 105


<210>  34
<211>  112
<212>  PRT
<213>  Artificial Sequence


<220>
<221>  CHAIN
<222>  (1)..(112)
<223>  hu9E5-L1a


<400>  34
Asp Ile Val Met Thr Gln Ser Pro Leu Ser Leu Pro Val Thr Pro Gly
1               5                   10                  15
Glu Pro Ala Ser Ile Ser Cys Arg Ser Ser Gln Asn Ile Val Asn Ser
            20                  25                  30
Gln Gly Asn Thr Tyr Leu Glu Trp Tyr Leu Gln Lys Pro Gly Gln Ser
            35                  40                  45
Pro Gln Leu Leu Ile Tyr Lys Val Thr Asn Arg Phe Ser Gly Val Pro
        50                  55                  60
Asp Arg Phe Ser Gly Ser Gly Ser Gly Thr Asp Phe Thr Leu Lys Ile
65                  70                  75                  80
Ser Arg Val Glu Ala Glu Asp Val Gly Val Tyr Tyr Cys Phe Gln Ala
            85                  90                  95
Ser Leu Val Pro Trp Thr Phe Gly Gly Gly Thr Lys Val Glu Ile Lys
            100                 105                 110


<210>  35
<211>  112
<212>  PRT
<213>  Artificial Sequence


<220>
<221>  CHAIN
<222>  (1)..(112)
<223>  hu9E5-L1b


<400>  35
Asp Val Val Met Thr Gln Ser Pro Leu Ser Leu Pro Val Thr Pro Gly
1               5                   10                  15
Glu Pro Ala Ser Ile Ser Cys Arg Ser Ser Gln Asn Ile Val Asn Ser
            20                  25                  30
Gln Gly Asn Thr Tyr Leu Glu Trp Tyr Leu Gln Lys Pro Gly Gln Ser
            35                  40                  45
Pro Gln Leu Leu Ile Tyr Lys Val Thr Asn Arg Phe Ser Gly Val Pro
        50                  55                  60
Asp Arg Phe Ser Gly Ser Gly Ser Gly Thr Asp Phe Thr Leu Lys Ile
65                  70                  75                  80
Ser Arg Val Glu Ala Glu Asp Val Gly Val Tyr Tyr Cys Phe Gln Ala
            85                  90                  95
Ser Leu Val Pro Trp Thr Phe Gly Gly Gly Thr Lys Val Glu Ile Lys
            100                 105                 110


<210>  36
<211>  112
<212>  PRT
<213>  Artificial Sequence


<220>
```

```
<221>  CHAIN
<222>  (1)..(112)
<223>  hu9E5-L1c


<400>  36
Asp Val Leu Met Thr Gln Ser Pro Leu Ser Leu Pro Val Thr Pro Gly
1               5                   10                  15
Glu Pro Ala Ser Ile Ser Cys Arg Ser Ser Gln Asn Ile Val Asn Ser
            20                  25                  30
Gln Gly Asn Thr Tyr Leu Glu Trp Tyr Leu Gln Lys Pro Gly Gln Ser
        35                  40                  45
Pro Gln Leu Leu Ile Tyr Lys Val Thr Asn Arg Phe Ser Gly Val Pro
    50                  55                  60
Asp Arg Phe Ser Gly Ser Gly Ser Gly Thr Asp Phe Thr Leu Lys Ile
65                  70                  75                  80
Ser Arg Val Glu Ala Glu Asp Val Gly Val Tyr Tyr Cys Phe Gln Ala
                85                  90                  95
Ser Leu Val Pro Trp Thr Phe Gly Gly Gly Thr Lys Val Glu Ile Lys
            100                 105                 110


<210>  37
<211>  116
<212>  PRT
<213>  Artificial Sequence


<220>
<221>  CHAIN
<222>  (1)..(116)
<223>  Sequence of heavy chain variable region 1D9 HCVR of 1D9


<400>  37
Gln Val Arg Leu Gln Gln Ser Gly Ala Glu Leu Val Arg Pro Gly Thr
1               5                   10                  15
Ser Met Arg Val Ser Cys Lys Ala Ser Gly Tyr Ala Phe Thr Asn Tyr
            20                  25                  30
Leu Ile Asn Trp Val Lys Gln Arg Pro Gly Gln Gly Leu Glu Trp Ile
        35                  40                  45
Gly Ile Leu Asn Pro Gly Ser Gly Gly Thr Asn Tyr Asn Glu Asn Phe
    50                  55                  60
Lys Asp Lys Ala Thr Leu Thr Ala Asp Lys Ser Asn Thr Ala Tyr
65                  70                  75                  80
Met Gln Leu Ser Ser Leu Thr Ser Glu Asp Ser Ala Val Tyr Phe Cys
                85                  90                  95
Ile Arg Gly Ser Pro Gly Phe Ala Tyr Trp Gly Gln Gly Thr Leu Val
            100                 105                 110
Thr Val Ser Ala
        115


<210>  38
<211>  107
<212>  PRT
<213>  Artificial Sequence


<220>
<221>  CHAIN
<222>  (1)..(107)
<223>  Sequence of light chain variable region 1D9 LCVR of 1D9


<400>  38
Asp Ile Gln Met Thr Gln Thr Thr Ser Ser Leu Ser Ala Ser Leu Gly
```

```
        1                    5                    10                    15
        Asp Arg Val Thr Ile Ser Cys Arg Ala Ser Gln Asp Ile Asn Ile Tyr
                        20                    25                    30
        Leu Asn Trp Tyr Gln Gln Lys Pro Asp Gly Thr Val Lys Leu Leu Ile
                        35                    40                    45
        Tyr Ser Thr Ser Gly Leu His Ser Gly Val Pro Ser Arg Phe Asn Gly
                        50                    55                    60
        Ser Gly Ser Gly Thr Asp Tyr Ser Leu Thr Ile Ser Asn Leu Glu Gln
        65                    70                    75                    80
        Glu Asp Ile Ala Thr Tyr Phe Cys Gln Gln Gly Tyr Thr Leu Pro Tyr
                        85                    90                    95
        Thr Phe Gly Gly Gly Thr Lys Leu Glu Ile Lys
                        100                   105


        <210>  39
        <211>  10
        <212>  PRT
        <213>  Artificial Sequence

        <220>
        <221>  DOMAIN
        <222>  (1)..(10)
        <223>  1D9 HCDR1


        <400>  39
        Gly Tyr Ala Phe Thr Asn Tyr Leu Ile Asn
        1                    5                    10


        <210>  40
        <211>  17
        <212>  PRT
        <213>  Artificial Sequence

        <220>
        <221>  DOMAIN
        <222>  (1)..(17)
        <223>  1D9 HCDR2


        <400>  40
        Ile Leu Asn Pro Gly Ser Gly Gly Thr Asn Tyr Asn Glu Asn Phe Lys
        1                    5                    10                    15
        Asp


        <210>  41
        <211>  7
        <212>  PRT
        <213>  Artificial Sequence

        <220>
        <221>  DOMAIN
        <222>  (1)..(7)
        <223>  1D9 HCDR3


        <400>  41
        Gly Ser Pro Gly Phe Ala Tyr
        1                    5

        <210>  42
        <211>  11
```

```
<212>  PRT
<213>  Artificial Sequence

<220>
<221>  DOMAIN
<222>  (1)..(11)
<223>  1D9 LCDR1


<400>  42
Arg Ala Ser Gln Asp Ile Asn Ile Tyr Leu Asn
1                5                    10

<210>  43
<211>  7
<212>  PRT
<213>  Artificial Sequence

<220>
<221>  DOMAIN
<222>  (1)..(7)
<223>  1D9 LCDR2


<400>  43
Ser Thr Ser Gly Leu His Ser
1                5

<210>  44
<211>  9
<212>  PRT
<213>  Artificial Sequence

<220>
<221>  DOMAIN
<222>  (1)..(9)
<223>  1D9 LCDR3


<400>  44
Gln Gln Gly Tyr Thr Leu Pro Tyr Thr
1                5

<210>  45
<211>  116
<212>  PRT
<213>  Artificial Sequence

<220>
<221>  CHAIN
<222>  (1)..(116)
<223>  Sequence of heavy chain variable region 14C10 HCVR of 14C10


<400>  45
Gln Val Gln Val Gln Gln Ser Gly Ala Glu Leu Val Arg Pro Gly Thr
1                5                    10                   15
Ser Val Lys Val Ser Cys Lys Ala Ser Gly Tyr Ala Phe Thr Asn Tyr
            20                   25                   30
Leu Ile Glu Trp Val Lys Gln Arg Pro Gly Gln Gly Leu Glu Trp Ile
         35                   40                   45
Gly Val Ile Asn Pro Glu Phe Gly Gly Thr Asn Tyr Asn Glu Lys Phe
      50                   55                   60
```

```
        Lys Gly Lys Ala Thr Leu Thr Ala Asp Lys Ser Ser Ser Thr Ala Tyr
        65              70              75                  80
        Met Gln Leu Ser Ser Leu Thr Ser Glu Asp Ser Ala Val Tyr Phe Cys
                        85              90                  95
        Ala Arg Gly Gly Gly Gly Phe Thr Tyr Trp Gly Gln Gly Thr Leu Val
                    100             105             110
        Thr Val Ser Ala
                    115


        <210>  46
        <211>  107
        <212>  PRT
        <213>  Artificial Sequence

        <220>
        <221>  CHAIN
        <222>  (1)..(107)
        <223>  Sequence of light chain variable region14C10 LCVR of 14C10


        <400>  46
        His Ile Gln Met Thr Gln Thr Thr Ser Ser Leu Ser Ala Ser Leu Gly
        1               5               10                  15
        Asp Arg Val Thr Ile Ser Cys Arg Ala Ser Gln Asp Ile Ser Ser His
                    20              25                  30
        Leu Asn Trp Tyr Gln Gln Lys Pro Asp Gly Thr Val Lys Leu Leu Ile
                    35              40                  45
        Ser Tyr Thr Ser Arg Leu His Ser Gly Val Pro Ser Arg Phe Ser Gly
                    50              55                  60
        Ser Gly Ser Gly Ala Asp Tyr Ser Leu Thr Ile Ser Asn Leu Glu Gln
        65              70              75                  80
        Glu Asp Ile Ala Thr Tyr Phe Cys Gln Gln Gly Asn Thr Leu Pro Trp
                        85              90                  95
        Thr Phe Gly Gly Gly Thr Lys Leu Glu Ile Lys
                    100             105


        <210>  47
        <211>  10
        <212>  PRT
        <213>  Artificial Sequence

        <220>
        <221>  DOMAIN
        <222>  (1)..(10)
        <223>  14C10 HCDR1


        <400>  47
        Gly Tyr Ala Phe Thr Asn Tyr Leu Ile Glu
        1               5                   10

        <210>  48
        <211>  17
        <212>  PRT
        <213>  Artificial Sequence

        <220>
        <221>  DOMAIN
        <222>  (1)..(17)
        <223>  14C10 HCDR2


        <400>  48
```

```
Val Ile Asn Pro Glu Phe Gly Gly Thr Asn Tyr Asn Glu Lys Phe Lys
1               5                   10                  15
Gly
```

```
<210>  49
<211>  7
<212>  PRT
<213>  Artificial Sequence

<220>
<221>  DOMAIN
<222>  (1)..(7)
<223>  14C10 HCDR3
```

```
<400>  49
Gly Gly Gly Gly Phe Thr Tyr
1               5
```

```
<210>  50
<211>  11
<212>  PRT
<213>  Artificial Sequence

<220>
<221>  DOMAIN
<222>  (1)..(11)
<223>  14C10 LCDR1
```

```
<400>  50
Arg Ala Ser Gln Asp Ile Ser Ser His Leu Asn
1               5                   10
```

```
<210>  51
<211>  7
<212>  PRT
<213>  Artificial Sequence

<220>
<221>  DOMAIN
<222>  (1)..(7)
<223>  14C10 LCDR2
```

```
<400>  51
Tyr Thr Ser Arg Leu His Ser
1               5
```

```
<210>  52
<211>  9
<212>  PRT
<213>  Artificial Sequence

<220>
<221>  DOMAIN
<222>  (1)..(9)
<223>  14C10 LCDR3
```

```
<400>  52
Gln Gln Gly Asn Thr Leu Pro Trp Thr
```

1                    5

<210> 53
<211> 119
<212> PRT
<213> Artificial Sequence

<220>
<221> CHAIN
<222> (1)..(119)
<223> Sequence of heavy chain variable region 38B4 HCVR of 38B4

<400> 53
Gln Val Arg Leu Lys Gln Ser Gly Ala Glu Leu Val Arg Pro Gly Ala
1                5                    10                   15
Ser Val Lys Val Ser Cys Lys Ala Ser Gly Tyr Thr Phe Thr Asp Tyr
            20                   25                   30
Tyr Ile Asn Trp Val Lys Gln Arg Pro Gly Gln Gly Leu Glu Trp Ile
        35                   40                   45
Ala Gly Ile Tyr Pro Gly Thr Gly Asn Thr Tyr Tyr Asn Glu Lys Phe
        50                   55                   60
Lys Gly Lys Ala Thr Leu Thr Ala Glu Arg Ser Ser Ser Thr Ala Tyr
65                   70                   75                   80
Met Gln Leu Thr Ser Leu Thr Ser Glu Asp Ser Ala Val Tyr Phe Cys
                85                   90                   95
Thr Arg Arg Gly Leu Pro Ser Leu Cys Phe Asp Tyr Trp Gly Gln Gly
            100                  105                  110
Thr Thr Leu Thr Val Ser Ser
            115

<210> 54
<211> 107
<212> PRT
<213> Artificial Sequence

<220>
<221> CHAIN
<222> (1)..(107)
<223> Sequence of light chain variable region 38B4 LCVR of 38B4

<400> 54
Asp Phe Gln Met Thr Gln Thr Thr Ser Ser Leu Ser Ala Ser Leu Gly
1                5                    10                   15
Asp Arg Val Thr Ile Ser Cys Ser Ala Ser Gln Gly Ile Ser Asn Tyr
            20                   25                   30
Leu Asn Trp Tyr Gln Gln Lys Pro Asp Gly Thr Val Lys Leu Leu Ile
        35                   40                   45
Tyr Tyr Thr Ser Ser Leu His Ser Gly Val Pro Ser Arg Phe Ser Gly
        50                   55                   60
Ser Gly Ser Gly Thr Asp Tyr Ser Leu Thr Ile Ser Asn Leu Glu Pro
65                   70                   75                   80
Glu Asp Ile Ala Thr Tyr Tyr Cys Gln Gln Tyr Ser Lys Leu Pro Pro
                85                   90                   95
Thr Phe Gly Gly Gly Thr Lys Leu Glu Ile Lys
            100                  105

<210> 55
<211> 10
<212> PRT
<213> Artificial Sequence

```
<220>
<221>  DOMAIN
<222>  (1)..(10)
<223>  38B4 HCDR1


<400>  55
Gly Tyr Thr Phe Thr Asp Tyr Tyr Ile Asn
1               5                   10

<210>  56
<211>  17
<212>  PRT
<213>  Artificial Sequence


<220>
<221>  DOMAIN
<222>  (1)..(17)
<223>  38B4 HCDR2


<400>  56
Gly Ile Tyr Pro Gly Thr Gly Asn Thr Tyr Tyr Asn Glu Lys Phe Lys
1               5                   10                  15
Gly


<210>  57
<211>  10
<212>  PRT
<213>  Artificial Sequence


<220>
<221>  DOMAIN
<222>  (1)..(10)
<223>  38B4 HCDR3


<400>  57
Arg Gly Leu Pro Ser Leu Cys Phe Asp Tyr
1               5                   10

<210>  58
<211>  11
<212>  PRT
<213>  Artificial Sequence

<220>
<221>  DOMAIN
<222>  (1)..(11)
<223>  38B4 LCDR1


<400>  58
Ser Ala Ser Gln Gly Ile Ser Asn Tyr Leu Asn
1               5                   10

<210>  59
<211>  7
<212>  PRT
<213>  Artificial Sequence

<220>
```

51

<221> DOMAIN
<222> (1)..(7)
<223> 38B4 LCDR2


<400> 59
Tyr Thr Ser Ser Leu His Ser
1               5


<210> 60
<211> 9
<212> PRT
<213> Artificial Sequence


<220>
<221> DOMAIN
<222> (1)..(9)
<223> 38B4 LCDR3


<400> 60
Gln Gln Tyr Ser Lys Leu Pro Pro Thr
1               5


<210> 61
<211> 446
<212> PRT
<213> Artificial Sequence


<220>
<221> CHAIN
<222> (1)..(446)
<223> hu2H6-M HC


<400> 61
Gln Val Gln Leu Val Gln Ser Gly Ala Glu Val Lys Lys Pro Gly Ser
1               5                   10                  15
Ser Val Lys Val Ser Cys Lys Ala Ser Gly Tyr Ala Phe Ser Asp Tyr
                20                  25                  30
Leu Ile Glu Trp Val Arg Gln Ala Pro Gly Gln Gly Leu Glu Trp Met
            35                  40                  45
Gly Val Ile Asn Pro Gly Ser Gly Gly Ser Asn Tyr Asn Glu Lys Ile
        50                  55                  60
Lys Asp Arg Val Thr Leu Thr Ala Asp Lys Ser Thr Ser Thr Ala Tyr
65                  70                  75                  80
Met Glu Leu Ser Ser Leu Arg Ser Glu Asp Thr Ala Val Tyr Tyr Cys
                85                  90                  95
Ala Arg Gly Gly Gly Gly Phe Thr Tyr Trp Gly Gln Gly Thr Leu Val
            100                 105                 110
Thr Val Ser Ser Ala Ser Thr Lys Gly Pro Ser Val Phe Pro Leu Ala
            115                 120                 125
Pro Ser Ser Lys Ser Thr Ser Gly Gly Thr Ala Ala Leu Gly Cys Leu
            130                 135                 140
Val Lys Asp Tyr Phe Pro Glu Pro Val Thr Val Ser Trp Asn Ser Gly
145                 150                 155                 160
Ala Leu Thr Ser Gly Val His Thr Phe Pro Ala Val Leu Gln Ser Ser
                165                 170                 175
Gly Leu Tyr Ser Leu Ser Ser Val Val Thr Val Pro Ser Ser Ser Leu
            180                 185                 190
Gly Thr Gln Thr Tyr Ile Cys Asn Val Asn His Lys Pro Ser Asn Thr
            195                 200                 205
Lys Val Asp Lys Lys Val Glu Pro Lys Ser Cys Asp Lys Thr His Thr

```
            210                      215                      220
Cys Pro Pro Cys Pro Ala Pro Glu Leu Leu Gly Gly Pro Ser Val Phe
225                      230                      235                      240
Leu Phe Pro Pro Lys Pro Lys Asp Thr Leu Met Ile Ser Arg Thr Pro
                245                      250                      255
Glu Val Thr Cys Val Val Val Asp Val Glu His Glu Asp Pro Glu Val
            260                      265                      270
Lys Phe Asn Trp Tyr Val Asp Gly Val Glu Val His Asn Ala Lys Thr
            275                      280                      285
Lys Pro Arg Glu Glu Gln Tyr Asn Ser Thr Tyr Arg Val Val Ser Val
        290                      295                      300
Leu Thr Val Leu His Gln Asp Trp Leu Asn Gly Lys Glu Tyr Lys Cys
305                      310                      315                      320
Lys Val Ser Asn Lys Ala Leu Pro Ala Pro Ile Glu Lys Thr Ile Ser
                325                      330                      335
Lys Ala Lys Gly Gln Pro Arg Glu Pro Gln Val Tyr Thr Leu Pro Pro
            340                      345                      350
Ser Arg Asp Glu Leu Thr Lys Asn Gln Val Ser Leu Thr Cys Leu Val
            355                      360                      365
Lys Gly Phe Tyr Pro Ser Asp Ile Ala Val Glu Trp Glu Ser Asn Gly
            370                      375                      380
Gln Pro Glu Asn Asn Tyr Lys Thr Thr Pro Pro Val Leu Asp Ser Asp
385                      390                      395                      400
Gly Ser Phe Phe Leu Tyr Ser Lys Leu Thr Val Asp Lys Ser Arg Trp
                405                      410                      415
Gln Gln Gly Asn Val Phe Ser Cys Ser Val Met His Glu Ala Leu His
            420                      425                      430
Asn His Tyr Thr Gln Lys Ser Leu Ser Leu Ser Pro Gly Lys
            435                      440                      445


<210>   62
<211>   446
<212>   PRT
<213>   Artificial Sequence

<220>
<221>   CHAIN
<222>   (1)..(446)
<223>   hu2H6-SELFNS HC



<400>   62
Gln Val Gln Leu Val Gln Ser Gly Ala Glu Val Lys Lys Pro Gly Ser
1               5                       10                      15
Ser Val Lys Val Ser Cys Lys Ala Ser Gly Tyr Ala Phe Ser Asp Tyr
            20                      25                      30
Leu Ile Glu Trp Val Arg Gln Ala Pro Gly Gln Gly Leu Glu Trp Met
        35                      40                      45
Gly Val Ile Asn Pro Gly Ser Gly Gly Ser Asn Tyr Asn Glu Lys Ile
        50                      55                      60
Lys Asp Arg Val Thr Leu Thr Ala Asp Lys Ser Thr Ser Thr Ala Tyr
65                      70                      75                      80
Met Glu Leu Ser Ser Leu Arg Ser Glu Asp Thr Ala Val Tyr Tyr Cys
                85                      90                      95
Ala Arg Gly Gly Gly Gly Phe Thr Tyr Trp Gly Gln Gly Thr Leu Val
            100                      105                      110
Thr Val Ser Ser Ala Ser Thr Lys Gly Pro Ser Val Phe Pro Leu Ala
            115                      120                      125
Pro Ser Ser Lys Ser Thr Ser Gly Gly Thr Ala Ala Leu Gly Cys Leu
            130                      135                      140
Val Lys Asp Tyr Phe Pro Glu Pro Val Thr Val Ser Trp Asn Ser Gly
145                      150                      155                      160
Ala Leu Thr Ser Gly Val His Thr Phe Pro Ala Val Leu Gln Ser Ser
```

```
                          165                     170                      175
        Gly Leu Tyr Ser Leu Ser Ser Val Val Thr Val Pro Ser Ser Ser Leu
                      180                     185                      190
        Gly Thr Gln Thr Tyr Ile Cys Asn Val Asn His Lys Pro Ser Asn Thr
                      195                     200                      205
        Lys Val Asp Lys Lys Val Glu Pro Lys Ser Cys Asp Lys Thr His Thr
                      210                     215                      220
        Cys Pro Pro Cys Pro Ala Pro Glu Leu Leu Gly Gly Pro Ser Val Phe
        225                     230                     235                      240
        Leu Phe Pro Pro Lys Pro Lys Asp Thr Leu Met Ile Ser Arg Thr Pro
                      245                     250                      255
        Glu Val Thr Cys Val Val Val Asp Val Glu His Glu Asp Pro Glu Val
                      260                     265                      270
        Lys Phe Asn Trp Tyr Val Asp Gly Val Glu Val His Asn Ala Lys Thr
                      275                     280                      285
        Lys Pro Arg Glu Glu Gln Tyr Asn Ser Thr Tyr Arg Val Val Ser Val
                      290                     295                      300
        Leu Thr Val Leu His Gln Asp Trp Leu Asn Gly Lys Glu Tyr Lys Cys
        305                     310                     315                      320
        Lys Val Ser Ser Lys Ala Phe Pro Ala Pro Ile Glu Lys Thr Ile Ser
                      325                     330                      335
        Lys Ala Lys Gly Gln Pro Arg Glu Pro Gln Val Tyr Thr Leu Pro Pro
                      340                     345                      350
        Ser Arg Asp Glu Leu Thr Lys Asn Gln Val Ser Leu Thr Cys Leu Val
                      355                     360                      365
        Lys Gly Phe Tyr Pro Ser Asp Ile Ala Val Glu Trp Glu Ser Asn Gly
                      370                     375                      380
        Gln Pro Glu Asn Asn Tyr Lys Thr Thr Pro Pro Val Leu Asp Ser Asp
        385                     390                     395                      400
        Gly Ser Phe Phe Leu Tyr Ser Lys Leu Thr Val Asp Lys Ser Arg Trp
                      405                     410                      415
        Gln Gln Gly Asn Val Phe Ser Cys Ser Val Met His Glu Ala Leu His
                      420                     425                      430
        Asn His Tyr Thr Gln Lys Ser Leu Ser Leu Ser Pro Gly Lys
                      435                     440                      445


        <210>   63
        <211>   442
        <212>   PRT
        <213>   Artificial Sequence

        <220>
        <221>   CHAIN
        <222>   (1)..(442)
        <223>   hu9E5-M HC


        <400>   63
        Gln Val Gln Leu Val Gln Ser Gly Ala Glu Val Lys Lys Pro Gly Ala
        1                   5                     10                      15
        Ser Val Lys Val Ser Cys Lys Ala Ser Gly Tyr Ile Leu Thr Thr Tyr
                      20                     25                     30
        Trp Ile Thr Trp Val Arg Gln Ala Pro Gly Gln Gly Leu Glu Trp Met
                      35                     40                     45
        Gly Asp Ile His Pro Gly Ser Gly Ser Thr Lys Tyr Asn Glu Lys Phe
                      50                     55                     60
        Lys Ser Arg Val Thr Leu Thr Val Asp Thr Ser Ile Ser Thr Ala Tyr
        65                     70                     75                      80
        Met Glu Leu Ser Arg Leu Arg Ser Glu Asp Thr Ala Val Tyr Tyr Cys
                      85                     90                     95
        Ala Arg Arg Asp Tyr Trp Gly Gln Gly Thr Thr Val Thr Val Ser Ser
                      100                     105                      110
        Ala Ser Thr Lys Gly Pro Ser Val Phe Pro Leu Ala Pro Ser Ser Lys
```

```
                115                    120                    125
        Ser Thr Ser Gly Gly Thr Ala Ala Leu Gly Cys Leu Val Lys Asp Tyr
            130                    135                    140
        Phe Pro Glu Pro Val Thr Val Ser Trp Asn Ser Gly Ala Leu Thr Ser
        145                    150                    155                    160
        Gly Val His Thr Phe Pro Ala Val Leu Gln Ser Ser Gly Leu Tyr Ser
                            165                    170                    175
        Leu Ser Ser Val Val Thr Val Pro Ser Ser Leu Gly Thr Gln Thr
                    180                    185                    190
        Tyr Ile Cys Asn Val Asn His Lys Pro Ser Asn Thr Lys Val Asp Lys
                    195                    200                    205
        Lys Val Glu Pro Lys Ser Cys Asp Lys Thr His Thr Cys Pro Pro Cys
            210                    215                    220
        Pro Ala Pro Glu Leu Leu Gly Gly Pro Ser Val Phe Leu Phe Pro Pro
        225                    230                    235                    240
        Lys Pro Lys Asp Thr Leu Met Ile Ser Arg Thr Pro Glu Val Thr Cys
                            245                    250                    255
        Val Val Val Asp Val Glu His Glu Asp Pro Glu Val Lys Phe Asn Trp
                    260                    265                    270
        Tyr Val Asp Gly Val Glu Val His Asn Ala Lys Thr Lys Pro Arg Glu
                    275                    280                    285
        Glu Gln Tyr Asn Ser Thr Tyr Arg Val Val Ser Val Leu Thr Val Leu
                    290                    295                    300
        His Gln Asp Trp Leu Asn Gly Lys Glu Tyr Lys Cys Lys Val Ser Asn
        305                    310                    315                    320
        Lys Ala Leu Pro Ala Pro Ile Glu Lys Thr Ile Ser Lys Ala Lys Gly
                            325                    330                    335
        Gln Pro Arg Glu Pro Gln Val Tyr Thr Leu Pro Pro Ser Arg Asp Glu
                    340                    345                    350
        Leu Thr Lys Asn Gln Val Ser Leu Thr Cys Leu Val Lys Gly Phe Tyr
                    355                    360                    365
        Pro Ser Asp Ile Ala Val Glu Trp Glu Ser Asn Gly Gln Pro Glu Asn
                    370                    375                    380
        Asn Tyr Lys Thr Thr Pro Pro Val Leu Asp Ser Asp Gly Ser Phe Phe
        385                    390                    395                    400
        Leu Tyr Ser Lys Leu Thr Val Asp Lys Ser Arg Trp Gln Gln Gly Asn
                            405                    410                    415
        Val Phe Ser Cys Ser Val Met His Glu Ala Leu His Asn His Tyr Thr
                    420                    425                    430
        Gln Lys Ser Leu Ser Leu Ser Pro Gly Lys
                    435                    440


        <210>   64
        <211>   442
        <212>   PRT
        <213>   Artificial Sequence

        <220>
        <221>   CHAIN
        <222>   (1)..(442)
        <223>   hu9E5-SELF HC


        <400>   64
        Gln Val Gln Leu Val Gln Ser Gly Ala Glu Val Lys Lys Pro Gly Ala
        1                   5                   10                  15
        Ser Val Lys Val Ser Cys Lys Ala Ser Gly Tyr Ile Leu Thr Thr Tyr
                    20                  25                  30
        Trp Ile Thr Trp Val Arg Gln Ala Pro Gly Gln Gly Leu Glu Trp Met
                    35                  40                  45
        Gly Asp Ile His Pro Gly Ser Gly Ser Thr Lys Tyr Asn Glu Lys Phe
            50                  55                  60
        Lys Ser Arg Val Thr Leu Thr Val Asp Thr Ser Ile Ser Thr Ala Tyr
```

```
                    65                      70                      75                      80
Met Glu Leu Ser Arg Leu Arg Ser Glu Asp Thr Ala Val Tyr Tyr Cys
                    85                      90                      95
Ala Arg Arg Asp Tyr Trp Gly Gln Gly Thr Thr Val Thr Val Ser Ser
                    100                     105                     110
Ala Ser Thr Lys Gly Pro Ser Val Phe Pro Leu Ala Pro Ser Ser Lys
                    115                     120                     125
Ser Thr Ser Gly Gly Thr Ala Ala Leu Gly Cys Leu Val Lys Asp Tyr
        130                     135                     140
Phe Pro Glu Pro Val Thr Val Ser Trp Asn Ser Gly Ala Leu Thr Ser
145                     150                     155                     160
Gly Val His Thr Phe Pro Ala Val Leu Gln Ser Ser Gly Leu Tyr Ser
                    165                     170                     175
Leu Ser Ser Val Val Thr Val Pro Ser Ser Ser Leu Gly Thr Gln Thr
                    180                     185                     190
Tyr Ile Cys Asn Val Asn His Lys Pro Ser Asn Thr Lys Val Asp Lys
                    195                     200                     205
Lys Val Glu Pro Lys Ser Cys Asp Lys Thr His Thr Cys Pro Pro Cys
        210                     215                     220
Pro Ala Pro Glu Leu Leu Gly Gly Pro Ser Val Phe Leu Phe Pro Pro
225                     230                     235                     240
Lys Pro Lys Asp Thr Leu Met Ile Ser Arg Thr Pro Glu Val Thr Cys
                    245                     250                     255
Val Val Val Asp Val Glu His Glu Asp Pro Glu Val Lys Phe Asn Trp
                    260                     265                     270
Tyr Val Asp Gly Val Glu Val His Asn Ala Lys Thr Lys Pro Arg Glu
        275                     280                     285
Glu Gln Tyr Asn Ser Thr Tyr Arg Val Val Ser Val Leu Thr Val Leu
        290                     295                     300
His Gln Asp Trp Leu Asn Gly Lys Glu Tyr Lys Cys Lys Val Ser Asn
305                     310                     315                     320
Lys Ala Phe Pro Ala Pro Ile Glu Lys Thr Ile Ser Lys Ala Lys Gly
                    325                     330                     335
Gln Pro Arg Glu Pro Gln Val Tyr Thr Leu Pro Pro Ser Arg Asp Glu
        340                     345                     350
Leu Thr Lys Asn Gln Val Ser Leu Thr Cys Leu Val Lys Gly Phe Tyr
        355                     360                     365
Pro Ser Asp Ile Ala Val Glu Trp Glu Ser Asn Gly Gln Pro Glu Asn
        370                     375                     380
Asn Tyr Lys Thr Thr Pro Pro Val Leu Asp Ser Asp Gly Ser Phe Phe
385                     390                     395                     400
Leu Tyr Ser Lys Leu Thr Val Asp Lys Ser Arg Trp Gln Gln Gly Asn
                    405                     410                     415
Val Phe Ser Cys Ser Val Met His Glu Ala Leu His Asn His Tyr Thr
                    420                     425                     430
Gln Lys Ser Leu Ser Leu Ser Pro Gly Lys
        435                     440
```

```
<210>   65
<211>   450
<212>   PRT
<213>   Artificial Sequence

<220>
<221>   CHAIN
<222>   (1)..(450)
<223>   005M-HC


<400>   65
Gln Val Gln Leu Val Glu Ser Gly Gly Gly Val Val Gln Pro Gly Arg
1                   5                   10                      15
Ser Leu Arg Leu Ser Cys Ala Ala Ser Gly Phe Ser Phe Ser Ser Thr
```

```
                        20                        25                        30
    Tyr Val Cys Trp Val Arg Gln Ala Pro Gly Lys Gly Leu Glu Trp Ile
        35                        40                        45
    Ala Cys Ile Tyr Thr Gly Asp Gly Thr Asn Tyr Ser Ala Ser Trp Ala
        50                        55                        60
    Lys Gly Arg Phe Thr Ile Ser Lys Asp Ser Ser Lys Asn Thr Val Tyr
    65                        70                        75                        80
    Leu Gln Met Asn Ser Leu Arg Ala Glu Asp Thr Ala Val Tyr Phe Cys
                    85                        90                        95
    Ala Arg Pro Asp Ile Thr Tyr Gly Phe Ala Ile Asn Phe Trp Gly Pro
                100                       105                       110
    Gly Thr Leu Val Thr Val Ser Ser Ala Ser Thr Lys Gly Pro Ser Val
            115                       120                       125
    Phe Pro Leu Ala Pro Ser Ser Lys Ser Thr Ser Gly Gly Thr Ala Ala
        130                       135                       140
    Leu Gly Cys Leu Val Lys Asp Tyr Phe Pro Glu Pro Val Thr Val Ser
    145                       150                       155                       160
    Trp Asn Ser Gly Ala Leu Thr Ser Gly Val His Thr Phe Pro Ala Val
                    165                       170                       175
    Leu Gln Ser Ser Gly Leu Tyr Ser Leu Ser Ser Val Val Thr Val Pro
                180                       185                       190
    Ser Ser Ser Leu Gly Thr Gln Thr Tyr Ile Cys Asn Val Asn His Lys
            195                       200                       205
    Pro Ser Asn Thr Lys Val Asp Lys Lys Val Glu Pro Lys Ser Cys Asp
        210                       215                       220
    Lys Thr His Thr Cys Pro Pro Cys Pro Ala Pro Glu Leu Leu Gly Gly
    225                       230                       235                       240
    Pro Ser Val Phe Leu Phe Pro Pro Lys Pro Lys Asp Thr Leu Met Ile
                    245                       250                       255
    Ser Arg Thr Pro Glu Val Thr Cys Val Val Val Asp Val Glu His Glu
                260                       265                       270
    Asp Pro Glu Val Lys Phe Asn Trp Tyr Val Asp Gly Val Glu Val His
                275                       280                       285
    Asn Ala Lys Thr Lys Pro Arg Glu Glu Gln Tyr Asn Ser Thr Tyr Arg
        290                       295                       300
    Val Val Ser Val Leu Thr Val Leu His Gln Asp Trp Leu Asn Gly Lys
    305                       310                       315                       320
    Glu Tyr Lys Cys Lys Val Ser Asn Lys Ala Leu Pro Ala Pro Ile Glu
                    325                       330                       335
    Lys Thr Ile Ser Lys Ala Lys Gly Gln Pro Arg Glu Pro Gln Val Tyr
                340                       345                       350
    Thr Leu Pro Pro Ser Arg Asp Glu Leu Thr Lys Asn Gln Val Ser Leu
            355                       360                       365
    Thr Cys Leu Val Lys Gly Phe Tyr Pro Ser Asp Ile Ala Val Glu Trp
        370                       375                       380
    Glu Ser Asn Gly Gln Pro Glu Asn Asn Tyr Lys Thr Thr Pro Pro Val
    385                       390                       395                       400
    Leu Asp Ser Asp Gly Ser Phe Phe Leu Tyr Ser Lys Leu Thr Val Asp
                    405                       410                       415
    Lys Ser Arg Trp Gln Gln Gly Asn Val Phe Ser Cys Ser Val Met His
                420                       425                       430
    Glu Ala Leu His Asn His Tyr Thr Gln Lys Ser Leu Ser Leu Ser Pro
                435                       440                       445
    Gly Lys
        450
```

```
<210>   66
<211>   215
<212>   PRT
<213>   Artificial Sequence

<220>
<221>   CHAIN
```

<222> (1)..(215)
<223> 005M-LC

<400> 66
Asp Ile Gln Met Thr Gln Ser Pro Ser Ser Leu Ser Ala Ser Val Gly
1               5                   10                  15
Asp Arg Val Thr Ile Lys Cys Gln Ala Ser Gln Ser Ile Ser Ser Arg
            20                  25                  30
Leu Ala Trp Tyr Gln Gln Lys Pro Gly Lys Pro Pro Lys Leu Leu Ile
        35                  40                  45
Tyr Arg Ala Ser Thr Leu Ala Ser Gly Val Pro Ser Arg Phe Ser Gly
    50                  55                  60
Ser Gly Ser Gly Thr Asp Phe Thr Leu Thr Ile Ser Ser Leu Gln Pro
65                  70                  75                  80
Glu Asp Val Ala Thr Tyr Tyr Cys Gln Cys Thr Gly Tyr Gly Ile Ser
                85                  90                  95
Trp Pro Ile Gly Gly Gly Thr Lys Val Glu Ile Lys Arg Thr Val Ala
            100                 105                 110
Ala Pro Ser Val Phe Ile Phe Pro Pro Ser Asp Glu Gln Leu Lys Ser
        115                 120                 125
Gly Thr Ala Ser Val Val Cys Leu Leu Asn Asn Phe Tyr Pro Arg Glu
    130                 135                 140
Ala Lys Val Gln Trp Lys Val Asp Asn Ala Leu Gln Ser Gly Asn Ser
145                 150                 155                 160
Gln Glu Ser Val Thr Glu Gln Asp Ser Lys Asp Ser Thr Tyr Ser Leu
                165                 170                 175
Ser Ser Thr Leu Thr Leu Ser Lys Ala Asp Tyr Glu Lys His Lys Val
            180                 185                 190
Tyr Ala Cys Glu Val Thr His Gln Gly Leu Ser Ser Pro Val Thr Lys
        195                 200                 205
Ser Phe Asn Arg Gly Glu Cys
    210                 215


<210> 67
<211> 442
<212> PRT
<213> Artificial Sequence

<220>
<221> CHAIN
<222> (1)..(442)
<223> hu9E5-SELFNS HC


<400> 67
Gln Val Gln Leu Val Gln Ser Gly Ala Glu Val Lys Lys Pro Gly Ala
1               5                   10                  15
Ser Val Lys Val Ser Cys Lys Ala Ser Gly Tyr Ile Leu Thr Thr Tyr
            20                  25                  30
Trp Ile Thr Trp Val Arg Gln Ala Pro Gly Gln Gly Leu Glu Trp Met
        35                  40                  45
Gly Asp Ile His Pro Gly Ser Gly Ser Thr Lys Tyr Asn Glu Lys Phe
    50                  55                  60
Lys Ser Arg Val Thr Leu Thr Val Asp Thr Ser Ile Ser Thr Ala Tyr
65                  70                  75                  80
Met Glu Leu Ser Arg Leu Arg Ser Glu Asp Thr Ala Val Tyr Tyr Cys
                85                  90                  95
Ala Arg Arg Asp Tyr Trp Gly Gln Gly Thr Thr Val Thr Val Ser Ser
            100                 105                 110
Ala Ser Thr Lys Gly Pro Ser Val Phe Pro Leu Ala Pro Ser Ser Lys
        115                 120                 125
Ser Thr Ser Gly Gly Thr Ala Ala Leu Gly Cys Leu Val Lys Asp Tyr

```
           130                      135                       140
Phe Pro Glu Pro Val Thr Val Ser Trp Asn Ser Gly Ala Leu Thr Ser
145                      150                       155                       160
Gly Val His Thr Phe Pro Ala Val Leu Gln Ser Ser Gly Leu Tyr Ser
               165                      170                       175
Leu Ser Ser Val Thr Val Pro Ser Ser Ser Leu Gly Thr Gln Thr
               180                      185                       190
Tyr Ile Cys Asn Val Asn His Lys Pro Ser Asn Thr Lys Val Asp Lys
       195                      200                       205
Lys Val Glu Pro Lys Ser Cys Asp Lys Thr His Thr Cys Pro Pro Cys
       210                      215                       220
Pro Ala Pro Glu Leu Leu Gly Gly Pro Ser Val Phe Leu Phe Pro Pro
225                      230                       235                       240
Lys Pro Lys Asp Thr Leu Met Ile Ser Arg Thr Pro Glu Val Thr Cys
                   245                      250                       255
Val Val Val Asp Val Glu His Glu Asp Pro Glu Val Lys Phe Asn Trp
               260                      265                       270
Tyr Val Asp Gly Val Glu Val His Asn Ala Lys Thr Lys Pro Arg Glu
       275                      280                       285
Glu Gln Tyr Asn Ser Thr Tyr Arg Val Val Ser Val Leu Thr Val Leu
       290                      295                       300
His Gln Asp Trp Leu Asn Gly Lys Glu Tyr Lys Cys Lys Val Ser Ser
305                      310                       315                       320
Lys Ala Phe Pro Ala Pro Ile Glu Lys Thr Ile Ser Lys Ala Lys Gly
                   325                      330                       335
Gln Pro Arg Glu Pro Gln Val Tyr Thr Leu Pro Pro Ser Arg Asp Glu
               340                      345                       350
Leu Thr Lys Asn Gln Val Ser Leu Thr Cys Leu Val Lys Gly Phe Tyr
       355                      360                       365
Pro Ser Asp Ile Ala Val Glu Trp Glu Ser Asn Gly Gln Pro Glu Asn
   370                      375                       380
Asn Tyr Lys Thr Thr Pro Pro Val Leu Asp Ser Asp Gly Ser Phe Phe
385                      390                       395                       400
Leu Tyr Ser Lys Leu Thr Val Asp Lys Ser Arg Trp Gln Gln Gly Asn
                   405                      410                       415
Val Phe Ser Cys Ser Val Met His Glu Ala Leu His Asn His Tyr Thr
               420                      425                       430
Gln Lys Ser Leu Ser Leu Ser Pro Gly Lys
       435                      440

<210>   68
<211>   450
<212>   PRT
<213>   Artificial Sequence

<220>
<221>   CHAIN
<222>   (1)..(450)
<223>   APX005-SELF HC


<400>   68
Gln Val Gln Leu Val Glu Ser Gly Gly Gly Val Val Gln Pro Gly Arg
1               5                       10                        15
Ser Leu Arg Leu Ser Cys Ala Ala Ser Gly Phe Ser Phe Ser Ser Thr
               20                      25                        30
Tyr Val Cys Trp Val Arg Gln Ala Pro Gly Lys Gly Leu Glu Trp Ile
           35                      40                        45
Ala Cys Ile Tyr Thr Gly Asp Gly Thr Asn Tyr Ser Ala Ser Trp Ala
       50                      55                        60
Lys Gly Arg Phe Thr Ile Ser Lys Asp Ser Ser Lys Asn Thr Val Tyr
65                      70                        75                        80
Leu Gln Met Asn Ser Leu Arg Ala Glu Asp Thr Ala Val Tyr Phe Cys
```

```
                         85                        90                        95
        Ala Arg Pro Asp Ile Thr Tyr Gly Phe Ala Ile Asn Phe Trp Gly Pro
                    100                       105                       110
        Gly Thr Leu Val Thr Val Ser Ser Ala Ser Thr Lys Gly Pro Ser Val
                    115                       120                       125
        Phe Pro Leu Ala Pro Ser Ser Lys Ser Thr Ser Gly Gly Thr Ala Ala
                    130                       135                       140
        Leu Gly Cys Leu Val Lys Asp Tyr Phe Pro Glu Pro Val Thr Val Ser
        145                       150                       155                       160
        Trp Asn Ser Gly Ala Leu Thr Ser Gly Val His Thr Phe Pro Ala Val
                    165                       170                       175
        Leu Gln Ser Ser Gly Leu Tyr Ser Leu Ser Ser Val Val Thr Val Pro
                    180                       185                       190
        Ser Ser Ser Leu Gly Thr Gln Thr Tyr Ile Cys Asn Val Asn His Lys
                    195                       200                       205
        Pro Ser Asn Thr Lys Val Asp Lys Lys Val Glu Pro Lys Ser Cys Asp
                    210                       215                       220
        Lys Thr His Thr Cys Pro Pro Cys Pro Ala Pro Glu Leu Leu Gly Gly
        225                       230                       235                       240
        Pro Ser Val Phe Leu Phe Pro Pro Lys Pro Lys Asp Thr Leu Met Ile
                    245                       250                       255
        Ser Arg Thr Pro Glu Val Thr Cys Val Val Asp Val Glu His Glu
                    260                       265                       270
        Asp Pro Glu Val Lys Phe Asn Trp Tyr Val Asp Gly Val Glu Val His
                    275                       280                       285
        Asn Ala Lys Thr Lys Pro Arg Glu Glu Gln Tyr Asn Ser Thr Tyr Arg
                    290                       295                       300
        Val Val Ser Val Leu Thr Val Leu His Gln Asp Trp Leu Asn Gly Lys
        305                       310                       315                       320
        Glu Tyr Lys Cys Lys Val Ser Asn Lys Ala Phe Pro Ala Pro Ile Glu
                    325                       330                       335
        Lys Thr Ile Ser Lys Ala Lys Gly Gln Pro Arg Glu Pro Gln Val Tyr
                    340                       345                       350
        Thr Leu Pro Pro Ser Arg Asp Glu Leu Thr Lys Asn Gln Val Ser Leu
                    355                       360                       365
        Thr Cys Leu Val Lys Gly Phe Tyr Pro Ser Asp Ile Ala Val Glu Trp
                    370                       375                       380
        Glu Ser Asn Gly Gln Pro Glu Asn Asn Tyr Lys Thr Thr Pro Pro Val
        385                       390                       395                       400
        Leu Asp Ser Asp Gly Ser Phe Phe Leu Tyr Ser Lys Leu Thr Val Asp
                    405                       410                       415
        Lys Ser Arg Trp Gln Gln Gly Asn Val Phe Ser Cys Ser Val Met His
                    420                       425                       430
        Glu Ala Leu His Asn His Tyr Thr Gln Lys Ser Leu Ser Leu Ser Pro
                    435                       440                       445
        Gly Lys
        450
```

<210> 69
<211> 450
<212> PRT
<213> Artificial Sequence

<220>
<221> CHAIN
<222> (1)..(450)
<223> APX005-SELFNS HC


<400> 69
```
        Gln Val Gln Leu Val Glu Ser Gly Gly Gly Val Val Gln Pro Gly Arg
        1               5                       10                      15
        Ser Leu Arg Leu Ser Cys Ala Ala Ser Gly Phe Ser Phe Ser Ser Thr
```

```
                      20                    25                    30
     Tyr Val Cys Trp Val Arg Gln Ala Pro Gly Lys Gly Leu Glu Trp Ile
             35              40                    45
     Ala Cys Ile Tyr Thr Gly Asp Gly Thr Asn Tyr Ser Ala Ser Trp Ala
             50              55                    60
     Lys Gly Arg Phe Thr Ile Ser Lys Asp Ser Ser Lys Asn Thr Val Tyr
     65              70              75              80
     Leu Gln Met Asn Ser Leu Arg Ala Glu Asp Thr Ala Val Tyr Phe Cys
                 85              90                    95
     Ala Arg Pro Asp Ile Thr Tyr Gly Phe Ala Ile Asn Phe Trp Gly Pro
                 100             105                   110
     Gly Thr Leu Val Thr Val Ser Ser Ala Ser Thr Lys Gly Pro Ser Val
             115             120                   125
     Phe Pro Leu Ala Pro Ser Ser Lys Ser Thr Ser Gly Gly Thr Ala Ala
         130                 135                   140
     Leu Gly Cys Leu Val Lys Asp Tyr Phe Pro Glu Pro Val Thr Val Ser
     145                 150                 155                   160
     Trp Asn Ser Gly Ala Leu Thr Ser Gly Val His Thr Phe Pro Ala Val
                 165                 170                   175
     Leu Gln Ser Ser Gly Leu Tyr Ser Leu Ser Ser Val Val Thr Val Pro
             180                 185                   190
     Ser Ser Ser Leu Gly Thr Gln Thr Tyr Ile Cys Asn Val Asn His Lys
             195                 200                   205
     Pro Ser Asn Thr Lys Val Asp Lys Lys Val Glu Pro Lys Ser Cys Asp
             210                 215                   220
     Lys Thr His Thr Cys Pro Pro Cys Pro Ala Pro Glu Leu Leu Gly Gly
     225                 230                 235                   240
     Pro Ser Val Phe Leu Phe Pro Pro Lys Pro Lys Asp Thr Leu Met Ile
                 245                 250                   255
     Ser Arg Thr Pro Glu Val Thr Cys Val Val Val Asp Val Glu His Glu
                 260                 265                   270
     Asp Pro Glu Val Lys Phe Asn Trp Tyr Val Asp Gly Val Glu Val His
             275                 280                   285
     Asn Ala Lys Thr Lys Pro Arg Glu Glu Gln Tyr Asn Ser Thr Tyr Arg
         290                 295                   300
     Val Val Ser Val Leu Thr Val Leu His Gln Asp Trp Leu Asn Gly Lys
     305                 310                 315                   320
     Glu Tyr Lys Cys Lys Val Ser Ser Lys Ala Phe Pro Ala Pro Ile Glu
                 325                 330                   335
     Lys Thr Ile Ser Lys Ala Lys Gly Gln Pro Arg Glu Pro Gln Val Tyr
                 340                 345                   350
     Thr Leu Pro Pro Ser Arg Asp Glu Leu Thr Lys Asn Gln Val Ser Leu
             355                 360                   365
     Thr Cys Leu Val Lys Gly Phe Tyr Pro Ser Asp Ile Ala Val Glu Trp
         370                 375                   380
     Glu Ser Asn Gly Gln Pro Glu Asn Asn Tyr Lys Thr Thr Pro Pro Val
     385                 390                 395                   400
     Leu Asp Ser Asp Gly Ser Phe Phe Leu Tyr Ser Lys Leu Thr Val Asp
                 405                 410                   415
     Lys Ser Arg Trp Gln Gln Gly Asn Val Phe Ser Cys Ser Val Met His
                 420                 425                   430
     Glu Ala Leu His Asn His Tyr Thr Gln Lys Ser Leu Ser Leu Ser Pro
             435                 440                   445
     Gly Lys
         450
```